# EUROPEAN PATENT APPLICATION

(11) **EP 3 370 065 A1**
(43) Date of publication of application: **05.09.2018**
(21) Application number: 17159243.9
(22) Date of filing: 03.03.2017
(51) Int. Cl.: G01N 33/569, A61K 38/00

(54) **IMMUNOGENIC PEPTIDES**

(71) Applicant: TREOS BIO KFT, 8200 Veszprem (HU)
(72) Inventor: LISZIEWICZ, Dr Julianna, Szorosi ut 13 (HU); MOLNAR, Levente, Somogyi-Bacsó u. 18 (HU); TOKE, Dr Eniko R, Orczy tér 4/A2, 2/17-18 (HU); TOTH, József, Újkut u. 72 (HU); LORINCZ, Dr Orsolya, Attila u. 27, fszt/13 (HU); CSISZOVSZKI, Dr Zsolt, Bikszádi u. 12/A, 4/9 (HU); SOMOGYI, Dr Eszter, Borgazda u. 3 (HU); PANTYA, Katalin, Szondi u. 44/B, 2/1 (HU); MEGYESI, Mónika, Táncsics Mihály u. 63 (HU)
(74) Representative: J A Kemp

(57) **Abstract**

The invention relates to methods of identifying fragments of a polypeptide that are immunogenic for a specific human subject, methods of preparing personalised pharmaceutical compositions comprising such polypeptide fragments, human subject-specific pharmaceutical compositions comprising such polypeptide fragments, and methods of treatment using such compositions. The methods comprise identifying a fragment of the polypeptide that binds to multiple HLA of the subject.

## Description

### Field of Invention

The invention relates to methods of identifying fragments of a polypeptide that are immunogenic for a specific human subject, methods of preparing personalised pharmaceutical compositions comprising such polypeptide fragments, human subject-specific pharmaceutical compositions comprising such polypeptide fragments, and methods of treatment using such compositions.

### Background to the invention

For decades, scientists have assumed that chronic diseases were beyond the reach of a person's natural defences. Recently, however, significant tumor regressions observed in individuals treated with antibodies that block immune inhibitory molecules have accelerated the field of cancer immunotherapy. These clinical findings demonstrate that re-activation of existing T cell responses results in meaningful clinical benefit for individuals. These advances have renewed enthusiasm for developing cancer vaccines that induce tumor specific T cell responses.

Despite the promise, current immunotherapy is effective only in a fraction of individuals. In addition, most cancer vaccine trials have failed to demonstrate statistically significant efficacy because of a low rate of tumor regression and antitumor T cell responses in individuals. Similar failures were reported with therapeutic and preventive vaccines that sought to include T cell responses in the fields of HIV and allergy. There is a need to overcome the clinical failures of immunotherapies and vaccines.

### Summary of the invention

In antigen presenting cells (APC) protein antigens are processed into peptides. These peptides bind to human leukocyte antigen molecules (HLAs) and are presented on the cell surface as peptide-HLA complexes to T cells. Different individuals express different HLA molecules and different HLA molecules present different peptides. Therefore, according to the state of the art, a peptide, or a fragment of a larger polypeptide, is identified as immunogenic for a specific human subject if it is presented by a HLA molecule that is expressed by the subject. In other words, the state of the art describes immunogenic peptides as HLA-restricted epitopes. However, HLA restricted epitopes induce T cell responses in only a fraction of individuals who express the HLA molecule. Peptides that activate a T cell response in one individual are inactive in others despite HLA allele matching. Therefore, it was unknown how an individual's HLA molecules present the antigen-derived epitopes that positively activate T cell responses.

The inventors have discovered that multiple HLA expressed by an individual need to present the same peptide in order to trigger a T cell response. Therefore the fragments of a polypeptide antigen that are immunogenic for a specific individual are those that can bind to multiple class I (activate cytotoxic T cells) or class II (activate helper T cells) HLAs expressed by that individual.

Accordingly, in a first aspect the invention provides a method of identifying a fragment of a polypeptide as immunogenic for a specific human subject, the method comprising the steps of
(i) determining the HLA class I genotype and/or the HLA class II genotype of the specific human subject;
(ii) determining whether the polypeptide comprises:
   a. a sequence that is a T cell epitope capable of binding to at least two HLA class I of the subject; or
   b. a sequence that is a T cell epitope capable of binding to at least two HLA class II of the subject; and
(iii) identifying said sequence as a fragment of the polypeptide that is immunogenic for the subject.

A specific polypeptide antigen may comprise more than one fragment that is a T cell epitope capable of binding to multiple HLA of a specific individual. The combined group of all such fragments characterize the individual's antigen specific T cell response set, wherein the amino acid sequence of each fragment characterizes the specificity of each activated T cell clone.

Accordingly in some cases steps (ii) and (iii) of the method above are repeated until all of the fragments of the polypeptide that are a T cell epitope capable of binding to at least two HLA class I and/or at least two HLA class II of the subject have been identified. This method characterises the immune response of the subject to the polypeptide.

The fragments of polypeptide that are determined to be immunogenic for a specific human subjects in accordance with the methods above can be used to prepare human subject-specific immunogenic compositions.

Accordingly in a further aspect, the invention provides a method of preparing a human subject-specific pharmaceutical composition, the method comprising:
(i) selecting a fragment of a polypeptide, which fragment has been identified as immunogenic for the subject by the method above;
(ii) selecting a sequence of up to 50 consecutive amino acids of the polypeptide, which consecutive amino acids comprise the amino acid sequence of the fragment;
(iii) selecting a second immunogenic fragment of the same or of a different polypeptide to the fragment selected in step (i), and selecting a sequence of up to 50 consecutive amino acids of the polypeptide, which consecutive amino acids comprise the amino acid sequence of the second fragment;
(iv) optionally selecting one or more additional immunogenic fragments from the same or different polypeptides to the fragments selected in the preceding steps, and selecting, for each additional fragment, a sequence of up to 50 consecutive amino acids of the polypeptide, which consecutive amino acids comprise the amino acid sequence of the additional fragment; and
(v) preparing a composition having as active ingredients one or more peptides having all of the amino acid sequences selected in the preceding steps, wherein each peptide either consists of one of the selected amino acid sequences, or consists of two or more of the amino acid sequences arranged end to end or overlapping in a single peptide.

The invention further provides a human subject-specific pharmaceutical composition prepared according to the method above.

In further aspects, the invention provides
- a method of treatment comprising administering to a human subject in need thereof a human subject-specific pharmaceutical composition prepared according to the method above, wherein the composition is specific for the subject;
- a human subject-specific immunogenic composition prepared according to the method above for use in a method of treatment of the specific human subject; and
- use of a human subject-specific pharmaceutical composition prepared according to the method above in the manufacture of a medicament; wherein the medicament is specific for the subject.

The invention will now be described in more detail, by way of example and not limitation, and by reference to the accompanying drawings. Many equivalent modifications and variations will be apparent, to those skilled in the art when given this disclosure. Accordingly, the exemplary embodiments of the invention set forth are considered to be illustrative and not limiting. Various changes to the described embodiments may be made without departing from the scope of the invention. All documents cited herein, whether supra or infra, are expressly incorporated by reference in their entirety.

The present invention includes the combination of the aspects and preferred features described except where such a combination is clearly impermissible or is stated to be expressly avoided. As used in this specification and the appended claims, the singular forms "a", "an", and "the" include plural referents unless the content clearly dictates otherwise. Thus, for example, reference to "a peptide" includes two or more such peptides.

Section headings are used herein for convenience only and are not to be construed as limiting in any way.

### Description of the Figures

**Fig.** 1
   ROC curve of HLA restricted PEPI biomarkers.
**Fig.** 2
   ROC curve of ≥1 PEPI3+ Test for the determination of the diagnostic accuracy.
**Fig.** 3
   Distribution of HLA class I PEPI3+ compared to CD8+ T cell responses measured by a state of art assay among peptide pools used in the CD8+ T cell response assays. **A**: HLA class I restricted PEPI3+s. The 90% Overall Percent of Agreement (OPA) among the T cell responses and PEPI3+ peptides demonstrate the utility of the invented peptides for prediction of vaccine induced T cell response set of individuals. **B:** Class I HLA restricted epitopes (PEPI1+). The OPA between predicted epitopes and CD8+ T cell responses was 28% (not statistically significant). Darkest grey: True positive (TP), both peptide and T cell responses were detected; Light grey: False negative (FN), only T cell responses were detected; Lightest grey: False positive (FP), only peptide were detected; Dark grey: True negative (TN): neither peptides nor T cell responses were detected.
**Fig.** 4
   Distribution of HLA class II PEPIs compared to CD4+ T cell responses measured by a state of art assay among peptide pools used in the assays. **A**: HLA class II restricted PEPI4+s. 67% OPA between PEPI4+ and CD4+ T-cell responses (p=0.002). **B:** The class II HLA restricted epitopes. OPA between class II HLA restricted epitopes and CD4+ T cell responses was 66% (not statistically significant). Darkest grey: True positive (TP), both peptide and T cell responses were detected; Light grey: False negative (FN), only T cell responses were detected; Lightest grey: False positive (FP), only peptide were detected; Dark grey: True negative (TN): neither peptides nor T cell responses were detected.
**Fig. 5**
   Multiple HLA binding peptides that define the HPV-16 LPV vaccine specific T cell response set of 18 VIN-3 and 5 cervical cancer patients. HLA class I restricted PEPI3 counts (**A** and **B**) and HLA class II restricted PEPI3 counts (**C** and **D**) derived from LPV antigens of each patient. Light grey: immune responders measured after vaccination in the clinical trial; Dark grey: Immune non-responders measured after vaccination in the clinical trial. Results show that ≧3 HLA class I binding peptides predict the CD8+ T cell reactivity and ≧4 HLA class II binding peptides predict the CD4+ T cell reactivity.
**Fig. 6**
   The multiple HLA class I binding peptides that define the HPV vaccine specific T cell response set of 2 patients. **A**: Four HPV antigens in the HPV vaccine. Boxes represent the length of the amino acid sequences from the N terminus to the C terminus. **B:** Process to identify the multiple HLA binding peptides of two patients: HLA sequences of the patients labelled as 4-digit HLA genotype right from the patient's ID. The location of the 1^{st} amino acid of the 54 and 91 epitopes that can bind to the patient 12-11 and patient 14-5 HLAs (PEPI1+) respectively are depicted with lines. PEPI2 represents the peptides selected from PEPI1+s that can bind to multiple HLAs of a patient (PEPI2+). PEPI3 represent peptides that can bind to ≧3 HLAs of a patient (PEPI3+). PEPI4 represent peptides that can bind to ≧4 HLAs of a patient (PEPI4+). PEPI5 represent peptides that can bind to ≧5 HLAs of a patient (PEPI5+). PEPI6 represent peptides that can bind to 6 HLAs of a patient (PEPI6). C: The DNA vaccine specific PEPI3+ set of two patients characterizes their vaccine specific T cell responses.
**Fig. 7**
**Fig. 8**
   Correlation between the ≧1 PEPI3+ Score and CTL response rates of peptide targets determined in clinical trials.
   Correlation between the ≧1 PEPI3+ Score and the clinical Immune Response Rate (IRR) of immunotherapy vaccines. Dashed lines: 95% confidence band.
**Fig 9**
   Correlation between the ≧2 PEPI3+ Score and Disease Control Rate (DCR) of immunotherapy vaccines. Dashed lines: 95% confidence band.
**Fig. 10**
   The IPI-responder HLA Test. Overall Survival (OS) of melanoma patients treated with Ipilimumab. Data of 4 independent clinical trials: HLA responders (black line) and HLA non responders (gray line). Statistical analysis: Cox Proportional Hazards Survival Regression. **A**: Trial 1: 18 HLA responders and 30 HLA non responders; **B:** Trial 2: 24 HLA responders and 20 HLA non responders; **C:** Trial 3: 6 HLA responders and 11 HLA non responders; D: Trial 4: 13 HLA responders and 38 HLA non responders
**Fig. 11**
   Multiple HLA binding peptides in mutational neoantigens. **A**: Correlation of mutational load, neoantigen load (neoantigens are neoepitopes according to van Allen) and **B:** Correlation of PEPI3+ load and clinical benefit (min-Q1-median-Q3-max).
**Fig. 12**
   Probability of vaccine antigen expression in the XYZ patient's tumor cells. There is over 95% probability that 5 out of the 12 target antigens in the vaccine regimen is expressed in the patient's tumor. Consequently, the 12 peptide vaccines together can induce immune responses against at least 5 ovarian cancer antigens with 95% probability (AGP95). It has 84% probability that each peptide will induce immune responses in the XYZ patient. AGP50 is the mean (expected value) =7.34 (it is a measure of the effectiveness of the vaccine in attacking the tumor of XYZ patient)
**Fig. 13**
   Probability of vaccine antigen expression in the ABC patient's tumor cells. There is over 95% probability that 4 out of the 13 target antigens in the vaccine is expressed in the patient's tumor. Consequently, the 12 peptide vaccines together can induce immune responses against at least 4 breast cancer antigens with 95% probability (AGP95). It has 84% probability that each peptide will induce immune responses in the ABC patient. AGP50 is the mean (expected value) of the discrete probability distribution = 6.64 (it is a measure of the effectiveness of the vaccine in attacking the tumor of ABC patient).

### Detailed Description

### HLA Genotypes

HLAs are encoded by the most polymorphic genes of the human genome. Each person has a maternal and a paternal allele for the three HLA class I molecules (HLA-A*, HLA-B*, HLA-C*) and four HLA class II molecules (HLA-DP*, HLA-DQ*, HLA-DRB1*, HLA-DRB3*/4*/5*). Practically, each person expresses a different combination of 6 HLA class I and 8 HLA class II molecules that present different epitopes from the same protein antigen. The function of HLA molecules is to regulate T cell responses. However up to date it was unknown how the HLAs of a person regulate T cell activation.

The nomenclature used to designate the amino acid sequence of the HLA molecule is as follows: gene name*allele:protein number, which, for instance, can look like: HLA-A*02:25. In this example, "02" refers to the allele. In most instances, alleles are defined by serotypes-meaning that the proteins of a given allele will not react with each other in serological assays. Protein numbers ("25" in the example above) are assigned consecutively as the protein is discovered. A new protein number is assigned for any protein with a different amino acid sequence (e.g. even a one amino acid change in sequence is considered a different protein number). Further information on the nucleic acid sequence of a given locus may be appended to the HLA nomenclature, but such information is not required for the methods described herein.

The HLA class I genotype or HLA class II genotype of an individual may refer to the actual amino acid sequence of each class I or class II HLA of an individual, or may refer to the nomenclature, as described above, that designates, minimally, the allele and protein number of each HLA gene. An HLA genotype may be determined using any suitable method. For example, the sequence may be determined via sequencing the HLA gene loci using methods and protocols known in the art. Alternatively, the HLA set of an individual may be stored in a database and accessed using methods known in the art.

### Polypeptide Antigens

Described herein is a method of identifying a fragment of a polypeptide as immunogenic for a specific human subject. As used herein, the term "polypeptide" refers to a full-length protein, a portion of a protein, or a peptide characterized as a string of amino acids. As used herein, the term "peptide" refers to a short polypeptide comprising between 2, or 3, or 4, or 5 or 6 or 7 or 8 or 9 and 20, or 25, or 30, or 35, or 40, or 45, or 50 amino acids.

The terms "fragment" or "fragment of a polypeptide" as used herein refer to a string of amino acids or an amino acid sequence of reduced length relative to the reference polypeptide and comprising, over the common portion, an amino acid sequence identical to the reference polypeptide. Such a fragment according to the invention may be, where appropriate, included in a larger polypeptide of which it is a constituent.

In some cases the polypeptide is expressed by a pathogenic organism (for example, a bacteria or a parasite), a virus, or a cancer cell, is associated with an autoimmune disorder or response or a disease-associated cell, or is an allergen, or an ingredient of a medicine such as a vaccine or immunotherapy composition.

The polypeptide may be expressed in the cells of the subject (e.g. a tumor-associated antigen, a polypeptide expressed by a virus, intracellular bacteria or parasite, or the *in vivo* product of a vaccine or immunotherapy composition) or acquired from the environment (e.g. a food, an allergen or a drug). The polypeptide may be present in a sample taken from the specific human subject. Both polypeptide antigens and HLAs can be exactly defined by amino acid or nucleotide sequences and sequenced using methods known in the art.

The polypeptide may be a cancer- or tumor-associated antigen (TAA). TAAs are proteins expressed in cancer or tumor cells. Examples of TAAs include new antigens (neoantigens) expressed during tumorigenesis, products of oncogenes and tumor suppressor genes, overexpressed or aberrantly expressed cellular proteins (e.g. HER2, MUC1), antigens produced by oncogenic viruses (e.g. EBV, HPV, HCV, HBV, HTLV), cancer testis antigens (CTA), (e.g. MAGE family, NY-ESO) and cell-type-specific differentiation antigens (e.g. MART-1). TAA sequences may be found experimentally, or in published scientific papers, or through publicly available databases, such as the database of the Ludwig Institute for Cancer Research (www.cta.lncc.br/), Cancer Immunity database (cancerimmunity.org/peptide/) and the TANTIGEN Tumor T cell antigen database (cvc.dfci.harvard.edu/tadb/).

In some cases the polypeptide is an antigen that is not expressed or is minimally expressed in normal healthy cells or tissues, but is expressed in a high proportion of (with a high frequency in) subjects having a type of cancer or a cancer derived from a particular cell type or tissue, for example breast cancer, ovarian cancer or melanoma. In some cases the polypeptide is expressed in at least 2%, 5%, 10%, 20%, 30%, 40%, 50%, 60%, 70, 80%, 90% or more of such individuals, or of a subject-matched human subpopulation.

In some cases the polypeptide is a cancer testis antigens (CTA. CTA are not typically expressed beyond embryonic development in healthy cells. In healthy adults, CTA expression is limited to male germ cells that do not express HLAs and cannot present antigens to T cells. Therefore, CTAs are considered expressional neoantigens when expressed in cancer cells. CTA expression is (i) specific for tumor cells, (ii) more frequent in metastases than in primary tumors and (iii) conserved among metastases of the same patient (Gajewski ed. Targeted Therapeutics in Melanoma. Springer New York. 2012).

Typically some or all of the tumor specific T cell clones that are induced by a tumor are inactive or poorly functional in metastatic cancer patients. Inactive tumor specific T cells cannot kill the tumor cells. A fraction of these inactive T cells may be re-activated by checkpoint inhibitors, for example monoclonal antibodies that recognize checkpoint molecules (e.g. CTLA-4, PD-1). In some cases, the methods of the invention may be used to identify one or more or the subset of T cell clones that may be reactivated by a checkpoint inhibitor or to predict likely responders to checkpoint inhibitor immunotherapies.

The polypeptide may be a mutational neoantigen, which is expressed by a cell, for example a cancer cell, of the individual, but altered from the analogous protein in a normal or healthy cell. Mutational neoantigens can be used to target disease-associated cells that express the neoantigen. Mutations in a polypeptide expressed by a cell, for example a cell in a sample taken from a subject, can be detected by, for example, sequencing, but the majority do not induce an immune response against the neoantigen-expressing cells. Currently, the identification of mutational neoantigens that do induce an immune response is based on prediction of mutational HLA restricted epitopes and further *in vitro* testing of the immunogenicity of predicted epitopes in individual's blood specimen. This process is inaccurate, long and expensive. The inventors have shown that the identification of mutational epitopes that bind to multiple HLA molecules reproducibly define the immunogenicity of mutational neoantigens. Therefore, in some cases in accordance with the invention, the polypeptide is a mutational neoantigen, and the immunogenic fragment of the polypeptide comprises a neoantigen specific mutation.

The polypeptide may be a viral protein that is expressed intracellularly. Examples include HPV16 E6, E7; HIV Tat, Rev, Gag, Pol, Env; HTLV-Tax, Rex, Gag, Env, Human herpes virus proteins, Dengue virus proteins. The polypeptide may be a parasite protein that is expressed intracellularly, for example malaria proteins.

The polypeptide may be an active ingredient of a vaccine or immunotherapy composition. The term "active ingredient" as used herein refers to a polypeptide that is intended to induce an immune response and may include a polypeptide product of a vaccine or immunotherapy composition that is produced *in vivo* after administration to a subject. For a DNA or RNA vaccine or immunotherapy composition, the polypeptide may be produced *in vivo* by the cells of a subject to whom the composition is administered. For a cell-based vaccine or immunotherapy composition, the polypeptide may be processed and/or presented by cells of the composition, for example autologous dendritic cells or antigen presenting cells pulsed with the polypeptide or comprising an expression construct encoding the polypeptide.

Several drugs made from TAAs, CTAs and viral oncogenes (see Table 1, Table 2 and Table 3) are under clinical investigations. Table 4 illustrates some of the most studied TAAs in the past few years in research and clinical studies. Presently, >200 clinical trials are investigating cancer vaccines with tumor antigens.

The differences among HLAs influence the probability of developing autoimmune diseases. In some cases the method of the invention may be used to identify a polypeptide or a fragment of a polypeptide that is associated with an auto-immune disorder or response. In some cases, the method comprises determining whether a polypeptide comprises a sequence that is a T cell epitope capable of binding to at least three, or at least four, or at least five HLA class I of a subject; or a sequence that is a T cell epitope capable of binding to at least four, or at least five, or at least six HLA class II of a subject; and identifying said sequence as related to an auto-immune disorder or an auto-immune response in the subject.

The differences among HLAs also influence the probability that a subject will experience immune-toxicity from a drug. Therefore, in some cases in accordance with the invention, the polypeptide is an active ingredient of a vaccine or immunotherapy composition and the method is used to identify a toxic immunogenic region of the polypeptide or to identify subjects who are likely to experience immune-toxicity in response to the polypeptide or a fragment thereof. In some cases the method comprises determining whether a polypeptide comprises a sequence that is a T cell epitope capable of binding to at least three, or at least four, or at least five HLA class I of a subject; or a sequence that is a T cell epitope capable of binding to at least four, or at least five, or at least six HLA class II of a subject; and identifying said sequence as toxic immunogenic for the subject.

The polypeptide may be an allergen that enters the body of an individual through, for example, the skin, lung or oral routes.

Non-limiting examples of suitable polypeptides include those listed in one or more of the Tables herein.

Genetic sequences may be obtained from the sequencing of biological materials. Sequencing can be done by any suitable method that determines DNA and/or RNA and/or amino acid sequences. The disclosure utilizes both the HLA genotypes and amino acid sequences. However, methods to identify HLA genotype from genetic sequences of an individual and methods of obtaining amino acid sequences derived from DNA or RNA sequence data are not the subject of the disclosure.

**Table 1. Selected tumor antigens**

| 5T4 | BHRF1 | CDCA1 | EEF2 | Ghrelin | KMT2D | MOB3B | PCM1 | RSL1D1 | TEKT5 |
|---|---|---|---|---|---|---|---|---|---|
| A1BG | BILF1 | CDCP1 | EFNA1 | GHSR | KOC1 | MORC1 | PCNXL2 | RTKN | TEX101 |
| A33 | BILF2 | CDH3 | EFS | GIPC1 | K-ras | MPHOSP H1 | PDGFB | RUNX1 | TEX14 |
| A4GALT | BIN1 | CDK2AP1 | EFTUD2 | GITR | KRIT1 | MPL | PDGFRA | RUNX2 | TEX15 |
| AACT | BING-4 | CDK4 | EGFL7 | GKAP1 | KU-CT-1 | MRAS | PEPP2 | RYK | TF |
| AAG | BIRC7 | CDK7 | EGFR | GLI1 | KW-12 | MRP1 | PGF | SAGE1 | TFDP3 |
| ABI1 | BLLF1 | CDKN1A | EGFRvIII | Glypican-3 | KW-2 | MRP3 | PGK1 | SART2 | TFE3 |
| ABI2 | BLLF2 | CDKN2A | EI24 | GML | KW-5 (SEBD4) | MRPL28 | PHLDA3 | SART3 | TGFBR1 |
| ABL1 | BMI1 | CEA | EIF4EBP1 | GNA11 | KW-7 | MRPL30 | PHLPP1 | SASH1 | TGFBR2 |
| ABL-BCR | BMLF1 | CEACAM1 | ELF3 | GNAQ | L1CAM | MRPS11 | PIAS1 | sCLU | THEG |
| ABLIM3 | BMPR1B | CENPK | ELF4 | GNB2L1 | L53 | MSLN | PIAS2 | SCP-1 | TIPRL |
| ABLL | BMRF1 | CEP162 | ELOVL4 | GOLGA5 | L6 | MTA1 | PIK3CA | SCRN1 | TLR2 |
| ABTB1 | BNLF2a | CEP290 | EMP1 | gp100 | LAG3 | MTA2 | PIK3CD | SDCBP | TMEFF1 |
| ACACA | BNLF2b | CEP55 | ENAH | gp75 | Lage-1 | MTA3 | PIK3R2 | SDF-1 | TMEFF2 |
| ACBD4 | BNRF1 | CFL1 | Endosialin | Gp96 | LATS1 | MTCP1 | PIM1 | SDHD | TMEM108 |
| ACO1 | BRAF1 | CH3L2 | ENO1 | GPAT2 | LATS2 | MTSS1 | PIM2 | SEC31A | TMEM127 |
| ACRBP | BRD4 | CHEK1 | ENO2 | GPATCH2 | LCMT2 | MUC-1 | PIM3 | SEC63 | TMPRSS1 2 |
| ACTL6A | BRDT | CK2 | ENO3 | GPNMB | LCP1 | MUC-2 | PIR | Semaphorin 4D | TNC |
| ACTL8 | BRI3BP | CLCA2 | ENTPD5 | GPR143 | LDHC | MUC-5AC | PIWIL1 | SEMG1 | TNFRSF1 7 |
| ACTN4 | BRINP1 | CLOCK | EpCAM | GPR89A | LDLR | MUM1 | PIWIL2 | SEPT2 | TNFSF15 |
| ACVR1 | BRLF1 | CLPP | EPHA2 | GRB2 | LEMD1 | MUM2 | PKN3 | SFN | TNK2 |
| ACVR1B | BTBD2 | CMC4 | EPHA3 | GRP78 | Lengsin | MYB | PLA2G16 | SH2B2 | TOMM34 |
| ACVR2B | BUB1B | CML66 | EPHB2 | GUCY1A3 | LETMD1 | MYC | PLAC1 | SH2D1B | TOP2A |
| ACVRL1 | BVRF2 | CO-029 | EPHB6 | H3F3A | LGALS3B P | MYCL | PLAG1 | SH3BP1 | TOP2B |
| ACS2B | BXLF1 | COTL1 | EPS8 | HAGE | LGALS8 | MYCLP1 | PLEKHG5 | SHB | TOR3A |
| ACSL5 | BZLF1 | COX6B2 | ERBB3 | hANP | LIN7A | MYCN | PLK3 | SHC3 | TP73 |
| ADAM-15 | C15orf60 | CPSF1 | ERBB4 | HBEGF | LIPI | MYD88 | PLS3 | SIRT2 | TPA1 |
| ADAM 17 | CA 12-5 | CPXCR1 | EREG | hCG-beta | LIV-1 | MYEOV | PLVAP | SIVA1 | TPGS2 |
| ADAM2 | CA 19-9 | CREBL2 | ERG | HDAC1 | LLGL1 | MYO1B | PLXNB1 | SKI | TPI1 |
| ADAM29 | CA195 | CREG1 | ERVK-18 | HDAC2 | LMO1 | NA17-A | PLXNB2 | SLBP | TPL2 |
| ADAM7 | CA9 | Cripto | ERVK-19 | HDAC3 | LMO2 | NA88-A | PML | SLC22A10 | TPM4 |
| ADAP1 | CABYR | CRISP2 | ESR1 | HDAC4 | LMP1 | NAE1 | PML-RARA | SLC25A47 | TPO |
| ADFP | CADM4 | CRK | ETAA1 | HDAC5 | LMP2 | Napsin-A | POTEA | SLC35A4 | TPPP2 |
| ADGRA3 | CAGE1 | CRKL | ETS1 | HDAC6 | LOXL2 | NAT6 | POTEB | SLC45A3 | TPR |
| ADGRF1 | CALCA | CRLF2 | ETS2 | HDAC7 | LRP1 | NBAS | POTEC | SLC4A1A P | TPTE |
| ADGRF2 | CALR3 | CT45 | ETV1 | HDAC8 | LRRN2 | NBPF12 | POTED | SLCO6A1 | TRAF5 |
| ADGRL2 | CAN | CT45A2 | ETV5 | HDAC9 | LTF | NCOA4 | POTEE | SLITRK6 | TRAG-3 |
| ADHFE1 | CASC3 | CT45A3 | ETV6 | HEATR1 | LTK | NDC80 | POTEG | Sm23 | TRGC2 |
| AEN | CASC5 | CT45A4 | EVI5 | Hepsin | LZTS1 | NDUFC2 | POTEH | SMAD5 | TRIM24 |
| AFF1 | CASP5 | CT45A5 | EWSR1 | Her2/neu | LYN | Nectin-4 | PP2A | SMAD6 | TRIM37 |
| AFF4 | CASP8 | CT46 | EYA2 | HERC2 | MAEA | NEK2 | PPAPDC1 B | SMO | TRIM68 |
| AFP | CBFA2T2 | CT47 | EZH2 | HERV-K104 | MAEL | NEMF | PPFIA1 | Smt3B | TRPM8 |
| AGAP2 | CBFA2T3 | CT47B1 | FABP7 | HEXB | MAF | NENF | PPIG | SNRPD1 | TSGA10 |
| AGO1 | CBL | CTAGE2 | FAM133A | HEXIM1 | MAFF | NEURL1 | PPP2R1B | SOS1 | TSP50 |
| AGO3 | CBLB | cTAGE5 | FAM13A | HGRG8 | MAFG | NFIB | PRAME | SOX-2 | TSPAN6 |
| AGO4 | CC3 | CTCFL | FAM46D | HIPK2 | MAFK | NFKB2 | PRDX5 | SOX-6 | TSPY1 |
| AGR2 | CCDC110 | CTDSP2 | FAM58BP | HJURP | MAGE-A1 | NF-X1 | PRKAA1 | SPA17 | TSPY2 |
| AIFM2 | CCDC33 | CTGF | FANCG | HMGB1 | MAGE-A10 | NFYC | PRKCI | SPACA3 | TSPY3 |
| AIM2 | CCDC36 | CTLA4 | FATE1 | HMOX1 | MAGE-A11 | NGAL | PRM1 | SPAG1 | TSPYL1 |
| AKAP-13 | CCDC6 | CTNNA2 | FBXO39 | HNRPL | MAGE-A12 | NKG2D-L1 | PRM2 | SPAG17 | TSSK6 |
| AKAP-3 | CCDC62 | CTNNB1 | FBXW11 | HOM-TES-85 | MAGE-A2 | NKG2D-L2 | PRMT3 | SPAG4 | TTC23 |
| AKAP-4 | CCDC83 | CTNND1 | FCHSD2 | HORMAD 1 | MAGE-A2B | NKG2D-L3 | PRMT6 | SPAG6 | TTK |
| AKIP1 | CCL13 | CTSH | FER | HORMAD 2 | MAGE-A3 | NKG2D-L4 | PDL1 | SPAG8 | TULP2 |
| AKT1 | CCL2 | CTTN | FES | HPSE | MAGE-A4 | NLGN4X | PROM1 | SPAG9 | TUSC2 |
| AKT2 | CCL7 | CXCR4 | FEV | HPV16 E6 | MAGE-A5 | NLRP4 | PRSS54 | SPANXA1 | TWEAK |
| AKT3 | CCNA1 | CXorf48 | FGF10 | HPV16 E7 | MAGE-A6 | NNMT | PRSS55 | Span-Xb | TXNIP |
| ALDH1A1 | CCNA2 | CXorf61 | FGF23 | HPV18 E6 | MAGE-A8 | NOL4 | PRTN3 | SPAN-Xc | TYMS |
| ALK | CCNB1 | CYP1 B1 | FGF3 | HPV18 E7 | MAGE-A9 | NOTCH2 | PRUNE | SPANXD | TYR |
| ALKBH1 | CCND1 | CypB | FGF4 | HRAS | MAGE-B1 | NOTCH3 | PRUNE2 | SPANXE | U2 snRNP B |
| ALPK1 | CCNE2 | CYR61 | FGF5 | HSD17B1 3 | MAGE-B2 | NOTCH4 | PSA | SPANXN1 | U2AF1 |
| AMIGO2 | CCNI | CS1 | FGFR1 | HSP105 | MAGE-B3 | NOV | PSCA | SPANXN2 | UBD |
| ANG2 | CCNL1 | CSAG1 | FGFR2 | HSP60 | MAGE-B4 | NPM1 | PSMA | SPANXN3 | UBE2A |
| ANKRD45 | CCR2 | CSDE1 | FGFR3 | HSPA1A | MAGE-B5 | NR6A1 | PSMD10 | SPANXN4 | UBE2V1 |
| ANO1 | CD105 | CSF1 | FGFR4 | HSPB9 | MAGE-B6 | N-RAS | PSP-94 | SPANXN5 | UBE4B |
| ANP32A | CD123 | CSF1R | FGR | HST-2 | MAGE-C1 | NRCAM | PTEN | SPATA19 | UBR5 |
| ANXA2 | CD13 | CSF3R | FLI1 | HT001 | MAGE-C2 | NRP1 | PTH-rP | SPEF2 | UBXD5 |
| APC | CD133 | CSK | FLT3 | hTERT | MAGE-C3 | NSE1 | PTK6 | SPI1 | UFL1 |
| APEH | CD137 | CSK23 | FMNL1 | HUS1 | mammagl obin-A | NSE2 | PTPN20A | SPINLW1 | URI1 |
| APOA2 | CD138 | DAPK3 | FMOD | IDH1 | MANF | NTRK1 | PTPRK | SPO11 | URLC10 |
| APOD | CD157 | DAZ1 | FMR1NB | IDO1 | MAP2K2 | NUAK1 | PTPRZ | SRC | UROC1 |
| APOL1 | CD16A | DCAF12 | FN1 | IER3 | MAP2K7 | NUGGC | PTTG-1 | SSPN | USP2 |
| AR | CD178 | DCT | Fn14 | IGF1R | MAP3K7 | NXF2 | PTTG2 | SSX-1 | USP4 |
| ARAF | CD19 | DCUN1D1 | FNIP2 | IGFS11 | MAP4K5 | NXF2B | PTTG3 | SSX-2 | VAV1 |
| ARF4L | CD194 | DCUN1D3 | FOLR1 | IL13RA2 | MART1 | NY-BR-1 | PXDNL | SSX-3 | VEGFR1 |
| ARHGEF5 | CD2 | DDR1 | FOS | IMP-3 | MART-2 | NY-ESO-1 | RAB11 FIP 3 | SSX-4 | VEGFR2 |
| ARID3A | CD20 | DDX3X | FosB | ING3 | MAS1 | NY-MEL-1 | RAB8A | SSX-5 | VHL |
| ARID4A | CD21 | DDX43 | FOSL1 | INPPL1 | MC1R | OCA2 | RAD1 | SSX-6 | VIM |
| ARL6IP5 | CD22 | DDX6 | FOXM1 | INTS6 | MCAK | ODF1 | RAD17 | SSX-7 | VWA5A |
| ARMC3 | CD229 | DEDD | FOXO1 | IRF4 | MCF2 | ODF2 | RAD51C | SSX-9 | WHSC2 |
| ARMC8 | CD23 | DEK | FOXO3 | IRS4 | MCF2L | ODF3 | RAF1 | ST18 | WISP1 |
| ARTC1 | CD27 | DENR | FRAT1 | ITGA5 | MCL1 | ODF4 | RAGE-1 | STAT1 | WNK2 |
| ARX | CD28 | DEPDC1 | FRMD3 | ITGB8 | MCTS1 | OGG1 | RAP1A | STEAP1 | WNT10B |
| ATAD2 | CD30 | DFNA5 | FSTL3 | ITPA | MCSP | OGT | RARA | STK11 | WNT3 |
| ATIC | CD317 | DGAT2 | FTHL17 | ITPR2 | MDM2 | OIP5 | RASSF10 | STK25 | WNT-5a |
| AURKC | CD33 | DHFR | FUNDC2 | JAK2 | MDM4 | OS9 | RB1 | STK3 | WT1 |
| AXIN1 | CD350 | DKK1 | FUS | JAK3 | ME1 | OTOA | RBL2 | STN | WWP1 |
| AXL | CD36 | DKK3 | FUT1 | JARID1B | ME491 | OX40 | RBM46 | SUPT7L | XAGE-1 |
| BAAT | CD37 | DKKL1 | FUT3 | JAZF1 | MECOM | OX40L | RBP4 | Survivin | XAGE-2 |
| BAFF | CD4 | DLEU1 | FYN | JNK1 | MELK | P53 | RCAS1 | SUV39H1 | XAGE-3 |
| BAGE-1 | CD40 | DMBT1 | GAB2 | JNK2 | MEN1 | P56-LCK | RCVRN | SYCE1 | XAGE-4 |
| BAGE-2 | CD40L | DMRT1 | GADD45G | JNK3 | MERTK | PA2G4 | RECQL4 | SYCP1 | XAGE-5 |
| BAGE-3 | CD45 | DNAJB8 | GAGE-1 | JTB | MET | PAGE1 | RET | SYT | XBP1 |
| BAGE-4 | CD47 | DNAJC8 | GAGE12B /C/D/E | JUN | MFGE8 | PAGE2 | RGS22 | TA-4 | XPO1 |
| BAGE-5 | CD51 | DNMT3A | GAGE12F | JUP | MFHAS1 | PAGE2B | RGS5 | TACC1 | XRCC3 |
| BAI1 | CD52 | DPPA2 | GAGE12G | K19 | MFI2 | PAGE3 | RHAMM | TAF1B | YB-1 |
| BAL | CD55 | DR4 | GAGE12H | KAAG1 | MGAT5 | PAGE4 | RhoC | TAF4 | YEATS4 |
| BALF2 | CD61 | DR5 | GAGE12I | Kallikrein 4 | Midkine | PAGE5 | RL31 | TAF7L | YES1 |
| BALF4 | CD70 | DRG1 | GAGE12J | KAT6A | MIF | PAK2 | RNASET2 | TAG-1 | YKL-40 |
| BALF5 | CD74 | DSCR8 | GAGE-2 | KDM1A | MKI67 | PANO1 | RNF43 | TAL1 | ZBTB7A |
| BARF1 | CD75 | E2F3 | GAGE-3 | KDM5A | MLH1 | PAP | RNF8 | TAL2 | ZBTB7C |
| BBRF1 | CD79B | E2F6 | GAGE-5 | KIAA0100 | MLL | PAPOLG | RON | TAPBP | ZEB1 |
| BCAN | CD80 | E2F8 | GAGE-6 | KIAA0336 | MLLT1 | PARK2 | Ropporin-1A | TATI | ZFYVE19 |
| BCAP31 | CD86 | EBNA1 | GAGE-7 | KIAA1199 | MLLT10 | PARK7 | ROR1 | TAX1BP3 | ZNF165 |
| BCL-2 | CD8a | EBNA2 | GAGE-8 | KIAA1641 | MLLT11 | PARP12 | RPA1 | TBC1D3 | ZNF185 |
| BCL2L1 | CD8b | EBNA3 | GALGT2 | KIF11 | MLLT3 | PASD1 | RPL10A | TBP-1 | ZNF217 |
| BCL6 | CD95 | EBNA4 | GAS7 | KIF1B | MLLT4 | PAX3 | RPL7A | TCL1A | ZNF320 |
| BCL9 | CD98 | EBNA6 | GATA-3 | KIF20A | MLLT6 | PAX5 | RPS2 | TCL1 B | ZNF395 |
| BCR | CDC123 | EBNA-LP=EBNA 5 | GBU4-5 | KIT | MMP14 | PBF | RPS6KA5 | TDHP | ZNF645 |
| BCRF1 | CDC2 | ECT2 | GCDFP-15 | KLF4 | MMP2 | PBK | RPSA | TDRD1 | ZUBR1 |
| BDLF3 | CDC27 | ECTL2 | GFAP | KLHL41 | MMP7 | PBX1 | RQCD1 | TDRD4 | ZW10 |
| BGLF4 | CDC73 | EDAG | GFI1 | KLK10 | MMP9 | PCDC1 | RRAS2 | TDRD6 | ZWINT |
| BHLF1 | | | | | | | | | |

| | | | |
|---|---|---|---|
| **DISEASE** | **SOURCE OF DISEASE** | **MAIN TARGETS** | **REF** |

**Table 2. Selected viral antigens.**

| | | | |
|---|---|---|---|
| AIDS (Acquired immunodeficiency syndrome) | HIV (Human immunodeficiency virus) | gag, env, pol, nef, tat, rev | A plasmid DNA immunogen expressing fifteen protein antigens and complex virus-like particles (VLP+) mimicking naturally occurring HIV. Somogyi E, Xu J, Gudics A, Toth J, Kovacs AL, Lori F, Lisziewicz J. Vaccine. 2011; 29(4):744-53. |
| Argentine hemorrhagic fever | Junin virus | capsular glycoprotein G38 | http://nihbrp.com/Citations/completed/HumanHealthEcologyTeam/Juni nVirus/Enria_JuninVirus_MicrobiolImmun_2002.pdf |
| Astrovirus infection | Astroviridae family | capsid protein VP29NP26 | Bass DM, Upadhyayula U. Characterization of human serotype 1 astrovirus-neutralizing epitopes. J Virol. 1997; 71(11):8666-71. |
| BK virus infection | BK virus | T-antigen | Li J, Melenhorst J, Hensel N, Rezvani K, Sconocchia G, Kilical Y, Hou J, Curfman B, Major E, Barrett AJ. T-cell responses to peptide fragments of the BK virus T antigen: implications for cross-reactivity of immune response to JC virus. J Gen Virol. 2006; 87(Pt 10):2951-60. |
| Bolivian hemorrhagic fever | Machupo virus | cell surface antigens G1 and G2, nuclear protein (NP) | Evans AS, Kaslow RA (ed.) Viral Infections of Humans: Epidemiology and Control. Part II. Springer Science & Business Media, 1997. |
| Brazilian hemorrhagic fever | Sabia | GPC, L, NP, and Z proteins | Kotturi MF, Botten J, Maybeno M, Sidney J, Glenn J, Bui HH, Oseroff C, Crotty S, Peters B, Grey H, Altmann DM, Buchmeier MJ, Sette A. Polyfunctional CD4+ T cell responses to a set of pathogenic arenaviruses provide broad population coverage. Immunome Res. 2010; 6:4. |
| Calicivirus infection (Norovirus and Sapovirus) | Caliciviridae family | VP1 - the major capsid protein and VP2 - the minor capsid protein | Debbink K, Lindesmith LC, Donaldson EF, Baric RS. Norovirus immunity and the great escape. PLoS Pathog. 2012; 8(10):e1002921. |
| Chickenpox, Shingles (Herpes zoster) | Varicella zoster virus (VZV) | ORF4-Transcriptional activator, ORF10 - Transactivator of immediate early genes, ORF 14 - Glycoprotein C, ORF31-Glycoprotein B, ORF62-Transcriptional regulator, ORF67 - Glycoprotein I, ORF68-Glycoprotein E, | Milikan JC, Kinchington PR, Baarsma GS, Kuijpers RW, Osterhaus AD, Verjans GM. Identification of viral antigens recognized by ocular infiltrating T cells from patients with varicella zoster virus-induced uveitis. Invest Ophthalmol Vis Sci. 2007; 48(8):3689-97. |
| Chikungunya | Alphavirus | Structural proteins E1, E2 and E3 | Vénien-Bryan C, Fuller SD. The organization of the spike complex of Semliki Forest virus. J Mol Biol. 1994; 236(2):572-83. |
| Colorado tick fever (CTF) | Colorado tick fever virus (CTFV) | coltivirus: structural proteins λ1, λ2, λ3, µl, µ2, σ1, σ2, or σ3, or nonstructural proteins σNS, µNS, or σls | Mucosal and systemic immunizations with alphavirus replicon particles. WO2007047749A1. 2005. Inventor: Michael Vajdy. |
| Common cold (Acute viral rhinopharyngitis Acute coryza) | rhinoviruses | capsid proteins: VP1, VP2, VP3 and VP4 | Smith TJ, Kremer MJ, Luo M, Vriend G, Arnold E, Kamer G, Rossmann MG, McKinlay MA, Diana GD, Otto MJ. The site of attachment in human rhinovirus 14 for antiviral agents that inhibit uncoating. Science. 1986 Sep; 233(4770):1286-93. |
| | coronaviruses | spike (S) protein, envelope (E) protein, membrane (M) protein, and nuclepcapsid (N) protein | Li SW, Lin CW. Human coronaviruses: Clinical features and phylogenetic analysis. Biomed. 2013; 3:43-50. |
| Crimean-Congo hemorrhagic fever (CCHF) | Crimean-Congo hemorrhagic fever virus | structural glycoproteins Gn and Gc. | Bergeron E, Vincent MJ, Nichol ST. Crimean-Congo hemorrhagic fever virus glycoprotein processing by the endoprotease SKI-1/S1P is critical for virus infectivity. J Virol. 2007; 81(23):13271-6. |
| Cytomegaloviru s infection | Cytomegaloviru s | pp65 (immune evasion), pp71 (gene expression), and pp150 and pp28 (assembly and egress) | Tomtishen JP 3rd. Human cytomegalovirus tegument proteins (pp65, pp71, pp150, pp28). Virol J. 2012; 9:22 |
| Dengue fever | Dengue viruses (DEN-1, DEN-2, DEN-3 and DEN-4)-Flaviviruses | Capsid, prM, and Envelope proteins | D. Brian Thomas (ed.) Viruses and the Cellular Immune Response. Section II. Flavivirus biology. CRC Press, 1993. |
| Ebola hemorrhagic fever | Ebolavirus (EBOV) | GP1 (glycoprotein) | Phoolcharoen W, Dye JM, Kilbourne J, Piensook K, Pratt WD, Arntzen CJ, Chen Q, Mason HS, Herbst-Kralovetz MM. A nonreplicating subunit vaccine protects mice against lethal Ebola virus challenge. Proc Natl Acad Sci U S A. 2011; 108(51):20695-700. |
| | | nucleoprotein (NP) | Tsuda Y, Caposio P, Parkins CJ, Botto S, Messaoudi I, Cicin-Sain L, Feldmann H, Jarvis MA. A replicating cytomegalovirus-based vaccine encoding a single Ebola virus nucleoprotein CTL epitope confers protection against Ebola virus. PLoS Negl Trop Dis. 2011; 5(8):e1275. |
| Enterovirus infection, Hand, foot and mouth disease (HFMD) | Enterovirus genus | structural proteins VP1, VP2, VP3, VP4 | Henke A, Zell R, Stelzner A. DNA vaccine-mediated immune responses in Coxsackie virus B3-infected mice. Antiviral Res. 2001; 49(1):49-54. |
| Erythema infectiosum (Fifth disease) | Parvovirus B19 | VP1 and VP2 capsid proteins | Bernstein DI, EI Sahly HM, Keitel WA, Wolff M, Simone G, Segawa C, Wong S, Shelly D, Young NS, Dempsey W. Safety and immunogenicity of a candidate parvovirus B19 vaccine. Vaccine. 2011; 29(43):7357-63. |
| Exanthem subitum (Sixth disease) | Human herpesvirus 6 (HHV-6) and Human herpesvirus 7 (HHV-7) | U24 protein (HHV-6) | Downregulation of the T-cell receptor complex and impairment of T-cell activation by human herpesvirus 6 u24 protein. Sullivan BM, Coscov L. J Virol. 2008; 82(2):602-8. |
| | | pp85 (HHV-7) | Stefan A, Secchiero P, Baechi T, Kempf W, Campadelli-Fiume G. The 85-kilodalton phosphoprotein (pp85) of human herpesvirus 7 is encoded by open reading frame U14 and localizes to a tegument substructure in virion particles. J Virol. 1997; 71(8):5758-63. |
| Hantavirus Pulmonary Syndrome (HPS) | Sin Nombre virus | M segment (full length M-gene) | Hooper JW, Josleyn M, Ballantyne J, Brocato R. A novel Sin Nombre virus DNA vaccine and its inclusion in a candidate panhantavirus vaccine against hantavirus pulmonary syndrome (HPS) and hemorrhagic fever with renal syndrome (HFRS). Vaccine. 2013; 31(40):4314-21. |
| Hemorrhagic fever with renal syndrome (HFRS) | Hantaan virus (Bunyaviridae family) | nucleoprotein | Ma Y, Wang J, Yuan B, Wang M, Zhang Y, Xu Z, Zhang C, Zhang Y, Liu B, Yi J, Yang K, Yang A, Zhuang R, Jin B. HLA-A2 and B35 restricted hantaan virus nucleoprotein CD8+ T-cell epitope-specific immune response correlates with milder disease in hemorrhagic fever with renal syndrome. PLoS Negl Trop Dis. 2013;7(2):e2076. |
| Hepatitis A | Hepatitis A Virus | VP1 protein | Schulte I, Hitziger T, Giugliano S, Timm J, Gold H, Heinemann FM, Khudyakov Y, Strasser M, König C, Castermans E, Mok JY, van Esch WJ, Bertoletti A, Schumacher TN, Roggendorf M. Characterization of CD8+ T-cell response in acute and resolved hepatitis A virus infection. J Hepatol. 2011;54(2):201-8. |
| Hepatitis B | Hepatitis B Virus | X, surface (S), and Core antigens (X-SCore) | King TH, Kemmler CB, Guo Z, Mann D, Lu Y, Coeshott C, Gehring AJ, Bertoletti A, Ho ZZ3, Delaney W, Gaggar A, Subramanian GM, McHutchison JG, Shrivastava S, Lee YJ, Kottilil S, Bellgrau D, Rodell T, Apelian D. A whole recombinant yeast-based therapeutic vaccine elicits HBV x, s and core specific T cells in mice and activates human T cells recognizing epitopes linked to viral clearance. PLoS One. 2014; 9(7):e101904. |
| Hepatitis C | Hepatitis C Virus | non-structural proteins (P7, NS2, NS3, NS4A, NS4B, NS5A, and NS5B) | Differential antigenic hierarchy associated with spontaneous recovery from hepatitis C virus infection: implications for vaccine design. Smyk-Pearson S, Tester IA, Lezotte D, Sasaki AW, Lewinsohn DM, Rosen HR. J Infect Dis. 2006; 194(4):454-63. |
| Hepatitis D | Hepatitis D Virus (occurs together with HBV) | p24 and p27 proteins | Fiedler M, Kosinska A, Schumann A, Brovko O, Walker A, Lu M, Johrden L, Mayer A, Wildner O, Roggendorf M. J Virol. 2013; 87(13):7708-16. Prime/boost immunization with DNA and adenoviral vectors protects from hepatitis D virus (HDV) infection after simultaneous infection with HDV and woodchuck hepatitis virus. |
| Hepatitis E | Hepatitis E Virus | capsid protein precursor (ORF2) and ORF3 protein | Suneetha PV, Pischke S, Schlaphoff V, Grabowski J, Fytili P, Gronert A, Bremer B, Markova A, Jaroszewicz J, Bara C, Manns MP, Cornberg M, Wedemeyer H. Hepatitis E virus (HEV)-specific T-cell responses are associated with control of HEV infection. Hepatology. 2012; 55(3):695-708. |
| Herpes simplex | Herpes simplex virus 1 and 2 (HSV-1 and HSV-2) | envelope protein glycoprotein D (gD2) | Dutton JL, Li B, Woo WP, Marshak JO, Xu Y, Huang ML, Dong L, Frazer IH, Koelle DM. A novel DNA vaccine technology conveying protection against a lethal herpes simplex viral challenge in mice.PLoS One. 2013; 8(10):e76407. |
| | | tegument proteins VP11/12(UL46), VP13/14(UL47) | Veselenak RL, Shlapobersky M, Pyles RB, Wei Q, Sullivan SM, Bourne N. A Vaxfectin(®)-adjuvanted HSV-2 plasmid DNA vaccine is effective for prophylactic and therapeutic use in the guinea pig model of genital herpes. Vaccine. 2012; 30(49):7046-51. |
| Human bocavirus infection | Human bocavirus (HBoV) | major capsid protein (VP2) | Deng ZH, Hao YX, Yao LH, Xie ZP, Gao HC, Xie LY, Zhong LL, Zhang B, Cao YD, Duan ZJ. Immunogenicity of recombinant human bocavirus-1,2 VP2 gene virus-like particles in mice. Immunology. 2014; 142(1):58-66. |
| Human metapneumovir us infection | Human metapneumovir us (hMPV) | fusion (F) protein, glycoprotein (G), small hydrophobic (SH) protein | Cox RG, Erickson JJ, Hastings AK, Becker JC, Johnson M, Craven RE, Tollefson SJ, Boyd KL, Williams JV. Human metapneumovirus virus-like particles induce protective B and T cell responses in a mouse model. J Virol. 2014 ;88(11):6368-79. |
| Human papillomavirus (HPV) infection | Human papillomavirus (HPV) | E6, E7 and L2 | Peng S, Song L, Knoff J, Wang JW, Chang YN, Hannaman D, Wu TC, Alvarez RD, Roden RB, Hung CF. Control of HPV-associated tumors by innovative therapeutic HPV DNA vaccine in the absence of CD4+ T cells.Cell Biosci. 2014; 4(1):11. |
| | | L1 capsid proteins | Siddiqui MA, Perry CM. Human papillomavirus quadrivalent (types 6, 11, 16, 18) recombinant vaccine (Gardasil). Drugs. 2006; 66(9):1263-71; discussion 1272-3. |
| | | | Crosbie EJ, Kitchener HC. Cervarix--a bivalent L1 virus-like particle vaccine for prevention of human papillomavirus type 16- and 18-associated cervical cancer. Expert Opin Biol Ther. 2007; 7(3):391-6. |
| Human parainfluenza virus infection | Human parainfluenza viruses (HPIV) | Hemagglutininneuraminidase (HN), Matrix Protein (M), Nucleoprotein (NP), Phosphoprotein (P) | Dave VP, Allan JE, Slobod KS, Smith FS, Ryan KW, Takimoto T, Power UF, Portner A, Hurwitz JL. Virology. 1994; 199(2):376-83. Viral cross-reactivity and antigenic determinants recognized by human parainfluenza virus type 1-specific cytotoxic T-cells. |
| Epstein-Barr Virus Infectious Mononucleosis (Mono) | Epstein-Barr Virus (EBV) | BZLF1, BRLF1, BMRF1, BMLF1, BALF2, BALF5, BGLF4, EBNA1, EBNA2, EBNA3A, EBNA3B, EBNA3C, EBNA-LP, LMP2 | Hislop AD, Taylor GS, Sauce D, Rickinson AB. Cellular responses to viral infection in humans: lessons from Epstein-Barr virus. Annu Rev Immunol. 2007; 25:587-617. |
| Influenza (flu) | Influenza A virus | nucleoprotein (NP) | Grant E, Wu C, Chan KF, Eckle S, Bharadwaj M, Zou QM, Kedzierska K, Chen W. Immunol Cell Biol. 2013; 91(2):184-94. Nucleoprotein of influenza A virus is a major target of immunodominant CD8+ T-cell responses. |
| | | hemagglutinin and matrix 1 proteins | Hemann EA, Kang SM, Legge KL. Protective CD8 T cell-mediated immunity against influenza A virus infection following influenza virus-like particle vaccination. J Immunol. 2013; 191(5):2486-94. |
| | Influenza B virus | hemagglutinin (HA) | Pushko P, Pearce MB, Ahmad A, Tretyakova I, Smith G, Belser JA, Tumpey TM. Influenza virus-like particle can accommodate multiple subtypes of hemagglutinin and protect from multiple influenza types and subtypes. Vaccine. 2011; 29(35):5911-8. |
| Lassa fever | Lassa virus | nucleoprotein (NP) and glycoprotein complex (GPC) | Goicochea MA, Zapata JC, Bryant J, Davis H, Salvato MS, Lukashevich IS. Evaluation of Lassa virus vaccine immunogenicity in a CBA/J-ML29 mouse model. Vaccine. 2012; 30(8):1445-52. |
| LCMV | Lymphocytic choriomeningiti s virus | GPC, NP, and Z proteins | Kotturi MF, Swann JA, Peters B, Arlehamn CL, Sidney J, Kolla RV, James EA, Akondy RS, Ahmed R, Kwok WW, Buchmeier MJ, Sette A.J Virol. 2011; 85(22):11770-80. Human CD8⁺ and CD4⁺ T cell memory to lymphocytic choriomeningitis virus infection. |
| Marburg hemorrhagic fever (MHF) | Marburg virus | GP, NP, and VP40 proteins | Kalina WV, Warfield KL, Olinger GG, Bavari S. Discovery of common marburgvirus protective epitopes in a BALB/c mouse model. Virol J. 2009; 6:132. |
| Measles | Measles virus | fusion protein, hemagglutinin, nucleoprotein | Jaye A, Magnusen AF, Sadiq AD, Corrah T, Whittle HC. Ex vivo analysis of cytotoxic T lymphocytes to measles antigens during infection and after vaccination in Gambian children. J Clin Invest. 1998; 102(11):1969-77. |
| Middle East respiratory syndrome (MERS) | Middle East respiratory syndrome coronavirus | spike protein | Zhao J, Li K, Wohlford-Lenane C, Agnihothram SS, Fett C, Zhao J, Gale MJ Jr, Baric RS, Enjuanes L, Gallagher T, McCray PB Jr, Perlman S. Rapid generation of a mouse model for Middle East respiratory syndrome. Proc Natl Acad Sci USA. 2014; 111(13):4970-5. |
| Molluscum contagiosum (MC) | Molluscum contagiosum virus (MCV) | MC133L and MC084L | Watanabe T, Morikawa S, Suzuki K, Miyamura T, Tamaki K, Ueda Y. Two major antigenic polypeptides of molluscum contagiosum virus. J Infect Dis. 1998; 177(2):284-92. |
| Monkeypox | Monkeypox virus | F8L and E9L | Song H, Sidney J, Wiseman RW, Josleyn N, Cohen M, Blaney JE, Jahrling PB, Sette A. Characterizing monkeypox virus specific CD8+ T cell epitopes in rhesus macaques. Virology. 2013; 447(1-2):181-6. |
| Mumps | Mumps virus | hemagglutininneuraminidase (HN) and fusion (F) protein | Matsuura M, Somboonthum P, Murakami K, Ota M, Shoji M, Kawabata K, Mizuguchi H, Gomi Y, Yamanishi K, Mori Y. Novel polyvalent live vaccine against varicella-zoster and mumps virus infections. Microbiol Immunol. 2013; 57(10):704-14. |
| Poliomyelitis | Poliovirus | VP1 capsid protein | Graham S, Wang EC, Jenkins O, Borysiewicz LK. Analysis of the human T-cell response to picornaviruses: identification of T-cell epitopes close to B-cell epitopes in poliovirus. J Virol. 1993; 67(3):1627-37. |
| Progressive multifocal leukoencephalo pathy | JC virus | VP1 major capsid protein | Du Pasquier RA, Stein MC, Lima MA, Dang X, Jean-Jacques J, Zheng Y, Letvin NL, Koralnik IJ. JC virus induces a vigorous CD8+ cytotoxic T cell response in multiple sclerosis patients. J Neuroimmunol. 2006; 176(1-2):181-6. |
| Rabies | Rabies virus | glycoprotein | Amann R, Rohde J, Wulle U, Conlee D, Raue R, Martinon O, Rziha HJ. A new rabies vaccine based on a recombinant ORF virus (parapoxvirus) expressing the rabies virus glycoprotein. J Virol. 2013; 87(3):1618-30. |
| Respiratory syncytial virus infection | Respiratory syncytial virus (RSV) | matrix, fusion, nucleo- and attachment proteins | Lukens MV, Claassen EA, de Graaff PM, van Dijk ME, Hoogerhout P, Toebes M, Schumacher TN, van der Most RG, Kimpen JL, van Bleek GM. Characterization of the CD8+ T cell responses directed against respiratory syncytial virus during primary and secondary infection in C57BL/6 mice. Virology. 2006; 352(1):157-68. |
| Rift Valley fever (RVF) | Rift Valley fever virus | nucleocapsid protein | Xu W, Watts DM, Costanzo MC, Tang X, Venegas LA, Jiao F, Sette A, Sidney J, Sewell AK, Wooldridge L, Makino S, Morrill JC, Peters CJ, Kan-Mitchell J. The nucleocapsid protein of Rift Valley fever virus is a potent human CD8+ T cell antigen and elicits memory responses.PLoS One. 2013;8(3):e59210. |
| Rotavirus infection | Rotavirus | capsid proteins VP2 and VP6 | Li T, Lin H, Zhang Y, Li M, Wang D, Che Y, Zhu Y, Li S, Zhang J, Ge S, Zhao Q, Xia N. Improved characteristics and protective efficacy in an animal model of E. coli-derived recombinant double-layered rotavirus virus-like particles. Vaccine. 2014; 32(17):1921-31. |
| Rubella | Rubella virus | capsid protein | Lovett AE, Hahn CS, Rice CM, Frey TK, Wolinsky JS. Rubella virus-specific cytotoxic T-lymphocyte responses: identification of the capsid as a target of major histocompatibility complex class I-restricted lysis and definition of two epitopes. J Virol. 1993; 67(10):5849-58. |
| SARS (Severe Acute Respiratory Syndrome) | SARS coronavirus | Replicase, spike, orf3, orf4, envelope, membrane | Li CK, Wu H, Yan H, Ma S, Wang L, Zhang M, Tang X, Temperton NJ, Weiss RA, Brenchley JM, Douek DC, Mongkolsapaya J, Tran BH, Lin CL, Screaton GR, Hou JL, McMichael AJ, Xu XN. T cell responses to whole SARS coronavirus in humans. J Immunol. 2008; 181(8):5490-500. |
| Venezuelan equine encephalitis | Venezuelan equine encephalitis virus | structural proteins | Paessler S, Fayzulin RZ, Anishchenko M, Greene IP, Weaver SC, Frolov I. Recombinant sindbis/Venezuelan equine encephalitis virus is highly attenuated and immunogenic. J Virol. 2003; 77(17):9278-86. |
| West Nile Fever | West Nile virus | capsid protein, NS3, NS4b, and NS5 | Kaabinejadian S, Piazza PA, McMurtrey CP, Vernon SR, Cate SJ, Bardet W, Schafer FB, Jackson KW, Campbell DM, Buchli R, Rinaldo CR, Hildebrand WH. Identification of class I HLA T cell control epitopes for West Nile virus. PLoS One. 2013; 8(6):e66298. |
| Yellow fever | Yellow fever virus | envelope glycoprotein, non-structural proteins NS3, NS1-NS2a-NS2b | Putnak JR, Schlesinger JJ. Protection of mice against yellow fever virus encephalitis by immunization with a vaccinia virus recombinant encoding the yellow fever virus non-structural proteins, NS1, NS2a and NS2b. J Gen Virol. 1990; 71 (Pt 8):1697-702. |

| | | | |
|---|---|---|---|
| **DISEASE** | **SOURCE OF DISEASE** | **MAIN TARGETS** | **REF** |

**Table 3. Selected antigens from yeast, bacteria, parasites and fungi.**

| | | | | | | |
|---|---|---|---|---|---|---|
| Acinetobacter infections | Acinetobacter baumannii | Biofilm associated protein (Bap) | Loehfelm TW, Luke NR, Campagnari AA. J Bacteriol. 2008; 190(3):1036-44. Identification and characterization of an Acinetobacter baumannii biofilm-associated protein. | | | |
| African sleeping sickness (African trypanosomiasis) | Trypanosoma brucei | Variable Surface Glycoprotein (VSG) | Lorger M, Engstler M, Homann M, Göringer HU. Targeting the variable surface of African trypanosomes with variant surface glycoprotein-specific, serum-stable RNA aptamers. Eukaryot Cell. 2003; 2(1):84-94. | | | |
| Amebiasis | Entamoeba histolytica | Gal lectin | Houpt E1, Barroso L, Lockhart L, Wright R, Cramer C, Lyerly D, Petri WA. Prevention of intestinal amebiasis by vaccination with the Entamoeba histolytica Gal/GalNac lectin. Vaccine. 2004; 22(5-6):611-7. | | | |
| Anaplasmosis | Anaplasma genus | type IV secretion system (TFSS) proteins VirB9, VirB10, and conjugal transfer protein (CTP). | Lopez JE, Palmer GH, Brayton KA, Dark MJ, Leach SE, Brown WC. Immunogenicity of Anaplasma marginale type IV secretion system proteins in a protective outer membranevaccine. Infect Immun. 2007; 75(5):2333-42. | | | |
| Anthrax | Bacillus anthracis | protective antigen (PA), lethal factor (LF) | Nayarisseri A, Yadav M, Ahmed S, Sahu J, Gupta P, Chourasia R, Mittal D. Identification of T cell epitope for protective antigen and lethal factor of Bacillus anthracis - An immunoinformatics approach. Int.J. Drug Discovery. 2012; 4(1):137-144. | | | |
| Aspergillosis | Aspergillus genus | Asp f16 | Ramadan G, Davies B, Kurup VP, Keever-Taylor CA. Generation of cytotoxic T cell responses directed to human leucocyte antigen Class I restricted epitopes from the Aspergillus f16 allergen. Clin Exp Immunol. 2005; 140(1): 81-91. | | | |
| Bacterial pneumonia | multiple bacteria, eg. Staphillococcus aureus | fibronectin-binding protein (FnbpA) | Weichhart T, Horky M, Söllner J, Gangl S, Henics T, Nagy E, Meinke A, von Gabain A, Fraser CM, Gill SR, Hafner M, von Ahsen U. Functional selection of vaccine candidate peptides from Staphylococcus aureus whole-genome expression libraries in vitro. Infect Immun. 2003 Aug;71(8):4633-41. | | | |
| Bacteroides infection | Bacteroides genus | polysaccharide A (PSA) | Bloem K, García-Vallejo JJ, Vuist IM, Cobb BA, van Vliet SJ, van Kooyk Y. Interaction of the Capsular Polysaccharide A from Bacteroides fragilis with DC-SIGN on Human Dendritic Cells isNecessary for Its Processing and Presentation to T Cells. Front Immunol. 2013; 4:103. | | | |
| Brucellosis | Brucella genus | outer membrane proteins (Omps) | Cassataro J, Velikovsky CA, de la Barrera S, Estein SM, Bruno L, Bowden R, Pasquevich KA, Fossati CA, Giambartolomei GH. A DNA vaccine coding for the Brucella outer membrane protein 31 confers protection against B. melitensis and B.ovis infection by eliciting a specific cytotoxic response. Infect Immun. 2005; 73(10):6537-46. | | | |
| Candidiasis (Moniliasis; Thrush) | usually Candida albicans and other Candida species | Als1, Als3 proteins | Bar E, Gladiator A, Bastidas S, Roschitzki B, Acha-Orbea H, Oxenius A, LeibundGut-Landmann S. A novel Th cell epitope of Candida albicans mediates protection from fungal infection. J Immunol. 2012; 188(11):5636-43. | | | |
| Cat-scratch disease | Bartonella henselae | Bartonella-translocated effector proteins (Beps) A-G | Schulein R, Guye P, Rhomberg TA, Schmid MC, Schröder G, Vergunst AC, Carena I, Dehio C. A bipartite signal mediates the transfer of type IV secretion substrates of Bartonella henselae into human cells. Proc Natl Acad Sci USA. 2005; 102(3):856-61. | | | |
| Chagas Disease (American trypanosomiasis) | Trypanosoma cruzi | surface proteins: ASP-1 ASP-2 | Low HP, Santos MA, Wizel B, Tarleton RL. Amastigote surface proteins of Trypanosoma cruzi are targets for CD8+ CTL. J Immunol. 1998; 160(4):1817-23. | | | |
| Chlamydophila pneumoniae infection (Taiwan acute respiratory agent or TWAR) | Chlamydophila pneumoniae | outer membrane proteins, 76 kDa protein, inclusion membrane protein | Wizel B1, Starcher BC, Samten B, Chroneos Z, Barnes PF, Dzuris J, Higashimoto Y, Appella E, Sette A. Multiple Chlamydia pneumoniae antigens prime CD8+ Tc1 responses that inhibit intracellular growth of this vacuolar pathogen. J Immunol. 2002;169(5):2524-35. | | | |
| Cholera | Vibrio cholerae | non-toxic B component of cholera toxin (CTB) | Bowen JC1, Nair SK, Reddy R, Rouse BT. Cholera toxin acts as a potent adjuvant for the induction of cytotoxic T-lymphocyte responses with non-replicating antigens. Immunology. 1994;81(3):338-42. | | | |
| Cryptococcosis | Cryptococcus neoformans | M98 membrane protein | MingLi GM; AnMei D; Ye Z; Yan C; Bo C, RenQian Z; Hai W; SunXiao C, 2008: Prediction and identification of HLA-A * 0201 restricted CD8+CTL epitopes in Cryptococcus neoformans MP98. Journal of Modern Laboratory Medicine 23(2): 1-4 | | | |
| Cryptosporidiosis | Cryptosporidium genus | cp15, p23 surface glycoproteins | Wang C1, Luo J, Amer S, Guo Y, Hu Y, Lu Y, Wang H, Duan M, He H. Multivalent DNA vaccine induces protective immune responses and enhanced resistance against Cryptosporidium parvum infection. Vaccine. 2010; 29(2):323-8. | | | |
| | | acidic ribosomal protein P2 | Benitez A1, Priest JW, Ehigiator HN, McNair N, Mead JR. Evaluation of DNA encoding acidic ribosomal protein P2 of Cryptosporidium parvum as a potential vaccine candidate for cryptosporidiosis. Vaccine. 2011; 29(49):9239-45. | | | |
| Group A streptococcal infection | Streptococcus pyogenes | fibronectin-binding protein I (SfbI) | Schulze K, Medina E, Chhatwal GS, Guzmán CA. Identification of B- and T-cell epitopes within the fibronectin-binding domain of the SfbI protein of Streptococcuspyogenes. Infect Immun. 2003; 71(12):7197-201. | | | |
| Helicobacter pylori infection | Helicobacter pylori | multiple, eg. cagA | Yamasaki R, Yokota K, Okada H, Hayashi S, Mizuno M, Yoshino T, Hirai Y, Saitou D, Akagi T, Oguma K. Immune response in Helicobacter pylori-induced low-grade gastric-mucosa-associated lymphoid tissue (MALT)lymphoma. J Med Microbiol. 2004; 53(Pt 1):21-9. | | | |
| Legionellosis (Legionnaires' disease) | Legionella pneumophila | peptidoglycan-associated lipoprotein (PAL) | Yoon WS, Park DH, Park YK, Park SC, Sin JI, Kim MJ. Comparison of Responses Elicited by Immunization with a Legionella Species Common Lipoprotein Delivered as Naked DNA or Recombinant Protein. DNA and Cell Biology. 2002; 21(2):99-107. | | | |
| Leptospirosis | Leptospira genus | Leptospiral immunoglobulin-like protein A (LigA) | Guo YJ, Wang KY, Sun SH. Identification of an HLA-A*0201-restricted CD8(+) T-cell epitope encoded within Leptospiral immunoglobulin-like protein A. Microbes Infect. 2010; 12(5):364-73. | | | |
| Listeriosis | Listeria monocytogenes | listeriolysin O (LLO) | Vijh S1, Pamer EG. Immunodominant and subdominant CTL responses to Listeria monocytogenes infection. J Immunol. 1997; 158(7):3366-71. | | | |
| Lyme disease (Lyme borreliosis) | usually Borrelia burgdorferi and other Borrelia species | outer surface protein (Osp) A, OspB, flagellin | Busch DH, Jassoy C, Brinckmann U, Girschick H, Huppertz HI. Detection of Borrelia burgdorferi-specific CD8+ cytotoxic T cells in patients with Lyme arthritis. J Immunol. 1996; 157(8):3534-41. | | | |
| Lymphatic filariasis (Elephantiasis) | Wuchereria bancrofti and Brugia malayi | troponin (of W.B.), small heatshock protein (BmHsp12.6) (of B.M.)7 | VS Gomase, NR Chitlange, AS Sherkhane, SS Changbhale, KV Kale. Prediction of Wuchereria Bancrofti Troponin Antigenic Peptides: Application in Synthetic Vaccine Design to Counter Lymphatic Filariasis. J Vaccines Vaccin 2013; 4 (1), 2 | | | |
| Malaria | Plasmodium genus | circumsporozoite (CS) protein | Malik A, Egan JE, Houghten RA, Sadoff JC, Hoffman SL. Human cytotoxic T lymphocytes against the Plasmodium falciparum circumsporozoite protein. Proc Natl Acad Sci U S A. 1991 Apr 15;88(8):3300-4. | | | |
| Meningococcal disease | Neisseria meningitidis | outermembrane prot ein | Chandra S, Singh D, Singh TR. Prediction and characterization of T-cell epitopes for epitope vaccine design from outer membrane protein ofNeisseria meningitidis serogroup B. Bioinformation. 2010; 5(4):155-61. | | | |
| Pneumococcal infection | Streptococcus pneumoniae | ZPS, surface proteins | Mertens J, Fabri M, Zingarelli A, Kubacki T, Meemboor S, Groneck L, Seeger J, Bessler M, Hafke H, Odenthal M, Bieler JG, Kalka C, Schneck JP, Kashkar H,Kalka-Moll WM. Streptococcus pneumoniae serotype 1 capsular polysaccharide induces CD8CD28 regulatory T lymphocytes byTCR crosslinking. PLoS Pathog. 2009; 5(9):e1000596. | | | |
| Syphilis | Treponema pallidum | glycerophosphodie ster phosphodiesterase (Gpd) | Zhao F, Wang S, Zhang X, Gu W, Yu J, Liu S, Zeng T, Zhang Y, Wu Y. Protective efficacy of a Treponema pallidum Gpd DNA vaccine vectored by chitosan nanoparticles and fused withinterleukin-2. Can J Microbiol. 2012; 58(2):117-23. | | | |
| Toxocariasis (Ocular Larva Migrans (OLM)) | Toxocara canis or Toxocara cati | secreted TES antigens | Maizels RM. Toxocara canis: molecular basis of immune recognition and evasion. Vet Parasitol. 2013; 193(4):365-74. | | | |
| Trinochccliasis | Toxoplasma gondii | GRA6 antigenic precursor, SAG1 surface antigen | Feliu V, Vasseur V, Grover HS, Chu HH, Brown MJ, Wang J, Boyle JP, Robey EA, Shastri N, Blanchard N. Location of the CD8 T cell epitope within the antigenic precursor determines immunogenicity and protectionagainst the Toxoplasma gondii parasite. PLoS Pathog. 2013; 9(6):e1003449. | | | |
| Trichomoniasis | Trichomonas vaginalis | surface proteins | L.B. Corbeil, L. Munson, C. Campero, R.H. BonDurant Bovine trichomoniasis as a model for development of vaccines against sexually-transmitted disease American Journal of Reproductive Immunology. 2001; 45(5):310-319 | | | |
| Tuberculosis | usually Mycobacterium tuberculosis | Ag 85 secreted protein complex (Ag85) | Geluk A, van Meijgaarden KE, Franken KL, Drijfhout JW, D'Souza S, Necker A, Huygen K, Ottenhoff TH. Identification of major epitopes of Mycobacterium tuberculosis AG85B that are recognized by HLA-A*0201-restricted CD8+ T cells in HLA-transgenic mice and humans. J Immunol. 2000;165(11):6463-71. | | | |
| Tularemia | Francisella tularensis | membrane-associated proteins, tranport proteins | Zvi A, Rotem S, Bar-Haim E, Cohen O, Shafferman A. Whole-genome immunoinformatic analysis of F. tularensis: predicted CTL epitopes clustered in hotspots areprone to elicit a T-cell response. PLoS One. 2011;6(5):e20050. | | | |
| Valley fever | | Coccidioides immitis or Coccidioides posadasii | Pep1, Amn1, and Plb | Hurtgen BJ, Hung CY, Ostroff GR, Levitz SM, Cole GT. Construction and evaluation of a novel recombinant T cell epitope-based vaccine against Coccidioidomycosis. Infect Immun. 2012; 80(11):3960-74. | | |
| White piedra (Tinea blanca) | | Trichosporon beigelii | not specified yet | Lyman CA, Garrett KF, Pizzo PA, Walsh TJ. of human polymorphonuclear leukocytes and monocytes to Trichosporon beigelii: host defenseagainst an emerging opportunistic pathogen. J Infect Dis. 1994; 170(6):1557-65. | | |
| Yersinia pseudotuberculosis infection | | Yersinia pseudotuberculosis | Yersinia outer membrane proteins (Yops) | Falgarone G, Blanchard HS, Riot B, Simonet M, Breban M. Cytotoxic T-cell-mediated response against Yersinia pseudotuberculosis in HLA-B27 transgenic rat. Infect Immun. 1999; 67(8):3773-9. | | |
| Yersiniosis | | Yersinia enterocolitica | Yop51 protein tyrosine phosphatase | Starnbach MN, Bevan MJ. Cells infected with Yersinia present an epitope to class I MHC-restricted CTL. J Immunol. 1994;153(4):1603-12. | | |

| **Actionable target** | **Vaccine type** | **ID** | **Intervention** | **Condition** | **Phase** | **Locations** |
|---|---|---|---|---|---|---|
| **NY-ESO-1** | **PEPTIDE** | NCT01308294 | NA-17, MAGE-3.A2 and NY-ESO-1 peptides | Melanoma | Phase 1 | Switzerland |
| | | | | | Phase 2 | |

**Table 4. Tumor antigens in drugs under investigations.**

| | | | | | | |
|---|---|---|---|---|---|---|
| **NY-ESO-1** | **PROTEIN** | NCT01584115 | Biological: NY-ESO-1 combined with MPLA | Cancer, Melanoma, Ovarian Cancer, Lung Cancer | Phase 1 | Brazil |
| | | | | | Phase 2 | |
| | **PROTEIN** | NCT01213472 | GSK Biologicals' 2241658A | NY-ESO-1-positive, Unresectable and Progressive Metastatic Cutaneous Melanoma | Phase 1 | WW (26) |
| | **CELL-BASED** | NCT01795976 | NY-ESO-1 transduced T cells | Oesophageal Cancer | Phase 2 | UK |
| | **CELL-BASED** | NCT01343043 | NYESO-1(C259) transduced autologous T cells | Synovial Sarcoma | Phase 1 | US (2) |
| | | | | | Phase 2 | |
| | **CELL-BASED** | NCT01697527 | Transfer of NY-ESO-1 TCR Engineered Peripheral Blood Mononuclear Cells | Malignant Neoplasm | Phase 2 | US |
| | **CELL-BASED** | NCT01159288 | Peptides pulsed onto DC | Non Small Cell Lung Cancer | Phase 2 | France |
| | **CELL-BASED** | NCT01567891 | NY-ESO-1-specific T cells | Ovarian Cancer | Phase 1 | US |
| | | | | | Phase 2 | |
| | **CELL-BASED** | NCT01892293 | NY-ESO-1c259-modified T cells | Multiple Myeloma | Phase 1 | US (3) |
| | | | | | Phase 2 | |
| | **CELL-BASED** | NCT01350401 | NY-ESO-1(C259) transduced autologous T cells | Melanoma | Phase 1 | US (2) |
| | | | | | Phase 2 | |
| | **PROTEIN** | UMIN000007954 | NY-ESO-1 protein | Individuals with NY-ESO-1-expressing advanced esophageal-, gastric-, lung cancer and melanoma | Phase 1 | Japan |
| **MAGE-A** | **PROTEIN** | NCT01437605 | Recombinant MAGE-A3 protein | Melanoma | Phase 2 | US |
| | **PROTEIN** | NCT01245673 | MAGE-A3 protein | Advanced Myeloma | Phase 2 | US (2) |
| | **PROTEIN** | NCT01266603 | Recombinant MAGE-A3 protein | Unresectable or Metastatic Melanoma | Phase 2 | US |
| | **PROTEIN** | NCT01435356 | Recombinant MAGE-A3 protein | Muscle Invasive Bladder Cancer (MAGNOLIA) | Phase 2 | Europe (33) |
| | **PEPTIDE** | NCT01308294 | NA-17, MAGE-3.A2 and NY-ESO-1 and MAGE-A3-DP4 peptides | Melanoma | Phase 1 | Switzerland |
| | | | | | Phase 2 | |
| | **PEPTIDE** | NCT01352286 | Autologous genetically modified T cells | Multiple Myeloma | Phase 1 | US (2) |
| | | | | | Phase 2 | |
| | **PEPTIDE** | NCT00480025 | MAGE-A3 peptide GSK1572932A | Non-Small Cell Lung Cancer | Phase 3 | WW (622) |
| | **CELL-BASED** | UMIN000010729 | lymphocytes transduced with MAGE-A4-specific TCR gene | Therapy-resistant esophageal cancer | Phase 1 | Japan |
| | **PROTEIN** | UMIN000002153 | CHP-MAGE-A4 protein complex vaccine | MAGE-A4-expressing refractory cancer individuals (non origin-limited) or individuals who refuse standard therapy | Phase 1 | Japan |
| **EBV** | **PROTEIN** | NCT01555892 | EBV specific CTLs | Hodgkin's Disease, Non-Hodgkin's Lymphoma, Lymphoproliferative Disease, Lymphoma | Phase 1 | US (2) |
| | **PROTEIN** | NCT00062868 | EBV specific CTLs | Hodgkin Disease, Non Hodgkin Lymphoma, Lymphoepithelioma, Leiomyosarcoma | Phase 1 | US |
| | **PROTEIN** | NCT01800071 | MVA-EBNA1/LMP2 vaccine | Nasopharyngeal Cancer, Epstein Barr Virus Infections | Phase 1 | UK (3) |
| | **PROTEIN** | NCT01094405 | Recombinant Epstein-Barr Virus (EBV) Vaccine | Nasopharyngeal Cancer, Epstein-Barr Virus Infections | Phase 2 | Hong Kong |
| | **CELL-BASED** | NCT01447056 | LMP specific T cells | Hodgkin Lymphoma, Non-Hodgkin Lymphoma, Lymphoproliferative Disorder, Nasopharyngeal Carcinoma, Leiomyosarcoma, Severe Chronic Active EBV (SCAEBV)Carcinoma | Phase 1 | US (2) |
| **HER2/Neu** | **PEPTIDE** | NCT01376505 | Her-2 peptide vaccine | Malignant Solid Tumor, Breast Cancer, Malignant Tumor of Colon, GIST, | Phase 1 | US |
| | | | | Ovarian Cancer | | |
| | **PEPTIDE** | NCT01355393 | Her-2/neu peptide vaccine | HER2-positive Breast, cancer, Male Breast Cancer | Phase 1 | US |
| | | | | | Phase 2 | |
| | **PEPTIDE** | NCT01922921 | Her-2/neu intracellular domain protein | HER2-positive Breast cancer, Male Breast Cancer, | Phase 1 | US |
| | | | | | Phase 2 | |
| | **PEPTIDE** | NCT01729884 | Her-2/neu peptide vaccine | HER2-positive Breast Cancer, Male Breast Cancer | Phase 2 | US |
| | **PEPTIDE** | NCT00524277 | HER2/neu GP2 peptide + GM-CSF, HER2/neu AE37 peptide+ GM-CSF | Breast Cancer, for HLA-A2 positive individuals: GP2 peptide, HLA-A2 negative individuals: AE37 peptide | Phase 2 | WW (13) |
| | **PEPTIDE** | NCT01632332 | Her-2/neu peptide vaccine | HER2-positive Breast Cancer, Male Breast Cancer | Phase 1 | US |
| | **PEPTIDE** | NCT01532960 | 9 Peptides from Her-2/neu | Breast Cancer (Stage IB-IIIA) | single arm, pilot study | US |
| | **PEPTIDE** | NCT01570036 | NeuVax vaccine+ Herceptine | Breast Cancer | Phase 2 | US (6) |
| | **PEPTIDE** | NCT01479244 | NeuVax vaccine | HER2 negative Breast Cancer | Phase 3 | WW (145) |
| | **CELL-BASED** | NCT01730118 | Autologous Ad HER2 dendritic cell vaccine | Breast Neoplasms, Breast Cancer, Adenocarcinomas | Phase 1 | US |
| | **CELL-BASED** | NCT01935843 | genetically engineered lymphocytes | AdvancedHER-2 Positive Solid Tumors | Phase 1 | China |
| | | | | | Phase 2 | |
| | **CELL-BASED** | NCT00902044 | Autologous HER2-specific T cells | Sarcoma | Phase 1 | US (2) |
| | **CELL-BASED** | NCT01109095 | Genetically modified HER.CAR CMV-specific CTLs | Glioblastoma Multiforme | Phase 1 | US (2) |
| | **NUCLEIC ACID** | NCT01526473 | virus-like replicon particles | (HER2+) Cancers Including Breast Cancer | Phase 1 | US |
| **MUC**-**1** | **PEPTIDE** | NCT01720836 | peptide vaccine and PolylCLC | Non-small Cell Lung Cancer (NSCLC) | Phase 1 | US |
| | | | | | Phase 2 | |
| | **PEPTIDE** | NCT01507103 | Peptide (Stimuvax®) | Rectal Cancer | Phase 2 | Netherlands |
| | **PEPTIDE** | NCT01462513 | Peptide (Stimuvax®) | Colon Carcinoma, Rectum Carcinoma | Phase 2 | Europe (18) |
| | **PEPTIDE** | NCT01556789 | 43 amino acid glycopeptide | Solid Tumors | Phase 1 | US |
| | **PEPTIDE** | NCT01496131 | Peptide (Stimuvax®) | Prostate Cancer | Phase 2 | US |
| | **PEPTIDE** | NCT01015443 | Peptide liposome | Non-Small Cell Lung Cancer | Phase 3 | Asia (47) |
| | **PEPTIDE** | 2011-004822-85 | Peptide (Stimuvax®) | primary breast cancer | Phase 2 | Austria |
| | **PEPTIDE** | 2008-005544-17 | Peptide (Stimuvax®) | Breast cancer | Phase 3 | Europe (2) |

Also disclosed herein is a method of identifying a fragment of a polypeptide as immunogenic for a specific human subject, the method comprising the steps of
(i) determining the HLA class I genotype and/or the HLA class II genotype of said specific human subject; and
(ii) determining whether the polypeptide comprises:
   a. a sequence of 7 to 11 consecutive amino acids that is capable of binding to at least two HLA class I of the subject; or
   b. a sequence of 13 to 17 consecutive amino acids that is capable of binding to at least two HLA class II of the subject; and
(iii) identifying said consecutive sequence of amino acids as the sequence of a fragment of the polypeptide that is immunogenic for the subject.

### HLA-epitope binding

A given HLA of a subject will only present to T cells a limited number of different peptides produced by the processing of protein antigens in an APC. As used herein, "display" or "present", when used in relation to HLA, references the binding between a peptide (epitope) and an HLA. In this regard, to "display" or "present" a peptide is synonymous with "binding" a peptide.

As used herein, the term "epitope" or "T cell epitope" refers to a sequence of contiguous amino acids contained within a protein antigen that possess a binding affinity for (is capable of binding to) one or more HLAs. An epitope is HLA- and antigen-specific (HLA-epitope pairs, predicted with known methods), but not subject specific, since a given epitope is recognised by the specific HLAs of some individuals and not others. An epitope, a T cell epitope, a polypeptide, a fragment of a polypeptide or a composition comprising a polypeptide or a fragment thereof is "immunogenic" for a specific human subject if it is capable of inducing a T cell response (a cytotoxic T cell response or a helper T cell response) in that subject. The terms "T cell response" and "immune response" are used herein interchangeably, and refer to the activation T cells and/or the induction of one or more effector functions following recognition of one or more HLA-epitope binding pairs. Effector functions include cytotoxicity, cytokine production and proliferation. According to the present invention, an epitope, a T cell epitope, or a fragment of a polypeptide is immunogenic for a specific subject if it is capable of binding to at least two class I or at least two class II HLAs of the subject.

For the purposes of this disclosure we have coined the term "personal epitope", or "PEPI". A "PEPI" is a fragment of a polypeptide consisting of a sequence of contiguous amino acids of the polypeptide that is a T cell epitope capable of binding to one or more HLA class I molecules or one or more HLA class II molecules of a specific human subject. In other words a "PEPI" is a T cell epitope that is recognised by the HLA set of a specific individual. In contrast to an "epitope", PEPIs are specific to an individual because different individuals have different HLA molecules which each bind to different T cell epitopes.

"PEPI1" as used herein refers to a peptide, or a fragment of a polypeptide, that can bind to one HLA class I of an individual. "PEPI1+" refers to a peptide, or a fragment of a polypeptide, that can bind to one or more HLA class I of an individual.

"PEPI2" refers to a peptide, or a fragment of a polypeptide, that can bind to two HLA class I of an individual. "PEPI2+" refers to a peptide, or a fragment of a polypeptide, that can bind to two or more HLA class I of an individual, i.e. a fragment identified according to a method of the invention.

"PEPI3" refers to a peptide, or a fragment of a polypeptide, that can bind to three HLA class I of an individual. "PEPI3+" refers to a peptide, or a fragment of a polypeptide, that can bind to three or more HLA class I of an individual.

"PEPI4" refers to a peptide, or a fragment of a polypeptide, that can bind to four HLA class I of an individual. "PEPI4+" refers to a peptide, or a fragment of a polypeptide, that can bind to four or more HLA class I of an individual.

"PEPI5" refers to a peptide, or a fragment of a polypeptide, that can bind to five HLA class I of an individual. "PEPI5+" refers to a peptide, or a fragment of a polypeptide, that can bind to five or more HLA class I of an individual.

"PEPI6" refers to a peptide, or a fragment of a polypeptide, that can bind to all 6 HLA class I of an individual.

Generally speaking, epitopes presented by HLA class I molecules are about nine amino acids long and epitopes presented by HLA class II molecules are about fifteen amino acids long. For the purposes of this disclosure, however, an epitope may be more or less than nine (for HLA Class I) or fifteen (for HLA Class II) amino acids long, as long as the epitope is capable of binding HLA. For example, an epitope that is capable of binding to class I HLA may be between 7, or 8 or 9 and 9 or 10 or 11 amino acids long. An epitope that is capable of binding to a class II HLA may be between 13, or 14 or 15 and 15 or 16 or 17 amino acids long.

Using techniques known in the art, it is possible to determine the epitopes that will bind to a known HLA. Any suitable method may be used, provided that the same method is used to determine multiple HLA-epitope binding pairs that are directly compared. For example, biochemical analysis may be used. It is also possible to use lists of epitopes known to be bound by a given HLA. It is also possible to use predictive or modelling software to determine which epitopes may be bound by a given HLA. Examples are provided in Table 5. In some cases a T cell epitope is capable of binding to a given HLA if it has an IC50 or predicted IC50 of less than 5000 nM, less than 2000 nM, less than 1000 nM, or less than 500 nM.

| **EPITOPE PREDICTION TOOLS** | **WEB LINK** |
|---|---|
| BIMAS, NIH | www-bimas.cit.nih.gov/molbio/hla bind/ |

| Table 5 | |
|---|---|
| PPAPROC, Tubingen Univ. | |
| MHCPred, Edward Jenner Inst. of Vaccine Res. | |
| EpiJen, Edward Jenner Inst. of Vaccine Res. | http://www.ddg-pharmfac.net/epijen/EpiJen/EpiJen.htm |
| NetMHC, Center for Biological Sequence Analysis | http://www.cbs.dtu.dk/services/NetMHC/ |
| SVMHC, Tubingen Univ. | http://abi.inf.uni-tuebingen.de/Services/SVMHC/ |
| SYFPEITHI, Biomedical Informatics, Heidelberg | http://www.syfpeithi.de/bin/MHCServer.dll/EpitopePrediction. htm |
| ETK EPITOOLKIT, Tubingen Univ. | http://etk.informatik.uni-tuebingen.de/epipred/ |
| PREDEP, Hebrew Univ. Jerusalem | http://margalit.huji.ac.il/Teppred/mhc-bind/index.html |
| RANKPEP, MIF Bioinformatics | http://bio.dfci.harvard.edu/RANKPEP/ |
| IEDB, Immune Epitope Database | http://tools.immuneepitope.org/main/html/tcell tools.html |

| **EPITOPE DATABASES** | **web link** |
|---|---|
| MHCBN, Institute of Microbial Technology, Chandigarh, INDIA | http://www.imtech.res.in/raghava/mhcbn/ |
| SYFPEITHI, Biomedical Informatics, Heidelberg | http://www.syfpeithi.de/ |
| AntiJen, Edward Jenner Inst. of Vaccine Res. | http://www.ddg-pharmfac.net/antijen/AntiJen/antijenhomepage.htm |
| EPIMHC database of MHC ligands, MIF Bioinformatics | http://immunax.dfci.harvard.edu/epimhc/ |
| IEDB, Immune Epitope Database | http://www.iedb.org/ |

The inventors have discovered that T cell epitope presentation by multiple HLAs of an individual is generally needed to trigger a T cell response. Accordingly, the methods of the invention comprise determining whether a polypeptide has a sequence that is a T cell epitope capable of binding to at least two HLA class I or at least two HLA class II (PEPI2+) of a specific human subject.

The best predictor of a cytotoxic T cell response to a given polypeptide, as determined by the inventors, is the presence of at least one T cell epitope that is presented by three or more HLA class I of an individual (≥1 PEPI3+). Accordingly, in some cases the method comprises determining whether a polypeptide has a sequence that is a T cell epitope capable of binding to at least three HLA class I of a specific human subject. In some cases the method comprises determining whether a polypeptide has a sequence that is a T cell epitope capable of binding to just three HLA class I of a specific human subject. A helper T cell may be predicted by the presence of at least one T cell epitope that is presented by three or more (≥1 PEPI3+) or 4 or more (≥1 PEPI4+) HLA class II of an individual. Therefore in some cases, the method comprises determining whether a polypeptide has a sequence that is a T cell epitope capable of binding to at least three HLA class II of a specific human subject. In other cases, the method comprises determining whether a polypeptide has a sequence that is a T cell epitope capable of binding to at least four HLA class II of a specific human subject. In other cases, the method comprises determining whether a polypeptide has a sequence that is a T cell epitope capable of binding to at just three and/or just four HLA class II of a specific human subject.

### Predicting the immunological response of an individual to a polypeptide antigen

Specific polypeptide antigens induce immune responses in only a fraction of human subjects. Currently, there is no diagnostic test that can predict whether a polypeptide antigen would likely induce an immune response in an individual. In particular, there is a need for a test that can predict whether a person is an immune responder to a vaccine or immunotherapy composition.

According to the present invention, the polypeptide antigen-specific T cell response of an individual is defined by the presence within the polypeptide of one or more fragments that may be presented by multiple HLA class I or multiple HLA class II of the individual.

In some cases in accordance with the invention steps (ii) and (iii) of the method of identifying a fragment of a polypeptide as immunogenic for a specific human subject are repeated until all of the fragments of the polypeptide that are a T cell epitope capable of binding to at least two HLA class I of the subject, and/or all of the fragments of the polypeptide that are a T cell epitope capable of binding to at least two HLA class II of the subject have been identified. In other cases, steps (ii) and (iii) are repeated until all of the fragments of the polypeptide that are a T cell epitope capable of binding to at least three HLA class I of the subject, and/or all of the fragments of the polypeptide that are a T cell epitope capable of binding to at least three HLA class II of the subject have been identified.

The method may further comprise predicting whether the polypeptide will elicit a cytotoxic T cell response and/or a helper T cell response in the subject, wherein
- the identification of at least one fragment of the polypeptide that is a T cell epitope capable of binding to at least two, or at least three HLA class I of the subject predicts that the polypeptide will elicit a cytotoxic T cell response in the subject;
- the identification of at least one fragment of the polypeptide that is a T cell epitope capable of binding to at least two, or at least three, or at least four HLA class II of the subject predicts that the polypeptide will elicit a helper T cell response in the subject;
- the identification of no fragments of the polypeptide that are T cell epitopes capable of binding to at least two, or at least three HLA class I of the subject predicts that the polypeptide will not elicit a cytotoxic T cell response in the subject; and
- the identification of no fragments of the polypeptide that are T cell epitopes capable of binding to at least two, or at least three, or at least four HLA class II of the subject predicts that the polypeptide will not elicit a helper T cell response in the subject.

There is also currently no test that can predict the likelihood that a person will have a clinical response to, or derive clinical benefit from, a vaccine or immunotherapy composition. This is important because currently T cell responses measured in a cohort of individuals participating in vaccine or immunotherapy clinical trials poorly correlate with clinical responses. That is, the clinical responder subpopulation is substantially smaller than the immune responder subpopulation. Therefore, to enable the personalization of vaccines and immunotherapies it is important to predict not only the likelihood of an immune response in a specific subject, but also whether the immune response induced by the drug will be clinically effective (e.g. can kill cancer cells or pathogen infected cells).

The inventors have discovered that the presence in a vaccine or immunotherapy composition of at least two epitopes that can bind to at least three HLA of an individual is predictive for a clinical response. In other words, if an individual has a total of ≥1 PEPI3+ within the active ingredient polypeptide(s) of a vaccine or immunotherapy composition (and these PEPI3+s are derived from antigen sequences that are in fact expressed in the individual, for example, target tumor cells of the individual express the target tumor-associated antigens), then the individual is a likely clinical responder (i.e. a clinically relevant immune responder).

Therefore, in some cases in accordance with the invention, the polypeptide is an active ingredient of a vaccine or immunotherapy composition having one or more active ingredient polypeptides, and the method of identifying a fragment of the polypeptide as immunogenic for a specific human subject further comprises repeating steps (ii) and (iii) until all of the fragments of the one or more polypeptides that are a T cell epitope capable of binding to at least two, or at least three HLA class I of the subject have been identified, and further predicting whether the subject is likely to have a clinical response to the active ingredient polypeptide in the vaccine or immunotherapy composition, wherein
- the identification of at least two fragments of the one or more active ingredient polypeptides, each of which is a T cell epitope capable of binding to at least two, or at least three HLA class I of the subject predicts that the subject is more likely to have, or will have a clinical response to the composition; and
- the identification of less than two fragments of the one or more polypeptides that are T cell epitopes capable of binding to at least two, or at least three HLA class I of the subject predicts that the subject is less likely to have, or will have, a clinical response to the composition.

A "clinical response" or "clinical benefit" as used herein may be the prevention or a delay in the onset of a disease or condition, the amelioration of one or more symptoms, the induction or prolonging of remission, or the delay of a relapse or recurrence or deterioration, or any other improvement or stabilisation in the disease status of a subject. Where appropriate, a "clinical response" may correlate to "disease control" or an "objective response" as defined by the Response Evaluation Criteria In Solid Tumors (RECIST) guidelines.

The results of a prediction as set out above may be used to inform a physician's decisions concerning treatment of the subject. Accordingly, in some cases the polypeptide is an active ingredient of a vaccine or immunotherapy composition, the method of the invention predicts that the subject will have or is likely to have a T cell response and/or a clinical response to the composition, and the method further comprises selecting the vaccine or immunotherapy composition for use in a method of treatment of the specific human subject. The method may further comprise administering the vaccine or immunotherapy composition to the specific human subject. Alternatively, the method may predict that the one or more polypeptide(s) of the composition will not elicit a T cell response and/or a clinical response in the subject and further comprise selecting a different treatment for the specific human subject.

### Predicting the immunological response of a human population to a polypeptide antigen

The method of identifying a fragment of a polypeptide as immunogenic for a specific human subject disclosed herein may further be used to conduct an *in silico* clinical trial that predicts the proportion of immune-responders or the proportion of clinical responders in a population for a given drug, such as a vaccine or immunotherapy composition. This is useful for pre-selecting drugs that are likely to have high rates of efficacy to undergo clinical testing.

A population of individuals or a subpopulation of individuals can comprise the study cohort of an *in silico* clinical trial conducted with a drug. Each individual in the study cohort is characterized by its HLA genotype. The proportion of individuals in the study cohort having ≥1 PEPI2+, or ≥1 PEPI3+, derived from the polypeptides of the drug is calculated. For the purposes of this disclosure we have termed this the "≥1 PEPI2+ Score" or the "≥1 PEPI3+ Score" (PEPI Score). This PEPI Score predicts the proportion of subjects with T cell responses in a clinical trial conducted with the same drug in a similar cohort of subjects.

Therefore disclosed herein is a method of conducting an *in silico* trial for a vaccine or immunotherapy composition having one or more polypeptide active ingredients, wherein the *in silico* trial predicts the cytotoxic T cell response rate of a human population, the method comprising
(i) defining an *in silico* model human population comprising a plurality of subjects each defined by HLA class I genotype, wherein *the in silico* model human population corresponds to or is representative of said human population;
(ii) determining for each subject in the *in silico* model human population whether the one or more active ingredient polypeptides comprise at least one sequence that is a T cell epitope capable of binding to at least two, or binding to at least three HLA class I of the subject; and
(iii) predicting the cytotoxic T cell response rate of said human population, wherein a higher proportion of the *in silico* model human population that meet the requirements of step (ii) predicts a higher response rate. The proportion of the *in silico* model human population that meet the requirements of step (ii) may correspond to the predicted response rate.

Correlation between HLA-restricted epitopes, immune response rates and clinical response rates has not been demonstrated during clinical trials. This raises the question about the mechanism of action of immunotherapies. The Examples provided herein show that activation of cytotoxic T lymphocytes (CTLs) against multiple targets is required for a clinically meaningful response, for example against heterogeneous tumors. So far, CTL responses reported in clinical trials neither account for multiple targets nor for multiple HLAs. For example, a melanoma peptide vaccine targeting two antigens (Tyrosinase and gp100) elicited CTL responses in 52% of patients, but only 12% had clinical benefit. Using an *in silico* Model Population of 433 subjects we determined a ≥1 PEPI3+ Score of 42% (in 42% a vaccine-derived epitope could be identified that could be presented by at least three HLA class I of the subject) and a ≥2 PEPI3+ Score of 6% (in 6% at least two vaccine-derived epitopes could be identified that could be presented by at least three HLA class II of the subject). This explains why the clinical investigators did not find correlation between CTL response rate and clinical response rate in their trial: the peptides in the vaccine performed poorly in the trial because there were only a few patients in which two vaccine peptides could activate CTL responses. The discrepancy between the results of the clinical trial and our *in silico* trial is based on the different populations, since the populations of each had subjects with different HLA genotypes. However, the response rate results provided by the *in silico* trial on the Model Population are a good prediction for the response rate outcome in the clinical trial population.

Therefore disclosed herein is a method of conducting an *in silico* trial for a vaccine or immunotherapy composition having one or more active ingredient polypeptides, wherein the *in silico* trial predicts the clinical response rate of a human population, the method comprising
(i) defining an *in silco* model human population comprising a plurality of subjects defined by HLA class I genotype, wherein the *in silico* model human population corresponds to or is representative of said human population;
(ii) determining for each subject in *the in silico* model human population whether the one or more active ingredient polypeptides comprise at least two different sequences each of which is a T cell epitope capable of binding to at least two, or at least three HLA class I of the subject; and
(iv) predicting the clinical response rate of said human population, wherein a higher proportion of *the in silico* model human population that meet the requirements of step (ii) predicts a higher response rate. The proportion of the *in silico* model human population that meet the requirements of step (ii) may correspond to the predicted response rate.

### Preparation of pharmaceutical compositions for an individual human subject

In some aspects the invention relates to a method of preparing a human subject-specific drug and immunogenic composition or pharmaceutical composition and compositions that may be obtained by that method. The composition has as active ingredients one or more peptides comprising two or more polypeptide fragments that have been identified as immunogenic for the subject by the method described above. The composition is a personalised medicine to prevent, diagnose, alleviate, treat, or cure a disease of an individual.

The method comprises selecting, for each fragment, a sequence of up to 50, 45, 40, 35, 30, 25, 20, 19, 18, 17, 16, 15, 14, 13, 12, 11, 10 or 9 consecutive amino acids of the polypeptide, which consecutive amino acids comprise the amino acid sequence of the fragment.

The inventors have discovered that the presence in a vaccine or immunotherapy composition of at least two polypeptide fragments (epitopes) that can bind to at least three HLA of an individual (≥2 PEPI3+) is predictive for a clinical response. In other words, if ≥2 PEPI3+ can be identified within the active ingredient polypeptide(s) of a vaccine or immunotherapy composition, then an individual is a likely clinical responder. The at least two multiple HLA-binding PEPIs of the composition polypeptides may both target a single antigen (e.g a polypeptide vaccine comprising two multiple HLA-binding PEPIs derived from a single tumor associated antigen targeted by the vaccine) or may target different antigens (e.g. a polypeptide vaccine comprising one multiple HLA-binding PEPI derived from one tumor associated antigen and a second multiple HLA-binding PEPI derived from a different tumor associated antigen).

Without wishing to be bound by theory, the inventors believe that one reason for the increased likelihood of deriving clinical benefit from a vaccine or immunotherapy composition comprising at least two multiple-HLA binding PEPIs, is that tumor similarly to HIV consist of heterogeneous cell population expressing different tumor associated antigens and therefore effective tumor therapy similarly to an effective HIV therapy must consist of combination of drug with different targets. In addition, a patient is less likely to develop resistance to the composition through mutation of the target PEPI(s). The likelihood of developing resistance is decreased when more multiple HLA-binding PEPIs are included.

Accordingly, in some cases the method comprises selecting a total of 3, 4, 5, 6, 7, 8, 9, 10, 11, 12, 13, 14, 15, 16, 17, 18, 19, 20, 21, 22, 23, 24 or 25 or more different fragments of one or more polypeptides, wherein each fragment has been identified as immunogenic for the subject by the methods described herein, and selecting for each fragment a sequence of up to 50 consecutive amino acids of the polypeptide, which consecutive amino acids comprise the amino acid sequence of the fragment.

Currently most vaccines and immunotherapy compositions target only a single polypeptide antigen. However according to the present invention it is in some cases beneficial for two or more of the polypeptide fragments that have been identified as immunogenic for the subject to be from different target polypeptide antigens. For example, most cancers or tumors are heterogeneous, meaning that different cancer or tumor cells of a subject express different antigens. The anti-cancer immunogenic compositions that are most likely to be effective are those that target multiple antigens expressed by the tumor, and therefore more cancer or tumor cells.

Therefore in some cases in accordance with the invention, the first and second and optionally one or more or each of the further immunogenic polypeptide fragments are from different polypeptide antigens, for example different cancer- or tumor-associated antigens. In some cases the immunogenic polypeptide fragments are from a total of 2, 3, 4, 5, 6, 7, 8, 9, 10, 11, 12, 13, 14, 15, 16, 17, 18, 19, 20, 21, 22, 23, 24 or 25 or more different polypeptide antigens. In some cases one or more or each of the polypeptides is a cancer testis antigen. In some cases the immunogenic polypeptide fragments are from a total of 3 different polypeptide antigens, optionally wherein 1, 2, or all three are CTAs, or from 4 different polypeptide antigens, optionally wherein 1, 2, 3 or all four are CTAs, or from 5 different polypeptide antigens, optionally wherein 1, 2, 3, 4 or all five are CTAs, or from 6 different polypeptide antigens, optionally wherein 1, 2, 3, 4, 5 or all six are CTAs, or from 7 different polypeptide antigens, optionally wherein 1, 2, 3, 4, 5, 6 or all 7 are CTAs.

In some cases one or more or each of the immunogenic polypeptide fragments is from a polypeptide that is present in a sample taken from the specific human subject. This indicates that the polypeptide is expressed in the subject, for example a cancer- or tumor-associated antigen or a cancer testis antigen expressed by cancer cells of the subject. In some cases one or more or each of the polypeptides is a mutational neoantigen of the subject and one or more or each fragment comprises a neoantigen specific mutation. Since mutational neoantigens are subject specific, a composition that targets one or more neoantigen specific is personalised with regard to both their specific disease and their specific HLA set.

In other cases one or more or each of the immunogenic polypeptide fragments is from a polypeptide that is not generally expressed or is minimally expressed in normal healthy cells or tissue, but is expressed in a high proportion of (with a high frequency in) subjects having a type of cancer or a cancer derived from a particular cell type or tissue. Non-limiting examples include melanoma and non-melanoma skin, lung, prostate, breast, colorectal, kidney, bladder, stomach, liver, cervix uteri, oesophagus, non-Hodgkin lymphoma, leukemia, pancreas, corpus uteri, lip, oral cavity, thyroid, brain, nervous system, ovary, gallbladder, larynx, pharynx, myeloma, nasopharynx, Hodgkin lymphoma, testis and Kaposi sarcoma. Alternatively, the polypeptide may be expressed at low levels in normal healthy cells, but at high levels in subjects having the type of cancer. In some cases the cancer- or tumor-associated antigen is expressed in, or expressed at a high level relative to normal healthy cells or subjects in, at least 2%, 5%, 10%, 20%, 30%, 40%, 50%, 60%, 70, 80%, 90% of cancers of a particular type or tissue, or the cancers of a subject-matched population. The expression frequencies can be determined from published figures and scientific publications.

In some cases two or more of the immunogenic polypeptide fragments are from different cancer- or tumor-associated antigens that are each expressed with a high frequency in subjects having a type of cancer or a cancer derived from a particular cell type or tissue. In some cases the immunogenic polypeptide fragments are from a total of 2, 3, 4, 5, 6, 7, 8, 9, 10, 11, 12, 13, 14, 15, 16, 17, 18, 19, 20, 21, 22, 23, 24 or 25 different such cancer- or tumor-associated polypeptides. In some cases one or more or each of the polypeptides is a cancer testis antigen (CTA). In some cases one or more or each of the polypeptides is selected from the antigens listed in Table 1 and/or the antigens listed in Table 4 above.
The methods of the invention predict with a high degree of certainty which cancer- or tumor-associated antigens could be effectively targeted by a vaccine or immunotherapy composition, if the cancer- or tumor-associated antigen is in fact expressed in the subject. By using the methods of the invention and targeting multiple cancer- or tumor-associated polypeptide antigens, it becomes unnecessary to determine actual expression of potential target cancer- or tumor-associated antigens in a subject, for example in a tumor sample obtained from the subject. The skilled person is able to combine data concerning the expression frequency of common cancer- or tumor-associated antigens in the population (or a subject's disease matched subpopulation) with the antigen specific PEPI count (PEPI2+ or PEPI3+) in order to predict the likelihood that a subject will have an immune response or a clinical response to a composition comprising two or more such PEPIs. Such an approach is useful for making personalised medicines such vaccine or immunotherapy compositions to prevent, diagnose, alleviate, treat, or cure a disease of an individual.

In some cases one or more of the polypeptide fragments comprises an amino acid sequence that is a T cell epitope capable of binding to at least two, or at least three HLA class I of the subject and one or more of the polypeptide fragments comprises an amino acid sequence that is a T cell epitope capable of binding to at least two, or at least three, or at least four HLA class II of the subject, wherein the HLA class I and HLA class II binding fragments may optionally overlap. A composition prepared by such a method may elicit both a cytotoxic T cell response and a helper T cell response in the specific human subject.

### Immunogenic and Pharmaceutical Compositions, Methods of Treatment and Modes of Administration

The immunogenic or pharmaceutical compositions described herein may comprise, in addition to one or more immunogenic peptides, a pharmaceutically acceptable excipient, carrier, diluent, buffer, stabiliser, preservative, adjuvant or other materials well known to those skilled in the art. Such materials are preferably non-toxic and preferably not interfere with the pharmaceutical activity of the active ingredient(s). The pharmaceutical carrier or diluent may be, for example, water containing solutions. The precise nature of the carrier or other material may depend on the route of administration, e.g. oral, intravenous, cutaneous or subcutaneous, nasal, intramuscular, intradermal, and intraperitoneal routes.

The pharmaceutical compositions of the invention may comprise one or more "pharmaceutically acceptable carriers". These are typically large, slowly metabolized macromolecules such as proteins, saccharides, polylactic acids, polyglycolic acids, polymeric amino acids, amino acid copolymers, sucrose (Paoletti et al., 2001, Vaccine, 19:2118), trehalose (WO 00/56365), lactose and lipid aggregates (such as oil droplets or liposomes). Such carriers are well known to those of ordinary skill in the art. The pharmaceutical compositions may also contain diluents, such as water, saline, glycerol, etc. Additionally, auxiliary substances, such as wetting or emulsifying agents, pH buffering substances, and the like, may be present. Sterile pyrogen-free, phosphate buffered physiologic saline is a typical carrier (Gennaro, 2000, Remington: The Science and Practice of Pharmacy, 20th edition, ISBN:0683306472).

The pharmaceutical compositions of the disclosure may be lyophilized or in aqueous form, i.e. solutions or suspensions. Liquid formulations of this type allow the compositions to be administered direct from their packaged form, without the need for reconstitution in an aqueous medium, and are thus ideal for injection. The pharmaceutical compositions may be presented in vials, or they may be presented in ready filled syringes. The syringes may be supplied with or without needles. A syringe will include a single dose, whereas a vial may include a single dose or multiple doses.

Liquid formulations of the disclosure are also suitable for reconstituting other medicaments from a lyophilized form. Where a pharmaceutical composition is to be used for such extemporaneous reconstitution, the disclosure provides a kit, which may comprise two vials, or may comprise one ready-filled syringe and one vial, with the contents of the syringe being used to reconstitute the contents of the vial prior to injection.

The pharmaceutical compositions of the disclosure may include an antimicrobial, particularly when packaged in a multiple dose format. Antimicrobials may be used, such as 2-phenoxyethanol or parabens (methyl, ethyl, propyl parabens). Any preservative is preferably present at low levels. Preservative may be added exogenously and/or may be a component of the bulk antigens which are mixed to form the composition (e.g. present as a preservative in pertussis antigens).

The pharmaceutical compositions of the disclosure may comprise detergent e.g. Tween (polysorbate), DMSO (dimethyl sulfoxide), DMF (dimethylformamide). Detergents are generally present at low levels, e.g. <0.01%, but may also be used at higher levels, e.g. 0.01 - 50%.

The pharmaceutical compositions of the disclosure may include sodium salts (e.g. sodium chloride) and free phosphate ions in solution (e.g. by the use of a phosphate buffer).

In certain embodiments, the pharmaceutical composition may be encapsulated in a suitable vehicle either to deliver the peptides into antigen presenting cells or to increase the stability. As will be appreciated by a skilled artisan, a variety of vehicles are suitable for delivering a pharmaceutical composition of the invention. Non-limiting examples of suitable structured fluid delivery systems may include nanoparticles, liposomes, microemulsions, micelles, dendrimers and other phospholipid-containing systems. Methods of incorporating pharmaceutical compositions into delivery vehicles are known in the art.

In order to increase the immunogenicity of the composition, the pharmacological compositions may comprise one or more adjuvants and/or cytokines.

Suitable adjuvants include an aluminum salt such as aluminum hydroxide or aluminum phosphate, but may also be a salt of calcium, iron or zinc, or may be an insoluble suspension of acylated tyrosine, or acylated sugars, or may be cationically or anionically derivatised saccharides, polyphosphazenes, biodegradable microspheres, monophosphoryl lipid A (MPL), lipid A derivatives (e.g. of reduced toxicity), 3-O-deacylated MPL [3D-MPL], quil A, Saponin, QS21, Freund's Incomplete Adjuvant (Difco Laboratories, Detroit, Mich.), Merck Adjuvant 65 (Merck and Company, Inc., Rahway, N.J.), AS-2 (Smith-Kline Beecham, Philadelphia, Pa.), CpG oligonucleotides, bioadhesives and mucoadhesives, microparticles, liposomes, polyoxyethylene ether formulations, polyoxyethylene ester formulations, muramyl peptides or imidazoquinolone compounds (e.g. imiquamod and its homologues). Human immunomodulators suitable for use as adjuvants in the disclosure include cytokines such as interleukins (e.g. IL-1, IL-2, IL-4, IL-5, IL-6, IL-7, IL-12, etc), macrophage colony stimulating factor (M-CSF), tumour necrosis factor (TNF), granulocyte, macrophage colony stimulating factor (GM-CSF) may also be used as adjuvants.

In some embodiments, the compositions comprise an adjuvant selected from the group consisting of Montanide ISA-51 (Seppic, Inc., Fairfield, N.J., United States of America), QS-21 (Aquila Biopharmaceuticals, Inc., Lexington, Mass., United States of America), tetanus helper peptides, GM-CSF, cyclophosamide, bacillus Calmette-Guerin (BCG), corynbacterium parvum, levamisole, azimezone, isoprinisone, dinitrochlorobenezene (DNCB), keyhole limpet hemocyanins (KLH), Freunds adjuvant (complete and incomplete), mineral gels, aluminum hydroxide (Alum), lysolecithin, pluronic polyols, polyanions, peptides, oil emulsions, dinitrophenol, diphtheria toxin (DT).

By way of example, the cytokine may be selected from the group consisting of a transforming growth factor (TGF) such as but not limited to TGF-α and TGF-β; insulin-like growth factor-I and/or insulin-like growth factor-II; erythropoietin (EPO); an osteoinductive factor; an interferon such as but not limited to interferon-.α, -β, and -γ; a colony stimulating factor (CSF) such as but not limited to macrophage-CSF (M-CSF); granulocyte-macrophage-CSF (GM-CSF); and granulocyte-CSF (G-CSF). In some embodiments, the cytokine is selected from the group consisting of nerve growth factors such as NGF-β; platelet-growth factor; a transforming growth factor (TGF) such as but not limited to TGF-α. and TGF-β; insulin-like growth factor-I and insulin-like growth factor-II; erythropoietin (EPO); an osteoinductive factor; an interferon (IFN) such as but not limited to IFN-α, IFN-β, and IFN-γ; a colony stimulating factor (CSF) such as macrophage-CSF (M-CSF); granulocyte-macrophage-CSF (GM-CSF); and granulocyte-CSF (G-CSF); an interleukin (I1) such as but not limited to IL-1, IL-1.alpha., IL-2, IL-3, IL-4, IL-5, IL-6, IL-7, IL-8, IL-9, IL-10, IL-11, IL-12; IL-13, IL-14, IL-15, IL-16, IL-17, IL-18; LIF; kit-ligand or FLT-3; angiostatin; thrombospondin; endostatin; a tumor necrosis factor (TNF); and LT.

It is expected that an adjuvant or cytokine can be added in an amount of about 0.01 mg to about 10 mg per dose, preferably in an amount of about 0.2 mg to about 5 mg per dose. Alternatively, the adjuvant or cytokine may be at a concentration of about 0.01 to 50%, preferably at a concentration of about 2% to 30%.

In certain aspects, the pharmaceutical compositions of the disclosure are prepared by physically mixing the adjuvant and/or cytokine with the PEPIs under appropriate sterile conditions in accordance with known techniques to produce the final product.

Examples of suitable compositions of the invented polypeptide fragments and methods of administration are provided in Esseku and Adeyeye (2011) and Van den Mooter G. (2006). Vaccine and immunotherapy composition preparation is generally described in Vaccine Design ("The subunit and adjuvant approach" (eds Powell M. F. & Newman M. J. (1995) Plenum Press New York). Encapsulation within liposomes, which is also envisaged, is described by Fullerton, US Patent 4,235,877.

Polynucleotide or oligonucleotide components may be naked nucleotide sequences or be in combination with cationic lipids, polymers or targeting systems. They may be delivered by any available technique. For example, the polynucleotide or oligonucleotide may be introduced by needle injection, preferably intradermally, subcutaneously or intramuscularly. Alternatively, the polynucleotide or oligonucleotide may be delivered directly across the skin using a delivery device such as particle-mediated gene delivery. The polynucleotide or oligonucleotide may be administered topically to the skin, or to mucosal surfaces for example by intranasal, oral, or intrarectal administration.

Uptake of polynucleotide or oligonucleotide constructs may be enhanced by several known transfection techniques, for example those including the use of transfection agents. Examples of these agents include cationic agents, for example, calcium phosphate and DEAE-Dextran and lipofectants, for example, lipofectam and transfectam. The dosage of the polynucleotide or oligonucleotide to be administered can be altered.

Administration is typically in a "prophylactically effective amount" or a "therapeutically effective amount" (as the case may be, although prophylaxis may be considered therapy), this being sufficient to result in a clinical response or to show clinical benefit to the individual, e.g. an effective amount to prevent or delay onset of the disease or condition, to ameliorate one or more symptoms, to induce or prolong remission, or to delay relapse or recurrence.

The dose may be determined according to various parameters, especially according to the substance used; the age, weight and condition of the individual to be treated; the route of administration; and the required regimen. The amount of antigen in each dose is selected as an amount which induces an immune response. A physician will be able to determine the required route of administration and dosage for any particular individual. The dose may be provided as a single dose or may be provided as multiple doses, for example taken at regular intervals, for example 2, 3 or 4 doses administered hourly. Typically peptides, polynucleotides or oligonucleotides are typically administered in the range of 1 pg to 1 mg, more typically 1 pg to 10 µg for particle mediated delivery and 1 µg to 1 mg, more typically 1-100 µg, more typically 5-50 µg for other routes. Generally, it is expected that each dose will comprise 0.01-3 mg of antigen. An optimal amount for a particular vaccine can be ascertained by studies involving observation of immune responses in subjects.

Examples of the techniques and protocols mentioned above can be found in Remington's Pharmaceutical Sciences, 20th Edition, 2000, pub. Lippincott, Williams & Wilkins.

In some cases in accordance with the invention, more than one peptide or composition of peptides is administered. Two or more pharmaceutical compositions may be administered together/simultaneously and/or at different times or sequentially. Thus, the invention includes sets of pharmaceutical compositions and uses thereof. The use of combination of different peptides, optionally targeting different antigens, is important to overcome the challenges of genetic heterogeneity of tumors and HLA heterogeneity of individuals. The use of peptides of the invention in combination expands the group of individuals who can experience clinical benefit from vaccination. Multiple pharmaceutical compositions of PEPIs, manufactured for use in one regimen, may define a drug product.

Routes of administration include but are not limited to intranasal, oral, subcutaneous, intradermal, and intramuscular. The subcutaneous administration is particularly preferred. Subcutaneous administration may for example be by injection into the abdomen, lateral and anterior aspects of upper arm or thigh, scapular area of back, or upper ventrodorsal gluteal area.

The skilled artisan will recognize that compositions of the invention may also be administered in one, or more doses, as well as, by other routes of administration. For example, such other routes include, intracutaneously, intravenously, intravascularly, intraarterially, intraperitnoeally, intrathecally, intratracheally, intracardially, intralobally, intramedullarly, intrapulmonarily, and intravaginally. Depending on the desired duration of the treatment, the compositions according to the invention may be administered once or several times, also intermittently, for instance on a monthly basis for several months or years and in different dosages.

Solid dosage forms for oral administration include capsules, tablets, caplets, pills, powders, pellets, and granules. In such solid dosage forms, the active ingredient is ordinarily combined with one or more pharmaceutically acceptable excipients, examples of which are detailed above. Oral preparations may also be administered as aqueous suspensions, elixirs, or syrups. For these, the active ingredient may be combined with various sweetening or flavoring agents, coloring agents, and, if so desired, emulsifying and/or suspending agents, as well as diluents such as water, ethanol, glycerin, and combinations thereof.

One or more compositions of the invention may be administered alone or in combination with other pharmacological compositions or treatments, for example chemotherapy and/or immunotherapy and/or vaccine. The other therapeutic compositions or treatments may for example be one or more of those discussed herein, and may be administered either simultaneously or sequentially with the composition or treatment of the invention.

As used herein, "immunotherapy" is the treatment of a disease or condition by inducing or enhancing an immune response in an individual. In certain embodiments, immunotherapy refers to a therapy that comprises the administration of one or more drugs to an individual to elicit T cell responses. In a specific embodiment, immunotherapy refers to a therapy that comprises the administration or expression of polypeptides that contain one or more PEPIs to an individual to elicit a T cell response to recognize and kill cells that display the one or more PEPIs on their cell surface in conjunction with a class I HLA. In another specific embodiment, immunotherapy comprises the administration of one or more PEPIs to an individual to elicit a cytotoxic T cell response against cells that display tumor associated antigens (TAAs) or cancer testis antigens (CTAs) comprising the one or more PEPIs on their cell surface. In another embodiment, immunotherapy refers to a therapy that comprises the administration or expression of polypeptides that contain one or more PEPIs presented by class II HLAs to an individual to elicit a T helper response to provide co-stimulation to cytotoxic T cells that recognize and kill diseased cells that display the one or more PEPIs on their cell surface in conjunction with a class I HLAs. In still another specific embodiment, immunotherapy refers to a therapy that comprises administration of one or more drugs to an individual that re-activate existing T cells to kill target cells. The theory is that the cytotoxic T cell response will eliminate the cells displaying the one or more PEPIs, thereby improving the clinical condition of the individual. In some instances, immunotherapy may be used to treat tumors. In other instances, immunotherapy may be used to treat intracellular pathogen-based diseases or disorders.

### Examples

### Example 1 - HLA-epitope binding prediction process and validation

Predicted binding between particular HLA and epitopes (9mer peptides) was based on the Immune Epitope Database tool for epitope prediction (www.iedb.org).

Our HLA I-epitope binding prediction process was validated by comparison with well-characterised HLA I-epitope pairs determined by laboratory experiments. A dataset was compiled of HLA I-epitope pairs reported in peer reviewed publications or public immunological databases.

We determined the rate of agreement with the experimentally determined dataset (Table 6). The binding HLA I-epitope pairs of the dataset were correctly predicted with a 93% probability. Coincidentally the non-binding HLA I-epitope pairs were also correctly predicted with a 93% probability.

**Table 6. Analytical specificity and sensitivity of the HLA-epitope binding prediction process.**

| ***HLA*-*epitope pairs*** | ***True epitopes (n=327)** (Binder match)* | ***False epitopes (n=100)** (Non-binder match)* |
|---|---|---|
| ***HIV*** | 91% (32) | 82% (14) |
| ***Viral*** | 100% (35) | 100% (11) |
| ***Tumor*** | 90% (172) | 94% (32) |
| ***Other (fungi*, *bacteria*, *etc.)*** | 100% (65) | 95% (36) |
| ***All*** | ***93% (304)*** | ***93% (93)*** |

Next we determined the accuracy of the prediction of multiple HLA binding epitopes. Based on the analytical specificity and sensitivity using the 93% probability for both true positive and true negative prediction and 7% (=100% - 93%) probability for false positive and false negative prediction, the probability of the existence of a multiple HLA binding epitope in a person can be calculated. The probability of multiple HLA binding to an epitope (7) shows the relationship between the number of HLAs binding an epitope and the expected minimum number of real binding. Per PEPI definition three is the expected minimum number of HLA to bind an epitope **(bold).**

**Table 7. Accuracy of multiple HLA binding epitopes predictions.**

| **Expected minimum number of real HLA binding** | **Predicted number of HLAs binding to an epitope** | | | | | | |
|---|---|---|---|---|---|---|---|
| | **0** | **1** | **2** | **3** | **4** | **5** | **6** |
| **1** | 35% | 95% | 100% | 100% | 100% | 100% | 100% |
| **2** | 6% | 29% | 90% | 99% | 100% | 100% | 100% |
| **3** | **1%** | **4%** | **22%** | **84%** | **98%** | **100%** | **100%** |
| **4** | 0% | 0% | 2% | 16% | 78% | 96% | 99% |
| **5** | 0% | 0% | 0% | 1% | 10% | 71% | 94% |
| **6** | 0% | 0% | 0% | 0% | 0% | 5% | 65% |

The validated HLA-epitope binding prediction process was used to determine all HLA-epitope binding pairs described in the Examples below.

### Example 2 - Epitope presentation by multiple HLA predicts cytotoxic T lymphocyte (CTL) response

This study investigates whether the presentation of one or more epitopes of a polypeptide antigen by one or more HLA I of an individual is predictive for a CTL response.

The study was carried out by retrospective analysis of six clinical trials, conducted on 71 cancer and 9 HIV-infected patients (Table 8)¹⁻⁷. Patients from these studies were treated with an HPV vaccine, three different NY-ESO-1 specific cancer vaccines, one HIV-1 vaccines and a CTLA-4 specific monoclonal antibody (Ipilimumab) that was shown to reactivate CTLs against NY-ESO-1 antigen in melanoma patients. All of these clinical trials measured antigen specific CD8+ CTL responses (immunogenicity) in the study subjects after vaccination. In some cases, correlation between CTL responses and clinical responses were reported.

No patient was excluded from the retroactive study for any reason other than data availability. The 157 patient datasets (Table H) were randomized with a standard random number generator to create two independent cohorts for training and evaluation studies. In some cases the cohorts contained multiple datasets from the same patient, resulting in a training cohort of 76 datasets from 48 patients and a test/validation cohort of 81 datasets from 51 patients.

**Table 8. Summary of patient datasets**

| **Clinical trial** | **Immunotherapy** | **Target Antigen** | **Disease** | **# Patients*** | **# Data sets** (#antigen x #patient) | **Immunoassay performed in the clinical trials**** | **HLA genotyping method** | **Ref** |
|---|---|---|---|---|---|---|---|---|
| 1 | VGX-3100 | HPV16-E6 | Cervical cancer | 17/18 | 5 x 17 | IFN-γ ELISPOT | High Resolution SBT | 1 |
| | | HPV16-E7 | | | | | | |
| | | HPV18-E6 | | | | | | |
| | | HPV18-E7 | | | | | | |
| | | HPV16/18 | | | | | | |
| 2 | HIVIS vaccine | HIV-1 Gag | AIDS | 9/12 | 2 x 9 | IFN-γ ELISPOT | Low-Medium Resolution SSO | 2 |
| | | HIV-1 RT | | | | | | |
| 3 | rNY-ESO-1 | NY-ESO-1 | Breast-and ovarian cancers, melanoma and | 18/18 | 1 x 18 | In vitro and Ex vivo IFN-γ ELISPOT | High Resolution SBT | 3 |
| | | | | | | | | 4 |
| | | | sarcoma | | | | | |
| 4 | Ipilimumab | NY-ESO-1 | Metastatic melanoma | 19/20 | 1 x 19 | ICS after T-cell stimulation | Low to medium resolution typing, SSP of genomic DNA, high resolution sequencing | 5 |
| 5 | NY-ESO-1f | NY-ESO-1 (91-110) | Esophageal-, non-small-cell lung-and gastric cancer | 10/10 | 1 x 10 | ICS after T-cell stimulation | SSO probing and SSP of genomic DNA | 6 |
| 6 | NY-ESO-1 overlapping peptides | NY-ESO-1 (79-173) | Esophageal-and lung cancer, malignant melanoma | 7/9 | 1 x 7 | ICS after T-cell stimulation | SSO probing and SSP of genomic DNA | 7 |
| **Total** | **6** | **7** | | **80** | **157** | **N/A** | | |
| | *Number of patients used in the retrospective analysis from the original number of patient of the clinical trials. | | | | | | | |
| | **Immunoassays are based on T cell stimulation with antigen-specific peptide pools and quantify the released cytokines by different techniques. | | | | | | | |
| | CT: Clinical trial; SBT: Sequence Based Typing; SSO: Sequence-Specific Oligonucleotide; ICS: Intracellular cytokine staining; SSP: Sequence-specific priming | | | | | | | |

We compared the reported CTL responses of the training dataset with the HLA I restriction profile of epitopes (9mers) of the vaccine antigens. The antigen sequences and the HLA I genotype of each patient were obtained from publicly available protein sequence databases or peer reviewed publications and the HLA I-epitope binding prediction process was blinded to patients' clinical CTL response data. The number of epitopes from each antigen predicted to bind to at least 1 (PEPI1+), or at least 2 (PEPI2+), or at least 3 (PEPI3+), or at least 4 (PEPI4+), or at least 5 (PEPI5+), or all 6 (PEPI6) HLA class I molecules of each patient was determined and the number of HLA bound were used as classifiers for the reported CTL responses. The true positive rate (sensitivity) and true negative rate (specificity) were determined from the training dataset for each classifier (number of HLA bound) separately.

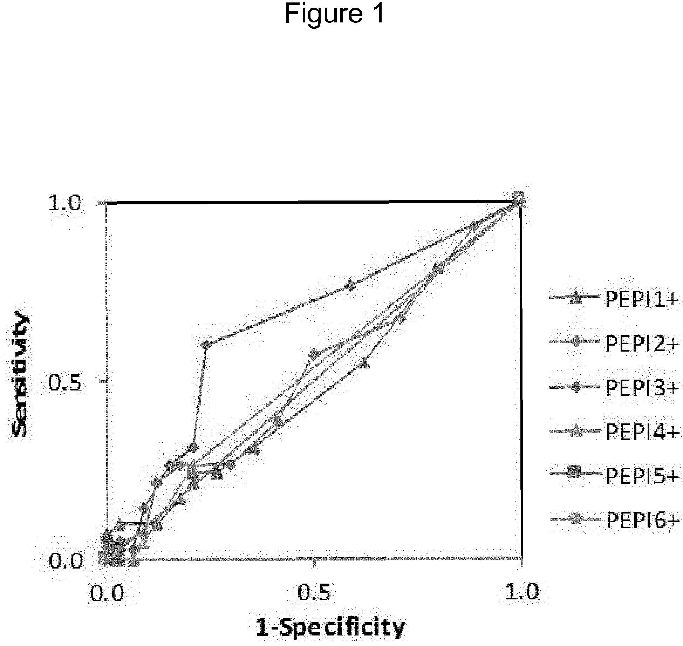

ROC analysis was performed for each classifier. In a ROC curve, the true positive rate (Sensitivity) is plotted in function of the false positive rate (1-Specificity) for different cut-off points (FIG. 1). Each point on the ROC curve represents a sensitivity/specificity pair corresponding to a particular decision threshold (epitope (PEPI) count). The area under the ROC curve (AUC) is a measure of how well the classifier can distinguish between two diagnostic groups (CTL responder or non-responder).

The analysis unexpectedly revealed that predicted epitope presentation by multiple class I HLAs of a subject (PEPI2+, PEPI3+, PEPI4+, PEPI5+, or PEPI6), was in every case a better predictor of CTL response than epitope presentation by merely one or more HLA class I (PEPI1+, AUC = 0.48, Table 9).

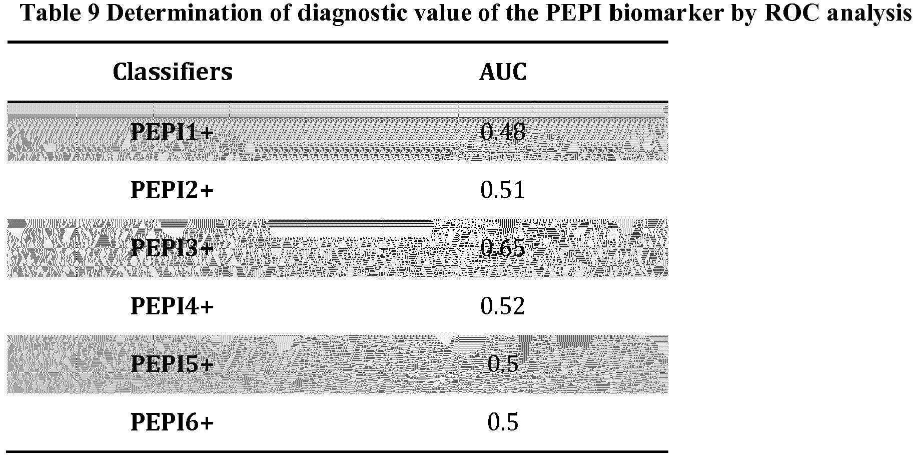

The CTL response of an individual was best predicted by considering the epitopes of an antigen that could be presented by at least 3 HLA class I of an individual (PEPI3+, AUC = 0.65, Table 9). The threshold count of PEPI3+ (number of antigen-specific epitopes presented by 3 or more HLA of an individual) that best predicted a positive CTL response was 1 (Table 10). In other words, at least one antigen-derived epitope is presented by at least 3 HLA class I of a subject (≥1 PEPI3+), then the antigen can trigger at least one CTL clone, and the subject is a likely CTL responder. Using the ≥1 PEPI3+ threshold to predict likely CTL responders ("≥1 PEPI3+ Test") provided 76% diagnostic sensitivity (Table 10).

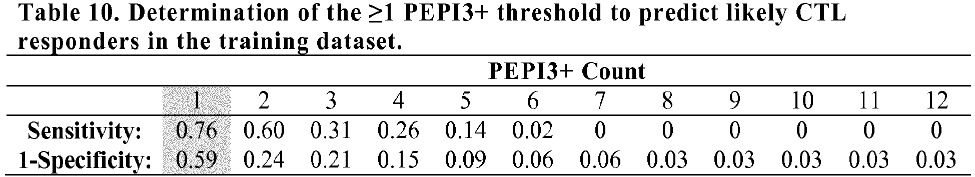

### Example 3 - Validation of the ≥1 PEPI3+ Test

The test cohort of 81 datasets from 51 patients was used to validate the ≥1 PEPI3+ threshold to predict an antigen-specific CTL response. For each dataset in the test cohort it was determined whether the ≥1 PEPI3+ threshold was met (at least one antigen-derived epitope presented by at least three class I HLA of the individual). This was compared with the experimentally determined CTL responses reported from the clinical trials (Table 11).

The clinical validation demonstrated that a PEPI3+ peptide induce CTL response in an individual with 84% probability. 84% is the same value that was determined in the analytical validation of the PEPI3+ prediction, epitopes that binds to at least 3 HLAs of an individual (Table 7). These data provide strong evidences that immune responses are induced by PEPIs in individuals.

**Table 11. Diagnostic performance characteristics of the ≥1 PEPI3+ Test (n=81).**

| **Performance characteristic** | | **Description** | **Result** |
|---|---|---|---|
| **Positive predictive value (PPV)** | 100%[A/(A + B)] | The likelihood that an individual that meets the ≥1 PEPI3+ threshold has antigen-specific CTL responses after treatment with immunotherapy. | ***84%*** |
| **Sensitivity** | 100%[A / (A+C)] | The proportion of subjects with antigen-specific CTL responses after treatment with immunotherapy who meet the ≥1 PEPI3+ threshold. | ***75%*** |
| **Specificity** | 100%[D / (B + D)] | The proportion of subjects without antigen-specific CTL responses after treatment with immunotherapy who do not meet the ≥1 PEPI3+ threshold. | ***55%*** |
| **Negative predictive value (NPV)** | 100%[D/(C +D)] | The likelihood that an individual who does not meet the ≥1 PEPI3+ threshold does not have antigen-specific CTL responses after treatment with immunotherapy. | ***42%*** |
| **Overall percent agreement (OPA)** | 100%[(A + D)/ N] | The percentage of predictions based on the ≥1 PEPI3+ threshold that match the experimentally determined result, whether positive or negative. | ***70%*** |
| **Fisher's exact (p)** | | | ***0.01*** |

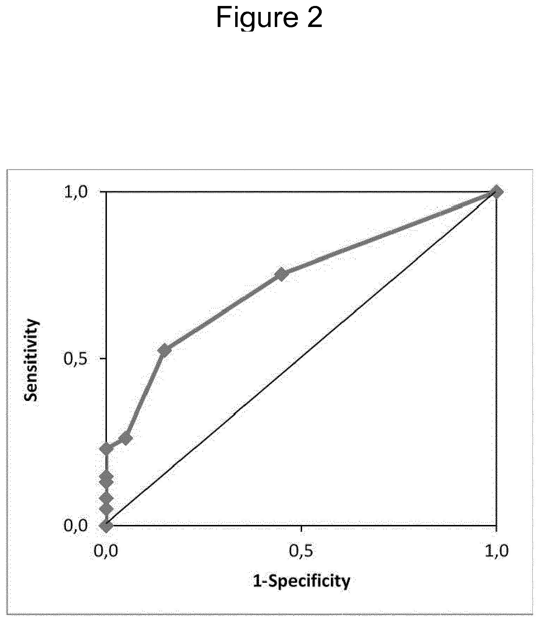

ROC analysis determined the diagnostic accuracy, using the PEPI3+ count as cut-off values (Fig. 2). The AUC value = 0.73. For ROC analysis an AUC of 0.7 to 0.8 is generally considered as fair diagnostic.

A PEPI3+ count of at least 1 (≥1 PEPI3+) best predicted a CTL response in the test dataset (Table 12). This result confirmed the threshold determined during the training (Table 9).

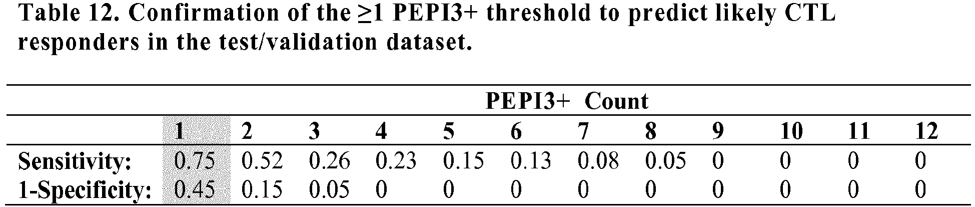

### Example 4 - The ≥1 PEPI3+ Test predicts CD8+ CTL reactivities

This study compares the ≥1 PEPI3+ Test with the prior art method for predicting a specific human subject's CTL response to peptide antigens.

We determined from DNA specimens the HLA genotypes of 28 cervical cancer and VIN-3 patients that received the HPV-16 synthetic long peptide vaccine (LPV) in two different clinical trials ^{8 9 10}. The LPV consists of long peptides covering the HPV-16 viral oncoproteins E6 and E7. The amino acid sequence of the LPV was obtained from these publications. The publications also report the T cell responses of each vaccinated patient to pools of overlapping peptides of the vaccine.

We identified for each patient epitopes (9mers) of the LPV that are presented by at least three patient class I HLA (PEPI3+s) and determined their distribution among the peptide pools. Peptides that comprised at least one PEPI3+ (≥1 PEPI3+) were predicted to induce a CTL response. Peptides that comprised no PEPI3+ were predicted not to induce a CTL response.

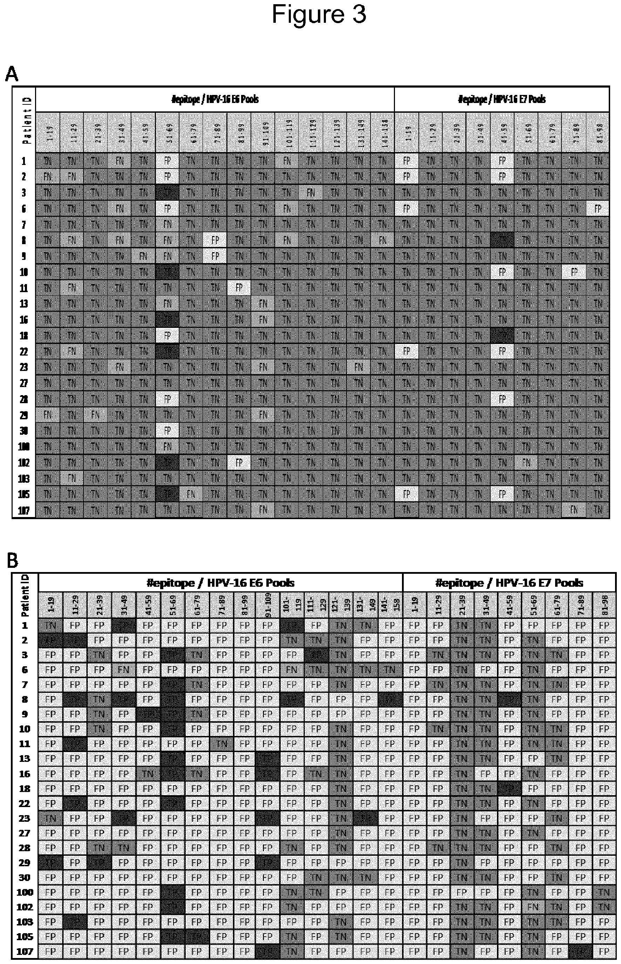

The ≥1 PEPI3+ Test correctly predicted 489 out of 512 negative CTL responses and 8 out of 40 positive CTL responses measured after vaccination (Fig. 3A). Overall, the agreement between the ≥1 PEPI3+ Test and experimentally determined CD8+ T cell reactivity was 90% (p<0.001).

We also determined for each patient the distribution among the peptide pools of epitopes that are presented by at least one patient class I HLA (≥1 PEPI1+, HLA restricted epitope prediction, prior art method). ≥1 PEPI1 + correctly predicted 116 out of 512 negative CTL responses and 37 out of 40 positive CTL responses measured after vaccination (FIG. 3B). Overall, the agreement between the HLA restricted epitope prediction (≥1 PEPI1+) and CD8+ T cell reactivity was 28% (not significant).

### Example 5 - Prediction of HLA class II restricted CD4+ helper T cell epitopes

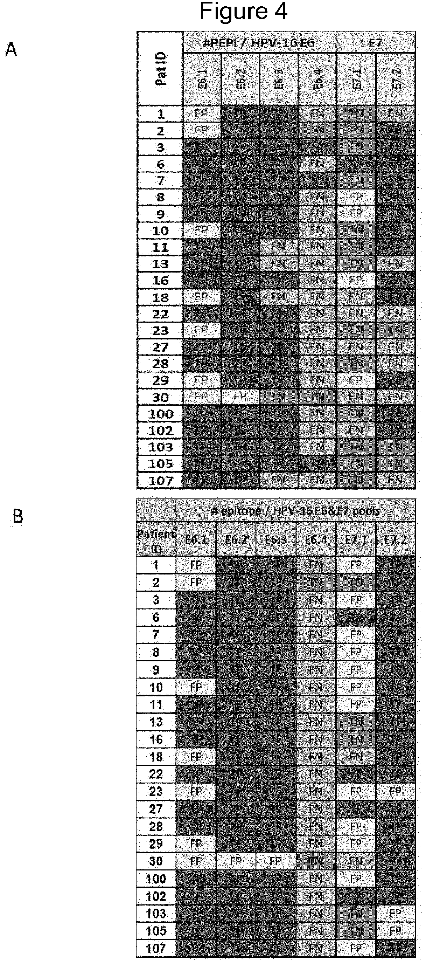

The 28 cervical cancer and VIN-3 patients that received the HPV-16 synthetic long peptide vaccine (LPV) in two different clinical trials (as detailed in Example 4) were investigated for CD4+ T helper responses following LPV vaccination (FIG. 4). The sensitivity of the prediction of HLA class II restricted epitopes was 78%, since the State of Art tool predicted 84 positive responses (positive CD4+ T cell reactivity to a peptide pool for a person's DP alleles) out of 107 (sensitivity = 78%). The specificity was 22% since it could rule out 7 negative responses out of 31. Overall, the agreement between HLA-restricted class II epitope prediction and CD4+ T cell reactivity was 66%, which was statistically not significant.

### Example 6 - The ≥1 PEPI3+ Test predicts T cell responses to full length LPV polypeptides

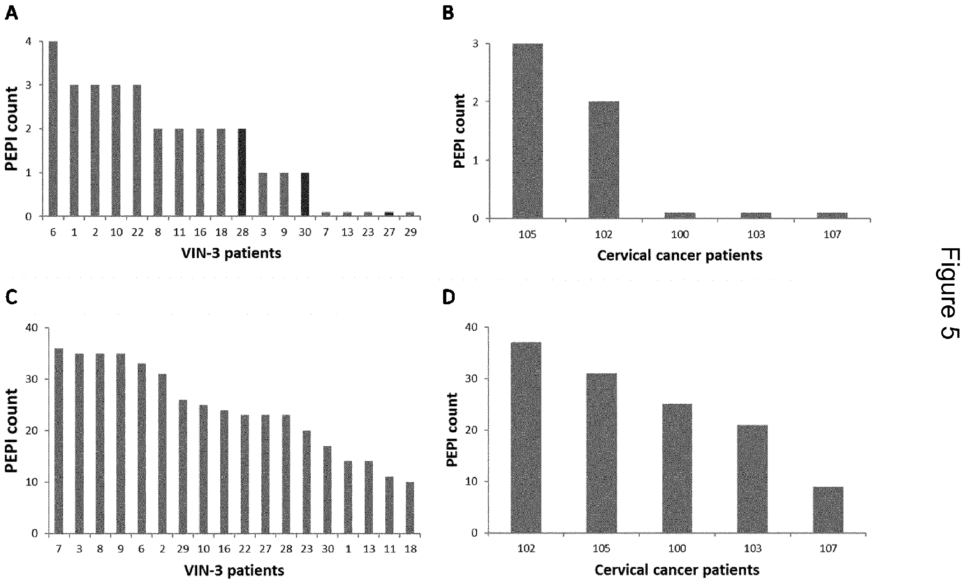

Using the same reported studies as Examples 4 and 5, we used the ≥1 PEPI3+ Test to predict patient CD8+ and CD4+ T cell responses to the full length E6 and E7 polypeptide antigens of the LPV vaccine and compared our predictions to the experimentally determined responses were reported. The Test correctly predicted the CD8+ T cell reactivity (PEPI3+) of 11 out of 15 VIN-3 patients with positive CD8+ T cell reactivity test results (sensitivity 73%, PPV 85%) and of 2 out of 5 cervical cancer patients (sensitivity 40%, PPV 100%). The CD4+ T cell reactivities (PEPI4+) were correctly predicted 100% both of VIN-3 and cervical cancer patients (Figure 5).

We also observed that the class I and class II HLA restricted PEPI3+ count correlated with the reported clinical benefit to LPV vaccinated patients. Patients with higher PEPI3+ counts had either complete or partial response already after 3 months. However the study was too small to establish the responder threshold.

### Example 7 - Case Study

pGX3001 is an HPV16 based DNA vaccine containing full length E6 and E7 antigens with a linker in between. pGX3002 is an HPV18 based DNA vaccine containing full length E6 and E7 antigens with a linker in between. A Phase II clinical trial investigated the T cell responses of 17 HPV-infected patients with cervical cancer who were vaccinated with both pGX3001 and pGX3002 (VGX-3100 vaccination)¹.

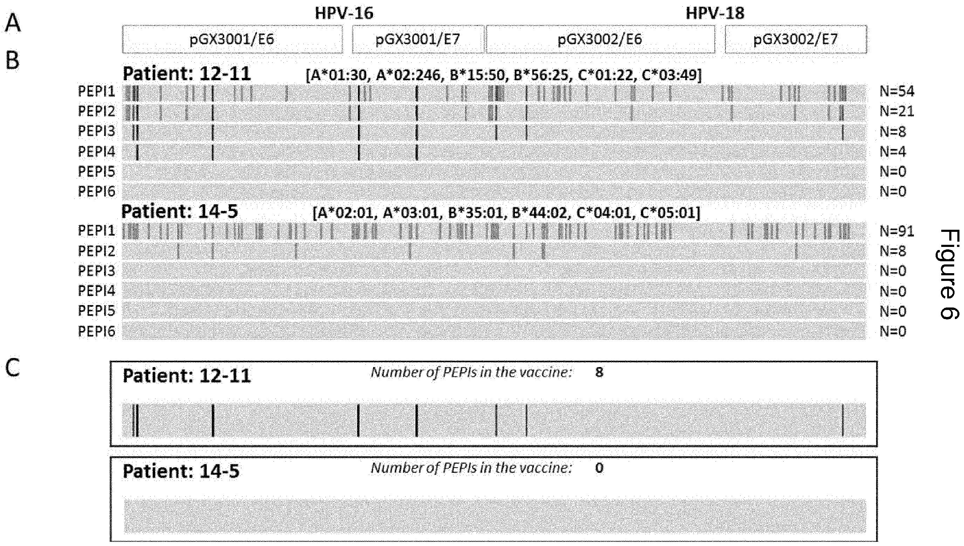

Fig. 5-6 shows for two illustrative patients (patient 12-11 and patient 14-5) the position of each epitope (9mer) presented by at least 1 (PEPI1+), at least 2 (PEPI2+), at least 3 (PEPI3+), at least 4 (PEPI4+), at least 5 (PEPI5+), or all 6 (PEPI6) class I HLA of these patients within the full length sequence of the two HPV-16 and two HPV-18 antigens.

Patient 12-11 had an overall PEPI1+ count of 54 for the combined vaccines (54 epitopes presented by one or more class I HLA). Patient 14-5 had a PEPI1+ count of 91. Therefore patient 14-5 has a higher PEPI1+ count than patient 12-11 with respect to the four HPV antigens. The PEPI1+s represent the distinct vaccine antigen specific HLA restricted epitope sets of patients 12-11 and 14-5. Only 27 PEPI1+s were common between these two patients.

For the PEPI3+ counts (number of epitopes presented by three or more patient class I HLA), the results for patients 12-11 and 14-5 were reversed. Patient 12-11 had a PEPI3+ count of 8, including at least one PEPI3+ in each of the four HPV16/18 antigens. Patient 14-5 had a PEPI3+ count of 0.

The reported immune responses of these two patients matched the PEPI3+ counts, not the PEPI1+ counts. Patient 12-11 developed immune responses to each of the four antigens post-vaccination as measured by ELISpot, whilst patient 14-5 did not develop immune responses to any of the four antigens of the vaccines. A similar pattern was observed when we compared the PEPI1+ and PEPI3+ sets of all 17 patients in the trial. There was no correlation between the PEPI1+ count and the experimentally determined T cell responses reported from the clinical trial. However, we found good agreements between the T cell immunity predicted by the ≥1 PEPI3+ Test and the reported T cell immunity. The ≥1 PEPI3+ Test predicted the immune responders to HPV DNA vaccine.

Moreover, the diversity of the patient's PEPI3+ set resembled the diversity of T cell responses generally found in cancer vaccine trials. Patients 12-3 and 12-6, similar to patient 14-5, did not have PEPI3+s predicting that the HPV vaccine could not trigger T cell immunity. All other patients had at least one PEPI3 predicting the likelihood that the HPV vaccine can trigger T cell immunity. 11 patients had multiple PEPI3+ predicting that the HPV vaccine likely triggers polyclonal T cell responses. Patients 15-2 and 15-3 could mount high magnitude T cell immunity to E6 of both HPV, but poor immunity to E7. Other patients 15-1 and 12-11 had the same magnitude response to E7 of HPV 18 and HPV 16, respectively.

### Example 8 - Design of a Model Population for conducting in silico trials and identifying candidate precision vaccine targets for large population

We compiled an *in silico* human trial cohort of 433 subjects with complete 4-digit HLA class I genotype (2 x HLA-A*xx:xx; 2 x HLA-B*xx:xx; 2 x HLA-C*xx:xx) and demographic information. This Model Population has subjects with mixed ethnicity having a total of 152 different HLA alleles that are representative for >85% of presently known allele G-groups.

We also established a database of a "Big Population" containing 7,189 subjects characterized with 4-digit HLA genotype and demographic information¹¹. The Big Population has 328 different HLA class I alleles. The HLA allele distribution of the Model Population significantly correlated with the Big Population (Table 13) (Pearson p<.001). Therefore the 433 patient Model Population is representative for a 16 times larger population.

We concluded that the Model Population is representative for 85% of the human race as given by HLA diversity as well as HLA frequency.

**Table 13. Statistical analysis of HLA distributions in "Model Population" vs. "Big Population".**

| **Group name 1** | **Group name 2** | **Pearson R value** | **Correlation** | **P Value** |
|---|---|---|---|---|
| 433 Model Population | 7,189 Big Population | 0.89 | Strong | P<0.001 |

### Example 9 -In silico trials based on the identification of multiple HLA binding epitopes predict the reported T cell response rates of clinical trials

The objective of this study was to determine whether a model population, such as the one described in Example 8, may be used to predict CTL reactivity rates of vaccines, i.e. used in an *in silico* efficacy trials.

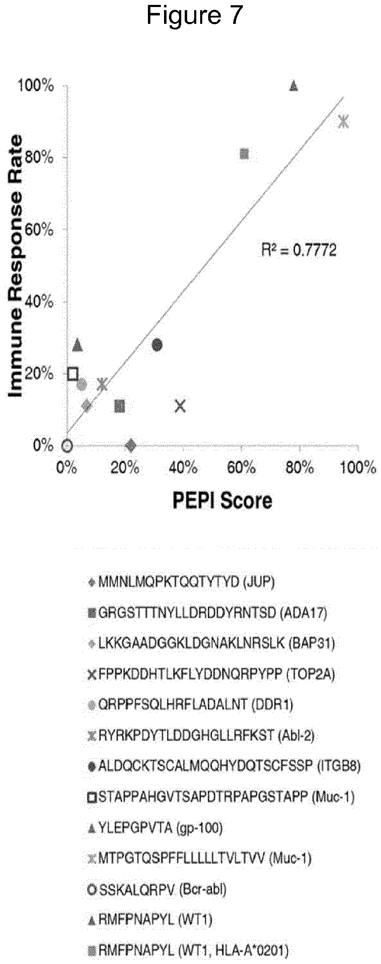

We identified from peer reviewed publications 12 peptide vaccines derived from cancer antigens that induced T cell responses in a subpopulation of subjects (Table 14). These peptides have been investigated in clinical trials enrolling a total of 172 patients (4 ethnicities). T cell responses induced by the vaccine peptides have been determined from blood specimens and reported. We calculated the immune response rate as the percentage of study subjects with positive T cell responses measured in the clinical trials (FIG. 7).

**Table 14. Clinical trials conducted with peptide vaccines.**

| *Peptide vaccines* | *Source antigen* | *Peptide length* | *T cell assay* | *Pop. (n)* | *Ethnicity* | *Ref.* |
|---|---|---|---|---|---|---|
| MMNLMQPKTQQTYTYD | JUP | 16mer | Multimer staining | 18 | Canadian | ¹² |
| GRGSTTTNYLLDRDDYRNTSD | ADA17 | 21mer | | | | |
| LKKGAADGGKLDGNAKLNRSLK | BAP31 | 22mer | | | | |
| FPPKDDHTLKFLYDDNQRPYPP | TOP2A | 22mer | | | | |
| RYRKPDYTLDDGHGLLRFKST | Abl-2 | 21mer | | | | |
| QRPPFSQLHRFLADALNT | DDR1 | 18mer | | | | |
| ALDQCKTSCALMQQHYDQTSCFSSIPITGB8 | | 25mer | | | | |
| STAPPAHGVTSAPDTRPAPGSTAPP | MUC-1 | 25mer | Proliferation | 80 | Canadian | ¹³ |
| YLEPGPVTA | gp100 | 9mer | Tetramer | 18 | US | ¹⁴ |
| MTPGTQSPFFLLLLLTVLTW | MUC-1 | 2-1 mer 21mer | Cytotoxicity | 10 | Israeli | ¹⁵ |
| SSKALQRPV | Bcr-Abl | 9mer | ELISPOT | 4 | US | ¹⁶ |
| RMFPNAPYL | WT-1 | 9mer | Multimer staining | 24 | US | ¹⁷ |
| RMFPNAPYL (HLA-A*0201) | WT-1 | 9mer | Cytokine staining | 18 | CEU | ¹⁸ |

The 12 peptides were investigated with the ≥1 PEPI3+ Test in each of the 433 subjects of the Model Population described in Example 8. The "≥1 PEPI3+ Score" for each peptide was calculated as the proportion of subjects in the Model Population having at least one vaccine derived epitope that could bind to at least three subject-specific HLA class I (≥1 PEPI3+). If the corresponding clinical trial stratified patients for HLA allele selected population, the Model Population was also filtered for subjects with the respective allele(s) (Example: WT1, HLA-A*0201).

The experimentally determined response rates reported from the trials were compared with the ≥1 PEPI3+ Scores. The Overall Percentage of Agreements (OPA) were calculated on the paired data (Table 15). We also found a linear correlation between ≥1 PEPI3+ Score and response rate (R² = 0.77) (FIG. 7). This result shows that the identification of peptides predicted to bind to multiple HLAs of an individual is useful to predict *in silico* the outcome of clinical trials.

**Table 15. Comparison of ≥1 PEPI3+ Scores and CTL response rates of 12 peptide vaccines.**

| ***Peptide vaccine*** | ***Source antigen*** | ***Response rate (Clinical Trials)*** | **≥*1 PEPI3*+ *Score** *(Model Population)*** | ***OPA*** |
|---|---|---|---|---|
| MMNLMQPKTQQTYTYD | JUP | 0% | 22% | NA |
| GRGSTTTNYLLDRDDYRNTSD | ADA17 | 11% | 18% | 61% |
| LKKGAADGGKLDGNAKLNRSLK | BAP31 | 11% | 7% | 64% |
| FPPKDDHTLKFLYDDNQRPYPP | TOP2A | 11% | 39% | 28% |
| RYRKPDYTLDDGHGLLRFKST | Abl-2 | 17% | 12% | 71% |
| QRPPFSQLHRFLADALNT | DDR1 | 17% | 5% | 29% |
| ALDQCKTSCALMQQHYDQTSCFSSP | ITGB8 | 28% | 31% | 90% |
| STAPPAHGVTSAPDTRPAPGSTAPP | MUC-1 | 20% | 2% | 10% |
| YLEPGPVTA | gp100 | 28% | 4% | 14% |
| MTPGTQSPFFLLLLLTVLTVV | MUC-1 | 90% | 95% | 95% |
| SSKALQRPV | Bcr-Abl | 0% | 0% | 100% |
| RMFPNAPYL | WT-1 | 100% | 78% | 78% |
| RMFPNAPYL (HLA-A*0201) | WT-1 | 81% | 61% | 75% |

| | | | | |
|---|---|---|---|---|
| * % *subjects in the Model Population with ≧1 vaccine derived PEPI3* + | | | | |

### Example 10. In silico trials based on the identification of multiple HLA binding epitopes predict the reported T cell response rates of clinical trials II

We identified a further 19 clinical trials with published immune response rates (IRR) conducted with peptide or DNA based vaccines (Table 16). These trials involved 604 patients (9 ethnicities) and covered 38 vaccines derived from tumor and viral antigens. Vaccine antigen specific CTL responses were measured in each study patient and the response rate in the clinical study populations was calculated and reported.

| Table 16. Response rates published in clinical trials. | | | | | |
|---|---|---|---|---|---|
| **Immunotherapy** | **Type** | **CTL assay** | **Pop. (n)** | **Race/ Ethnicity** | **Ref.** |
| StimuVax | peptide | Proliferation | 80 | Canadian | 13 |
| gp100 vaccine | DNA | Tetramer | 18 | US | 14 |
| IMA901 phase I | peptide | ELISPOT | 64 | CEU | 19 |
| IMA901 phase II | peptide | Multimer staining | 27 | CEU | |
| ICT107 | peptide | ICC | 15 | US | 20 |
| ProstVac | DNA | ELISPOT | 32 | CEU87%, Afr. Am. 12%, Hisp. 1% | 21 |
| Synchrotope TA2M | DNA | Tetramer | 26 | US | 22 |
| MELITAC 12.1 | peptide | ELISPOT | 167 | US | 23 |
| WT1 vaccine | peptide | Tetramer | 22 | Japanese | 24 |
| Ipilimumab (NY-ESO-1) | checkpoi nt inhibitor* * | ICC | 19 | US | 5 |
| VGX-3100 | DNA | ELISPOT | 17 | US | 1 |
| HIVIS-1 | DNA | ELISPOT | 12 | CEU98%, Asian 1%, Hisp. 1% | 2 |
| ImMucin | peptide | Cytotoxicity | 10 | Israeli | 15 |
| NY-ESO-1 OLP | peptide | IFN-gamma | 7 | Japanese | 7 |
| GVX301 | peptide | Proliferation | 14 | CEU | 25 |
| WT1 vaccine | peptide | ELISPOT | 12 | US | 26 |
| WT1 vaccine | peptide | ICC | 18 | CEU | 18 |
| DPX-0907* | peptide | Multimer staining | 18 | Canadian | 12 |
| Melanoma peptide vaccine | peptide | ELISPOT | 26 | White | 27 |

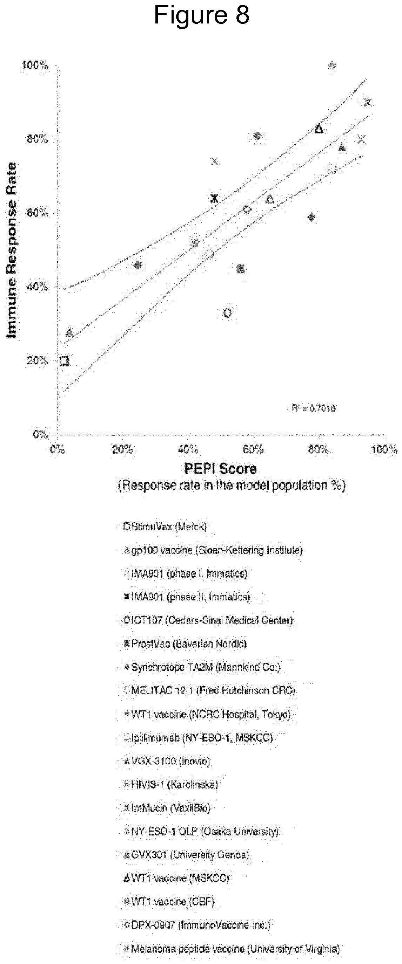

Each vaccine peptide of the 19 clinical trials was investigated with the ≥1 PEPI3+ Test in each subject of the Model Population. The ≥1 PEPI3+ Score for each peptide was calculated as the proportion of subjects in the Model Population having at least one vaccine derived PEPI3+. The experimentally determined response rates reported from the trials were compared with the PEPI Scores, as in Example 9 (Table 17). We found a linear correlation between the response rate and ≥1 PEPI3+ Score (R² = 0.70) (FIG. 8). This result confirms that the identification of peptides predicted to bind to multiple HLAs of an individual can predict T cell responses of subjects, and *in silico* trials can predict the outcome of clinical trials.

| **Immunotherapy** | **Clinical Trial Response Rate** | **≥1 PEPI3+ Score*** | **OPA** |
|---|---|---|---|
| StimuVax (failed to show efficacy in Phase III) | 20% | 2% | 10% |
| | | | |

| Table 17. Linear correlation between PEPI Score and response rate (R² = 0.7). | | | |
|---|---|---|---|
| gp100 vaccine | 28% | 4% | 14% |
| IMA901 phase I | 74% | 48% | 65% |
| IMA901 phase II | 64% | 48% | 75% |
| ICT107 | 33% | 52% | 63% |
| ProstVac | 45% | 56% | 80% |
| Synchrotope TA2M | 46% | 24% | 52% |
| MELITAC 12.1 | 49% | 47% | 96% |
| WT1 vaccine | 59% | 78% | 76% |
| Ipilimumab (NY-ESO-1*) | 72% | 84% | 86% |
| VGX-3100 | 78% | 87% | 90% |
| HIVIS-1 | 80% | 93% | 86% |
| ImMucin | 90% | 95% | 95% |
| NY-ESO-1 OLP | 100% | 84% | 84% |
| GVX301 | 64% | 65% | 98% |
| WT1 vaccine | 83% | 80% | 96% |
| WT1 vaccine | 81% | 61% | 75% |
| DPX-0907 | 61% | 58% | 95% |
| Melanoma peptide vaccine | 52% | 42% | 81% |

| | | | |
|---|---|---|---|
| * *% subjects in the Model Population with ≧1 vaccine derived PEPI3*+ | | | |

### Example 11 - In silico trial based on the identification of multiple HLA binding epitopes in a multi-peptide vaccine predict the reported clinical trial immune response rate

IMA901 is a therapeutic vaccine for renal cell cancer (RCC) comprising 9 peptides derived from tumor-associated peptides (TUMAPs) that are naturally presented in human cancer tissue ¹⁹. A total of 96 HLA-A*02+ subjects with advanced RCC were treated with IMA901 in two independent clinical studies (phase I and phase II)¹⁹. Each of the 9 peptides of IMA901 have been identified in the prior art as HLA-A2-restricted epitopes. Based on currently accepted standards, they are all strong candidate peptides to boost T cell responses against renal cancer in the trial subjects, because their presence has been detected in renal cancer patients, and because the trial patients were specifically selected to have at least one HLA molecule capable of presenting each of the peptides.

We determined for each subject in the Model population how many of the nine peptides of the IMA901 vaccine were capable of binding to three or more HLA. Since each peptide in the IMA901 vaccine is a 9mer this corresponds to the PEPI3+ count. The results were compared with the immune response rates reported for the Phase I and Phase II clinical trials (Table 18).

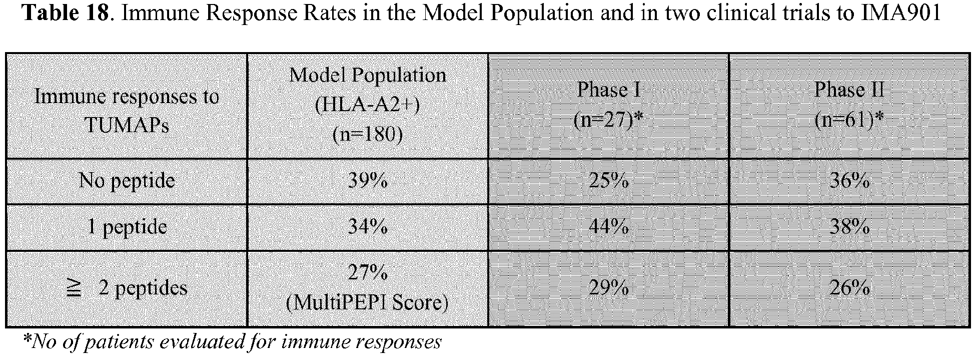

The phase I and phase II study results show the variability of the immune responses to the same vaccine in different trial cohorts. Overall, however, there was a good agreement between response rates predicted by the ≥2 PEPI3+ Test and the reported clinical response rates.

In a retrospective analysis, the clinical investigators of the trials discussed above found that subjects who responded to multiple peptides of the IMA901 vaccine were significantly (*p* = 0.019) more likely to experience disease control (stable disease, partial response) than subjects who responded only to one peptide or had no response. 6 of 8 subjects (75%) who responded to multiple peptides experienced clinical benefit in the trial, in contrast to 14% and 33% of 0 and 1 peptide responders, respectively. The randomized phase II trial confirmed that immune responses to multiple TUMAPs were associated with a longer overall survival.

The reported disease control rate (DCR) of the phase I and phase II clinical trials were 43% and 22% respectively. The tumors of the vaccinated patients were reasonably assumed to present the target TUMAPs. The reported disease control rates approximate to the percentage of subjects in our Model Population predicted by the ≥1 PEPI3+ Test to mount an immune response to at least two of the nine IMA901 9mer peptides (27%), also suggesting that this is the threshold for identifying likely clinical responders to the IMA901 vaccine. The Disease Control Rate of the clinical trials was predicted with 75% probability by the identification of ≥1 PEPI3+ in at least two of the 9mer peptide antigens of the IMA901 vaccine (corresponds for this vaccine of nine 9mer peptides to ≥2 PEPI3+ in total). In contrast, a single PEPI3+ in the 9 peptide vaccine was not predictive of clinical benefit.

### Example 12 - In silico trial based on the identification of vaccine-derived multiple HLA binding epitopes predict reported experimental clinical response rates

We demonstrate here a correlation between the ≥2 PEPI3+ Score of immunotherapy vaccines determined in our Model Population and the reported Disease Control Rate (DCR, proportion of patients with complete responses and partial responses and stable disease) determined in clinical trials.

In peer reviewed scientific journals we found 17 clinical trials, conducted with peptide- and DNA-based cancer immunotherapy vaccines that have published Disease Control Rates (DCRs) or objective response rate (ORR) (Table 19). These trials involved 594 patients (5 ethnicities) and covered 29 tumor and viral antigens. DCRs were determined according to the Response Evaluation Criteria in Solid Tumors (RECIST), which is the current standard for clinical trials, in which clinical responses are based on changes in maximum cross-sectional dimensions^{42,43,44.} In case there was no available DCR data, objective response rate (ORR) data was used, which is also defined according to the RECIST guidelines.

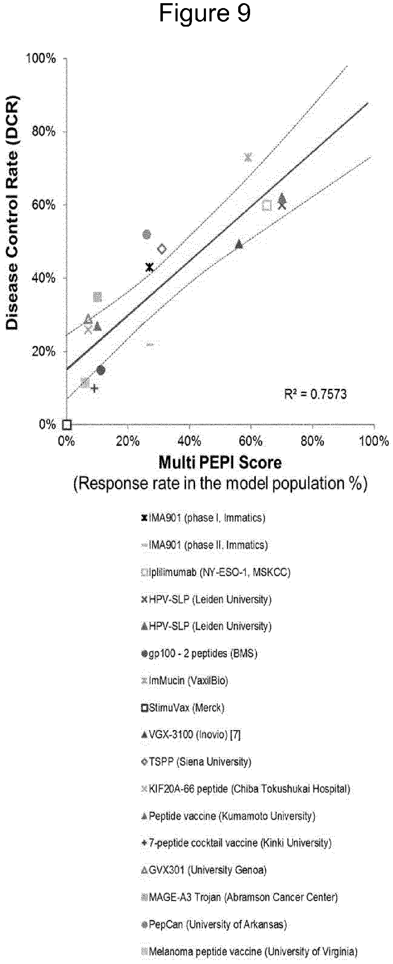

Table 20 compares the ≥2 PEPI3+ Score for each vaccine in the Model Population and the published DCR or ORR. We found a remarkable correlation between the predicted and measured DCR providing further evidence that not only the immunogenicity but also the potency of cancer vaccines depends on the multiple HLA sequences of individuals (R2 = 0.76) (FIG. 9).

**Table 19.Clinical trials selected for Disease Control Rate (DCR) prediction.**

| **Immuno-therapy** | **Antigen** | **Sponsor** | **Disease** | **Pop. (n)** | **Study pop./ Ethnicity** | **HLA restriction** | **Adm form** | **Dose (mg)** | **Dosing schedule** | **Assessment time (weeks)** | **Ref.** |
|---|---|---|---|---|---|---|---|---|---|---|---|
| **IMA901 phase I** | 9 TAAs | Immatics | Renal cell cancer | 28 | CEU | A02 | i.d. | 0.4 | 8x in 10 wks | 12 | 19 |
| **IMA901 phase II** | 9 TAAs | Immatics | Renal cell cancer | 68 | CEU | A02 | i.d | 0.4 | 7x in 5 wks then 10x 3 wks | 24 | 19 |

| | | | | | | | | | | | |
|---|---|---|---|---|---|---|---|---|---|---|---|
| **Ipilimumab** | NY-ESO-1 | MSKCC | Melanoma | 19 | US | no | i.v. | 0.3 | 4 x every 3 wks | 24 | 5 |
| | | | | | | | | 3 | | | |
| | | | | | | | | 10 | | | |
| **HPV-SLP*** | HPV-16 E6, E7 | Leaden University | VIN | 20 | CEU | no | s.c. | 0.3 | 3 x every 3 wks | 12 | 9 |
| **HPV-SLP*** | | Leaden University | HPV-related cervical cancer | 5 | CEU | no | s.c. | 0.3 | 3 x every 3 wks | 12 (OR) | 10 |
| **gp100 - 2 peptides*** | gp100 | BMS | Melanoma | 136 | US | A*0201 | s.c. | 1 | 4 x every 3 wks | 12 | ²8 |
| **Immucin** | Muc-1 | VaxilBio | Myeloma | 15 | Israeli | no | s.c. | 0.1 | 6 x every 2 wks | 12** | 29 |
| **StimuVax** | Muc-1 | Merck | NSCLC | 80 | Canadian | no | s.c. | 1 | 8x wkly then every 6 wks | 12 | 13, 30 |
| **VGX-3100** | HPV-16&18 | Inovio | HPV-related cervical cancer | 125 | US | no | i.m. | 6 | 0, 4, 12 wks | 36 | ³1 |
| **TSPP peptide vaccine** | Thymidylate synthase | Siena University | CRC, NSCLC, Gallbladder carc., Breast-, Gastric cancer | 21 | CEU | no | s.c. | 0.1 | 3 x 3 wks | 12 | 32 |
| | | | | | | | | 0.2 | | | |
| | | | | | | | | 0.3 | | | |
| **KIF20A-66 peptide vaccine*** | KIF20A | Chiba Tokushukai Hospital | Metastatic pancreatic cancer | 29 | Japanese | A*2402 | s.c. | 1 3 | 2 cycles 1, 8, 15, 22 days | 12 (OR) | 33 |
| | | | | | | | | | then every 2 wks | | |
| **Peptide vaccine*** | 3 TAAs | Kumamoto University | HNSCC | 37 | Japanese | A*2402 | s.c. | 1 | 8 x wkly then every 4 wks | 12 | ³4 |
| **7-peptide cocktail vaccine*** | 7 TAAs | Kinki University | Metastatic colorectal cancer | 30 | Japanese | A*2402 | s.c. | 1 | Cycles: 5 x wkly then 1 wk rest | 10 (OR) | ³5 |
| **GVX301*** | hTERT | University Genoa | Prostate and renal cancer | 14 | Japanese | A02 | i.d. | 0.5 | 1, 3, 5, 7, 14, 21, 35, 63 days | 12 | 25 |
| **MAGE-A3 Trojan*** | MAGE-A3 | Abramson Cancer Center | Multiple myeloma | 26 | US | no | s.c. | 0.3 | 14, 42, 90, 120, 150 days | 24 | ³6 |
| **PepCan** | HPV-16 E6 | University of Arkansas | CIN2/3 | 23 | US | no | i.m. | 0.05 | 4x3wks | 24 | ³7 |
| | | | | | | | | 0.1 | | | |
| | | | | | | | | 0.25 | | | |
| | | | | | | | | 0.5 | | | |
| **Melanoma peptide vaccine*** | Tyrosinase, gp100 | University of Virginia | Melanoma | 26 | US | A1, A2 or A3 | s.c. | 0.1 | 6 cycles: 0, 7, 14, 28, 35, 42 days | 6 | 27 |

| | | | | | | | | | | | |
|---|---|---|---|---|---|---|---|---|---|---|---|
| *Montanide ISA51 VG as adjuvant **Disease response was assessed according to the International Myeloma Working Group response criteria⁴⁵ | | | | | | | | | | | |

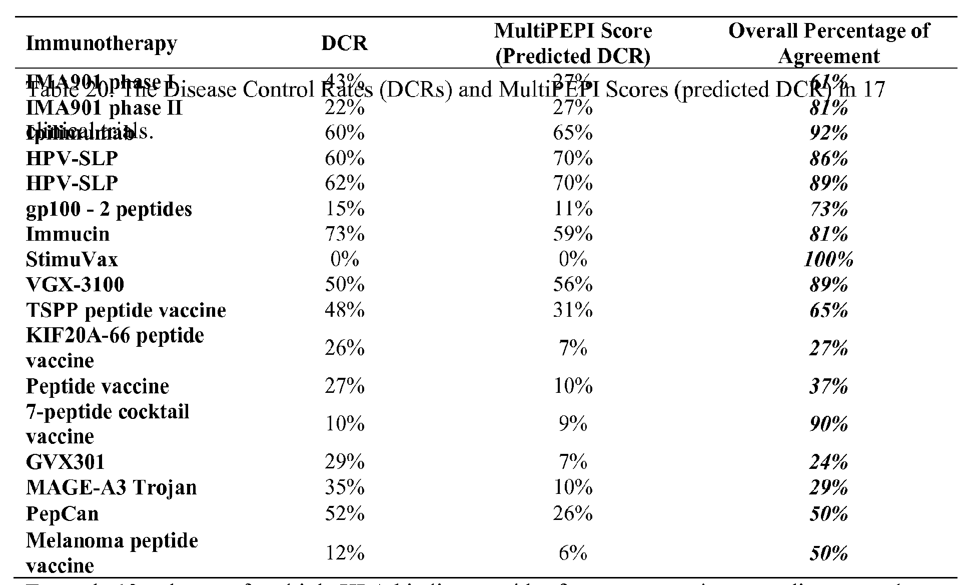

### Example 13 - the set of multiple HLA binding peptides from tumor antigens predict responders to the checkpoint inhibitor immunotherapy Ipilimumab

We found that survival benefit of melanoma patients treated with the checkpoint inhibitor Ipilimumab can be predicted by the number of melanoma-specific PEPI3+s that are potentially expressed in the patient's tumor.

We identified 80 melanoma associated antigens (TAAs) and selected a panel of PEPI3+s (IPI-PEPI panel: 627 PEPIs) that are shared by Ipilimumab treated melanoma patients with a prolonged clinical benefit and are absent in those without a prolonged benefit. These PEPI3+ define the specific T cells that are re-activated by Ipilimumab to attack the patient's tumor cells. Patients with certain HLA sequences that can present more melanoma-specific PEPIs have more T cells re-activated by Ipilimumab and a higher chance to benefit from Ipilimumab immunotherapy.

We predicted the clinical benefit from Ipilimumab treatment for 160 patients from four independent clinical trial cohorts. Two cohorts were from the trials CA184-007 (10 mg/kg Ipilimumab) and CA184-002 (3 mg/kg Ipilimumab) and two cohorts from published clinical trials 10 mg/kg and 3/mg/kg Ipilimumab datasets^{5, 38, 39}.

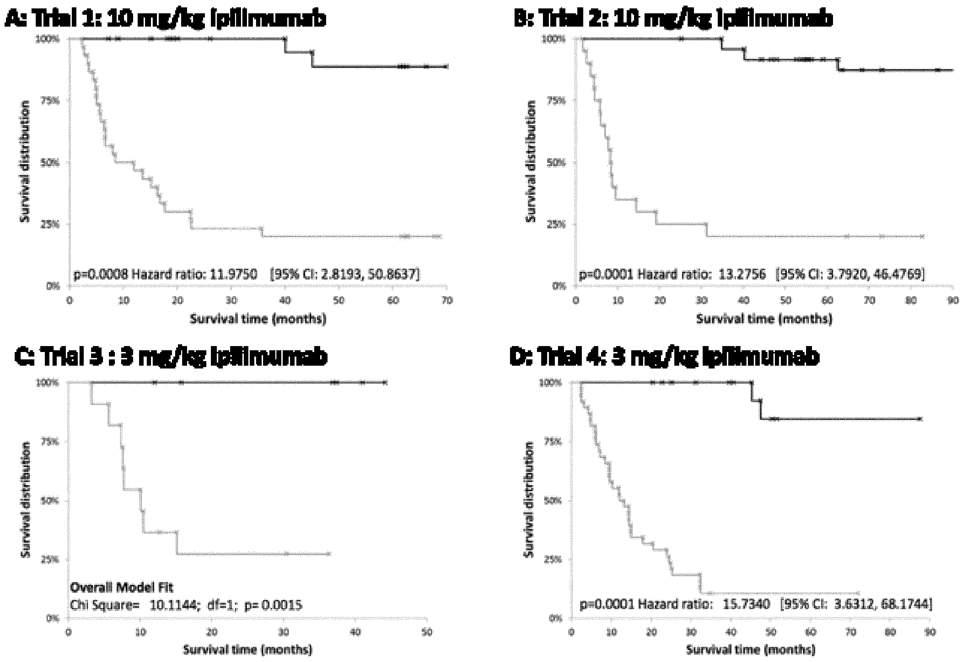

First, we predicted epitopes from 80 melanoma antigens restricted to all the 6 HLA class I of each patient. Then, we computed the number of melanoma-specific PEPI3+s restricted to at least 3 class I HLAs of each patient (4,668 PEPIs). Each patient with at least one out of 627 PEPIs qualified as responder. The IPI-PEPI panel predicts the overall survival of both 10 mg/kg and 3 mg/kg Ipilimumab. Results were highly significant and consistent in the four independent cohort (Fig. 10).

### Example 14: Multiple HLA binding epitopes define patient mutational neoantigens

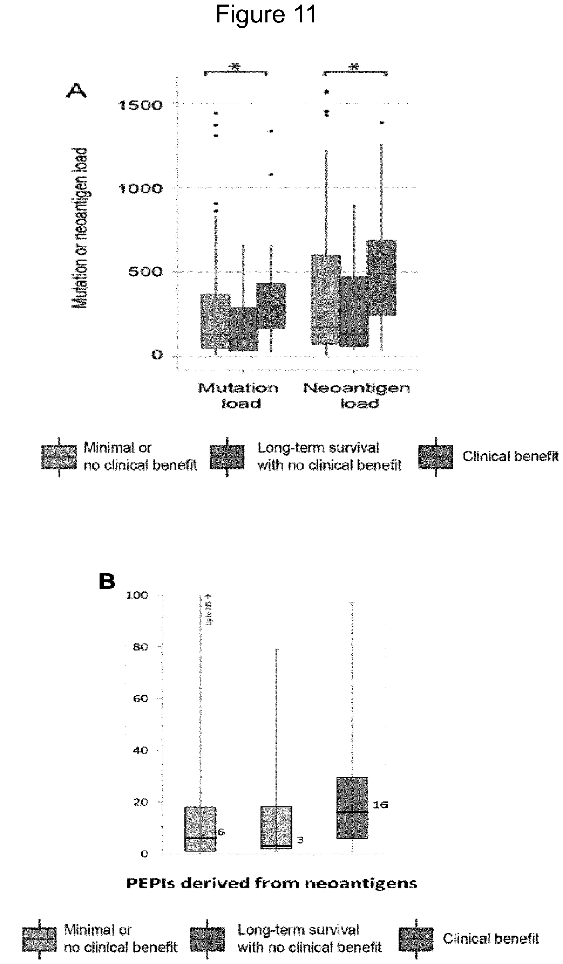

To show the capability of the PEPI3+ to identify neoantigens from mutations we predicted PEPI3+s of 110 melanoma patients treated with Ipilimumab using published exome mutation data³⁹. The authors of the publication found mutations in 9,502 antigens from the 110 patients (Fig. 11A). Median nonsynonymous mutational load per sample was highly variable, 309 (29-4,738) in the clinical benefit cohort and 147 (7-5,854) in the minimal or no clinical benefit cohort. Due to their epitope prediction results these mutations had 211 (8-1950) and 56 (2-3444) neoepitopes in the clinical benefit cohort and the minimal or no clinical benefit cohorts, respectively.

We predicted mutational PEPI3+ neoepitopes from the published mutations (Fig. 11B and Table 21). In our prediction these mutations result in median 16 PEPIs and 6 PEPIs neoepitopes in clinical benefit cohort and the minimal or no clinical benefit cohorts, respectively.

Our results suggest that PEPIs can define the mutational neoantigens derived from genetically altered proteins expressed in an individual. Such neoantigens are PEPI3+ peptides that capable to activate T cells in the patient's body. If a genetic alteration occurs in the tumor cell of the individual that creates a PEPI3+ then this PEPI3+ can induce T cell responses. These PEPI3+ containing peptides could be included in a drug (e.g. vaccine, T cell therapy) to induce immune response against the individual tumor.

Table 21. Mutational neoantigen prediction using PEPI Test: Analysis results of Van Allen et al. and PEPI Test on 110 melanoma patients.

| Parameters | **Results published by Van Allen et al.** | | **Result obtained from PEPI Test analyses** | |
|---|---|---|---|---|
| Patients | Clinical benefit (n=27) | Minimal or no clinical Benefit (n=73) | Clinical benefit (n=27) | Minimal or no clinical benefit (n=73) |
| Median mutations | 555 | 281 | - | - |
| **Median nonsynonymous mutations** | 309 | 147 | - | - |
| **Median expressed mutational antigens** | 198 | | - | - |
| **Median neoepitope (only 9mer)** | 211 | 56 | 130 | 50 |
| **Recurrent neoepitopes** | 28 | Not provided | 10 | 76 |
| **Median PEPI neoepitopes** | - | - | 16 | 6 |
| **Recurrent PEPI neoepitopes** | - | - | 1 | 5 |

### Example 15. Multiple HLA binding peptides of individuals can predict immune-toxicity

Thrombopoietin (TPO) is a highly immunogenic protein drug causing toxicity in many patients. EpiVax/Genentech used State of Art technology to identify class II HLA restricted epitopes and found that the most immunogenic region of the TPO is located in the C-terminal end of TPO (US20040209324 A1).

According to the present invention we defined the multiple class II HLA binding epitopes (PEPI3+s) from TPO in 400 HLA class II genotyped US subjects. Most of the PEPI3+ peptides of these individuals located within the N terminal region of the TPO between 1-165 amino acids. We sporadically found PEPI3+ in some subjects also in the C terminal region. However, our results were different from the State of Art.

The published literature confirmed our results, demonstrating experimental proofs for the immunotoxic region is located at the N-terminal end of TPO^{40,41}. Most individuals treated with TPO drug made anti-drug antibodies (ADA) ADA against this region of the drug. These antibodies are not only abolished the therapeutic effect of the drug but also caused systemic adverse events, i.e. immune-toxicity, like antibody -dependent cytotoxicity (ADCC) and complement-dependent cytotoxicity associated with thrombocytopenia, neutropenia and anemia. These data demonstrate that the identification of multiple HLA binding peptides of individuals can predict the immune-toxicity of TPO. Therefore, the invention is useful to identify the toxic immunogenic region of drugs and also identify subjects who likely experience immune-toxicity from drugs.

### Example 16 - Case Study - Design of Personalised Immunotherapy Composition for Treatment of Ovarian Cancer

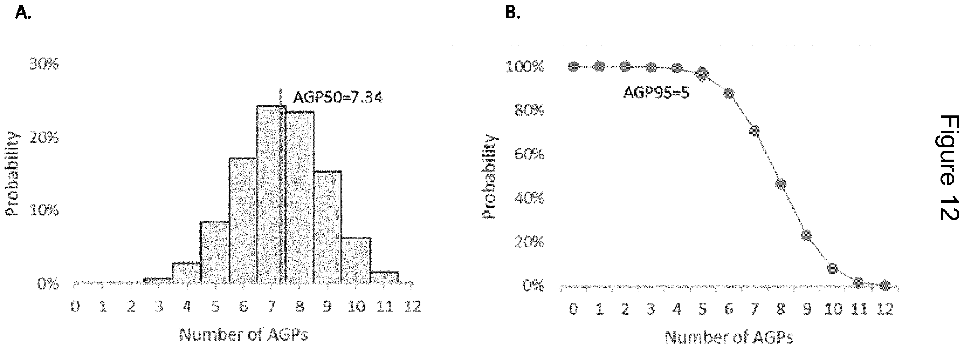

The HLA genotype of ovarian cancer patient XYZ was determined from a saliva sample. To make a personalized pharmaceutical composition for XYZ patient twelve peptides were selected, each of which met the following two criteria: (i) derived from an antigen that is expressed in ovarian cancers, as reported in peer reviewed scientific publications; and (ii) comprises a fragment that is a T cell epitope capable of binding to three HLA class I of patient XYZ (Table 22). In addition, each peptide is optimized to bind the maximum number of HLA class II of the patient. Each peptide in this immunotherapy composition can induce immune responses in XYZ with 84% probability and the immune responses target 12 antigens expressed in ovarian cancer. Expression of these cancer antigens in patient XYZ is not tested. Instead the probability of successful killing of cancer cells can be determined based on the probability of antigen expression in the patient's cancer cells and the positive predictive value of the ≥1 PEPI3+ Test. The probability that patient XYZ will express one or more of the 12 antigens is shown in Fig. 12. Based on clinical validation of the ≥1 PEPI3+ Test (Example 3), there is an 84% probability that each peptide in the composition will induce immune responses in patient XYZ that can kill cancer cells, if the antigen is expressed.

| **Table 22. 12 peptides for XYZ ovarian cancer patient** | | |
|---|---|---|
| **(Antigen) & Peptide sequences** | | **Antigen Expression Rate in ovarian cancers** |
| P1 | (AKAP4) NSLQKQLQAVLQWIAASQFN | 89% |
| P2 | (BORIS) SGDERSDEIVLTVSNSNVEE | 82% |
| P3 | (SPAG9) VQKEDGRVQAFGWSLPQKYK | 76% |
| P4 | (OY-TES-1) EVESTPMIMENIQELIRSAQ | 75% |
| P5 | (SP17) AYFESLLEKREKTNFDPAEW | 69% |
| P6 | (WT1) PSQASSGQARMFPNAPYLPS | 63% |
| P7 | (HIWI) RRSIAGFVASINEGMTRWFS | 63% |
| P8 | (PRAME) MQDIKMILKMVQLDSIEDLE | 60% |
| P9 | (AKAP-3) ANSVVSDMMVSIMKTLKIQV | 58% |
| P10 | (MAGE-A4) REALSNKVDELAHFLLRKYR | 37% |
| P11 | (MAGE-A9) ETSYEKVINYLVMLNAREPI | 37% |
| P12 | (MAGE-A3) FSKASSSLQLVFGIELMEVD | 32% |

| | | |
|---|---|---|
| P1 also recognizes AKAP3 (58%). | | |

The pharmaceutical composition for patient XYZ is comprised of at least 2 from the 12 peptides (Table 22), because we have discovered that the presence in a vaccine or immunotherapy composition of at least two polypeptide fragments (epitopes) that can bind to at least three HLA of an individual (≥2 PEPI3+) is predictive for a clinical response. The peptides are synthetized, solved in a pharmaceutically acceptable solvent and mixed with an adjuvant prior to injection. The patient receive personalized immunotherapy with at least two peptide vaccines, but preferable more to increase the probability of killing cancer cells and decrease the chance of relapse.

### Example 17 - Case Study - Design of Personalised Immunotherapy Composition for Treatment of Breast Cancer

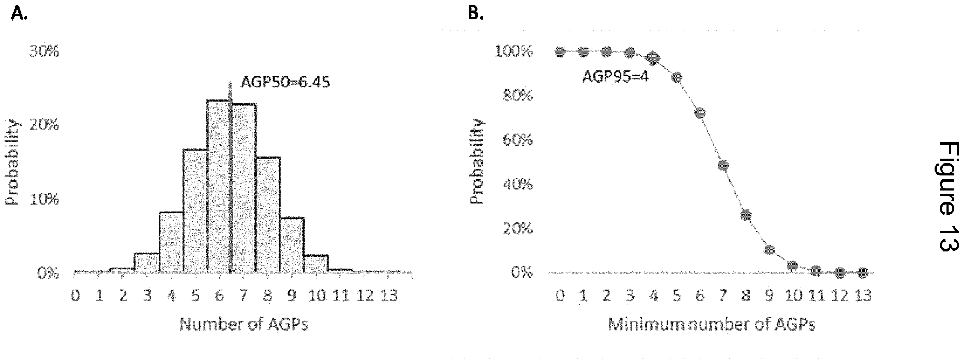

The HLA genotype of ovarian cancer patient ABC was determined from a saliva sample. To make a personalized pharmaceutical composition for ABC patient twelve peptides were selected, each of which met the following two criteria: (i) derived from an antigen that is expressed in breast cancers, as reported in peer reviewed scientific publications; and (ii) comprises a fragment that is a T cell epitope capable of binding to three HLA class I of patient ABC (Table 23). In addition, each peptide is optimized to bind the maximum number of HLA class II of the patient. The twelve peptides target twelve breast cancer antigens. The calculated potential benefit to patient ABC is shown in Figure 13.

| **Table 23.12 peptides for ABC breast cancer patient** | | |
|---|---|---|
| **#** | **(Antigen) & Peptide sequences** | **Antigen Expression Rate in breast cancers** |
| P1 | (FSIP1) ISDTKDYFMSKTLGIGRLKR | 49% |
| P2 | (SPAG9) FDRNTESLFEELSSAGSGLI | 88% |
| P3 | (AKAP4 (CT99)) SQKMDMSNIVLMLIQKLLNE | 85% |
| P4 | (BORIS) SAVFHERYALlQHQKTHKNE | 71% |
| P5 | (MAGE-A11) DVKEVDPTSHSYVLVTSLNL | 59% |
| P6 | (NY-SAR-35) ENAHGQSLEEDSALEALLNF | 49% |
| P7 | (HOM-TES-85) MASFRKLTLSEKVPPNHPSR | 47% |
| P8 | (NY-BR-1) KRASQYSGQLKVLIAENTML | 47% |
| P9 | (MAGE-A9) VDPAQLEFMFQEALKLKVAE | 44% |
| P10 | (SCP-1) EYEREETRQVYMDLNNNIEK | 38% |
| P11 | (MAGE-A1) PEIFGKASESLQLVFGIDVK | 37% |
| P12 | (MAGE-C2) DSESSFTYTLDEKVAELVEF | 21% |

| | | |
|---|---|---|
| P12 also recognizes MAGE-C1 (12%). | | |

### References

¹ Bagarazzi et al. Immunotherapy against HPV16/18 generates potent TH1 and cytotoxic cellular immune responses. Science Translational Medicine. 2012; 4(155):155ra138.
² Gudmundsdotter et al. Amplified antigen-specific immune responses in HIV-1 infected individuals in a double blind DNA immunization and therapy interruption trial. Vaccine. 2011; 29(33):5558-66.
³ Bioley et al. HLA class I - associated immunodominance affects CTL responsiveness to an ESO recombinant protein tumor antigen vaccine. Clin Cancer Res. 2009; 15(1):299-306.
⁴ Valmori et al. Vaccination with NY-ESO-1 protein and CpG in Montanide induces integrated antibody/Th1 responses and CD8 T cells through cross-priming. Proceedings of the National Academy of Sciences of the United States of America. 2007; 104(21):8947-52.
⁵ Yuan et al. Integrated NY-ESO-1 antibody and CD8+ T-cell responses correlate with clinical benefit in advanced melanoma patients treated with ipilimumab.Proc Natl Acad Sci USA. 2011;108(40):16723-16728.
⁶ Kakimi et al. A phase I study of vaccination with NY-ESO-1f peptide mixed with Picibanil OK-432 and Montanide ISA-51 in patients with cancers expressing the NY-ESO-1 antigen.Int J Cancer. 2011;129(12):2836-46.
⁷ Wada et al. Vaccination with NY-ESO-1 overlapping peptides mixed with Picibanil OK-432 and montanide ISA-51 in patients with cancers expressing the NY-ESO-1 antigen. J Immunother. 2014;37(2):84-92.
⁸ Welters et al. Induction of tumor-specific CD4+ and CD8+ T-cell immunity in cervical cancer patients by a human papillomavirus type 16 E6 and E7 long peptides vaccine. Clin. Cancer Res. 2008; 14(1):178-87.
⁹ Kenter et al. Vaccination against HPV-16 oncoproteins for vulvar intraepithelial neoplasia. N Engl J Med. 2009; 361(19):1838-47.
¹⁰ Welters et al. Success or failure of vaccination for HPV16-positive vulvar lesions correlates with kinetics and phenotype of induced T-cell responses. PNAS. 2010; 107(26):11895-9.
¹¹ http://www.ncbi.nlm.nih.gov/projects/gv/mhc/main.fcgi?cmd=initThe MHC database, NCBI (Accessed Mar 7, 2016).
¹² Karkada et al. Therapeutic vaccines and cancer: focus on DPX-0907. Biologics. 2014;8:27-38.
¹³ Butts et al. Randomized phase IIB trial of BLP25 liposome vaccine in stage IIIB and IV non-small-cell lung cancer. J Clin Oncol. 2005;23(27):6674-81.
¹⁴ Yuan et al.Safety and immunogenicity of a human and mouse gp100 DNA vaccine in a phase I trial of patients with melanoma.Cancer Immun. 2009;9:5.
¹⁵ Kovjazin et al. ImMucin: a novel therapeutic vaccine with promiscuous MHC binding for the treatment of MUC1-expressing tumors. Vaccine. 2011;29(29-30):4676-86.
¹⁶ Cathcart et al. Amultivalent bcr-abl fusion peptide vaccination trial in patients with chronic myeloid leukemia.Blood. 2004;103:1037-1042.
¹⁷ Chapuis et al. Transferred WT1-reactive CD8+ T cells can mediate antileukemic activity and persist in post-transplant patients. Sci Transl Med. 2013;5(174):174ra27.
¹⁸ Keilholz et al. A clinical and immunologic phase 2 trial of Wilms tumor gene product 1 (WT1) peptide vaccination in patients with AML and MDS. Blood; 2009; 113(26):6541-8.
¹⁹ Walter et al. Multipeptide immune response to cancer vaccine IMA901 after single-dose cyclophosphamide associates with longer patient survival. Nat Med. 2012;18(8):1254-61.
²⁰ Phuphanich et al. Phase I trial of a multi-epitope-pulsed dendritic cell vaccine for patients with newly diagnosed glioblastoma. Cancer Immunol Immunother. 2013;62(1):125-35.
²¹ Kantoff et al. Overall survival analysis of a phase II randomized controlled trial of a Poxviral-based PSA-targeted immunotherapy in metastatic castration-resistant prostate cancer. J Clin Oncol. 2010;28(7):1099-105.
²² Tagawa et al. Phase I study of intranodal delivery of a plasmid DNA vaccine for patients with Stage IV melanoma. Cancer. 2003;98(1):144-54.
²³ Slingluff et al. Randomized multicenter trial of the effects of melanoma-associated helper peptides and cyclophosphamide on the immunogenicity of a multipeptide melanoma vaccine.J Clin Oncol. 2011;29(21):2924-32.
²⁴ Kaida et al. Phase 1 trial of Wilms tumor 1 (WT1) peptide vaccine and gemcitabine combination therapy in patients with advanced pancreatic or biliary tract cancer. J Immunother. 2011;34(1):92-9.
²⁵ Fenoglio et al. A multi-peptide, dual-adjuvant telomerase vaccine (GX301) is highly immunogenic in patients with prostate and renal cancer. Cancer Immunol Immunother; 2013; 62:1041-1052.
²⁶Krug et al. WT1 peptide vaccinations induce CD4 and CD8 T cell immune responses in patients with mesothelioma and non-small cell lung cancer. Cancer Immunol Immunother; 2010; 59(10):1467-79.
²⁷Slingluff et al. Clinical and immunologic results of a randomized phase II trial of vaccination using four melanoma peptides either administered in granulocyte-macrophage colony-stimulating factor in adjuvant or pulsed on dendritic cells. J Clin Oncol; 2003; 21(21):4016-26.
²⁸Hodi et al. Improved survival with ipilimumab in patients with metastatic melanoma. N Engl J Med; 2010;363(8):711-23.
²⁹ Carmon et al. Phase I/II study exploring ImMucin, a pan-major histocompatibility complex, anti-MUC1 signal peptide vaccine, in multiple myeloma patients. Br J Hematol. 2014; 169(1):44-56. ³⁰http://www.merckgroup.com/en/media/extNewsDetail.html?newsId=EB4A46A2AC4A52E7C 1257AD9001F3186&newsType=1(Accessed Mar 28, 2016)
³¹ Trimble et al. Safety, efficacy, and immunogenicity of VGX-3100, a therapeutic synthetic DNA vaccine targeting human papillomavirus 16 and 18 E6 and E7 proteins for cervical intraepithelial neoplasia 2/3: a randomised, double-blind, placebo-controlled phase 2b trial. Lancet. 2015;386(10008):2078-88.
³²Cusi et al. Phase I trial of thymidylate synthase poly epitope peptide (TSPP) vaccine in advanced cancer patients. Cancer Immunol Immunother; 2015; 64:1159-1173. ³³Asahara et al. Phase I/II clinical trial using HLA-A24-restricted peptide vaccine derived from KIF20A for patients with advanced pancreatic cancer. J Transl Med; 2013; 11:291.
³⁴Yoshitake et al. Phase II clinical trial of multiple peptide vaccination for advanced head and neck cancer patients revealed induction of immune responses and improved OS. Clin Cancer Res; 2014;21(2):312-21.
³⁵Okuno et al. Clinical Trial of a 7-Peptide Cocktail Vaccine with Oral Chemotherapy for Patients with Metastatic Colorectal Cancer. Anticancer Res; 2014; 34: 3045-305.
³⁶Rapoport et al. Combination Immunotherapy after ASCT for Multiple Myeloma Using MAGE-A3/Poly-ICLC Immunizations Followed by Adoptive Transfer of Vaccine-Primed and Costimulated Autologous T Cells. Clin Cancer Res; 2014; 20(5): 1355-1365.
³⁷Greenfield et al. A phase I dose-escalation clinical trial of a peptidebased human papillomavirus therapeutic vaccine with Candida skin test reagent as a novel vaccine adjuvant for treating women with biopsy-proven cervical intraepithelial neoplasia 2/3. Oncoimmunol; 2015; 4:10, e1031439.
³⁸ Snyder et al. Genetic basis for clinical response to CTLA-4 blockade in melanoma. N Engl J Med. 2014; 371(23):2189-99.
³⁹ Van Allen et al. Genomic correlates of response to CTLA-4 blockade in metastatic melanoma. Science; 2015; 350:6257.
⁴⁰ Li et al. Thrombocytopenia caused by the development of antibodies to thrombopoietin. Blood; 2001; 98:3241-3248
⁴¹ Takedatsu et al. Determination of Thrombopoietin-Derived Peptides Recognized by Both Cellular and Humoral Immunities in Healthy Donors and Patients with Thrombocytopenia. 2005; 23(7): 975-982
⁴² Eisenhauer et al. New response evaluation criteria in solid tumors: revised RECIST guideline (version 1.1). Eur J Cancer; 2009; 45(2):228-47.
⁴³ Therasse et al. New guidelines to evaluate the response to treatment in solid tumors: European Organization for Research and Treatment of Cancer, National Cancer Institute of the United States, National Cancer Institute of Canada. J Natl Cancer Inst; 2000; 92:205-216.
⁴⁴ Tsuchida & Therasse. Response evaluation criteria in solid tumors (RECIST): New guidelines. Med Pediatr Oncol. 2001; 37:1-3.
⁴⁵ Durie et al. International uniform response criteria for multiple myeloma. Leukemia; 2006;20:1467-1473.

## Claims

1. A method of identifying a fragment of a polypeptide as immunogenic for a specific human subject, the method comprising the steps of
(i) determining the HLA class I genotype and/or the HLA class II genotype of the specific human subject;
(ii) determining whether the polypeptide comprises:
a. a sequence that is a T cell epitope capable of binding to at least two HLA class I of the subject; or
b. a sequence that is a T cell epitope capable of binding to at least two HLA class II of the subject; and
(iii) identifying said sequence as a fragment of the polypeptide that is immunogenic for the subject.

2. The method of claim 1, wherein the fragment is capable of binding to at least two HLA class I of the subject and consists of 9 consecutive amino acids of the polypeptide, or wherein the fragment is capable of binding to at least two HLA class II of the subject and consists of 15 consecutive amino acids of the polypeptide.

3. The method of claim 1 or claim 2, wherein step (i) comprises determining the HLA class I genotype of the subject, and step (ii) comprises determining whether the polypeptide comprises a sequence that is a T cell epitope capable of binding to at least two HLA class I of the subject.

4. The method of claim 3, wherein step (ii) comprises determining whether the polypeptide comprises a sequence that is a T cell epitope capable of binding to at least three different HLA class I molecules of the subject.

5. The method of any one of the preceding claims, wherein the polypeptide is expressed by a pathogenic organism, a virus or a cancer cell, is associated with an autoimmune disorder, or is an allergen or an ingredient of a pharmaceutical composition.

6. The method of any one of the preceding claims, wherein the polypeptide is selected from the antigens listed in Tables 1 to 4.

7. The method of any one of the preceding claims, wherein the polypeptide is an antigen or neoantigen expressed by a cancer cell, optionally wherein the cancer cell is in a sample taken from the subject.

8. The method of any one of claims 1 to 7, wherein steps (ii) and (iii) are repeated until all of the fragments of the polypeptide that are a T cell epitope capable of binding to at least two different HLA class I molecules of the subject have been identified.

9. The method of claim 8 further comprising predicting whether the polypeptide will induce a cytotoxic T cell response in the subject, wherein
a. the identification of at least one fragment of the polypeptide that is a T cell epitope capable of binding to at least two HLA class I of the subject predicts that the polypeptide will induce a cytotoxic T cell response in the subject; and
b. the identification of no fragments of the polypeptide that are T cell epitopes capable of binding to at least two HLA class I of the subject predicts that the polypeptide will not induce a cytotoxic T cell response in the subject.

10. The method of claim 8 wherein the polypeptide is an active ingredient of a vaccine or immunotherapy composition having one or more polypeptide as active ingredients; and wherein the method further comprises
repeating the method of claim 8 for each polypeptide that is an active ingredient of the composition; and
predicting the likelihood that the subject will have a clinical response to the composition, wherein
a. the identification of at least two fragments of the one or more polypeptides, each of which is a T cell epitope capable of binding to at least two HLA class I of the subject predicts that the subject is more likely to have a clinical response to the composition; and
b. the identification of less than two fragments of the one or more polypeptides that are T cell epitopes capable of binding to at least two different HLA class I of the subject predicts that the subject is less likely to have a clinical response to the composition.

11. A method of preparing a human subject-specific pharmaceutical composition, the method comprising:
(i) selecting a first fragment of a polypeptide, which fragment has been identified as immunogenic for the subject by the method of any one of claims 1 to 7;
(ii) selecting a sequence of up to 50 consecutive amino acids of the polypeptide, which consecutive amino acids comprise the amino acid sequence of the first fragment;
(iii) selecting a second immunogenic fragment of the same or of a different polypeptide to the fragment selected in step (i), and selecting a sequence of up to 50 consecutive amino acids of the polypeptide, which consecutive amino acids comprise the amino acid sequence of the second fragment; and
(iv) optionally selecting one or more additional immunogenic fragments from the same or different polypeptides to the fragments selected in the preceding steps, and selecting, for each additional fragment, a sequence of up to 50 consecutive amino acids of the polypeptide, which consecutive amino acids comprise the amino acid sequence of the additional fragment; and
(v) preparing a composition having as active ingredients one or more peptides having all of the amino acid sequences selected in the preceding steps, wherein each peptide either consists of one of the selected amino acid sequences, or consists of two or more of the amino acid sequences arranged end to end or overlapping in a single peptide.

12. A human subject-specific pharmaceutical composition prepared according to the method of claim 11, which optionally comprises at least one pharmaceutically acceptable diluent, carrier, or preservative.

13. A method of treatment comprising administering to a human subject in need thereof a human subject-specific pharmaceutical composition of claim 13, wherein the composition is specific for the subject, optionally wherein the method is for the treatment of cancer.


SEQUENCE LISTING

<110>  Epitope Kft

<120>  IMMUNOGENIC PEPTIDES

<130>  N409650EP

<140>  tbc
<141>  2017-03-02

<160>  36

<170>  PatentIn version 3.5

<210>  1
<211>  16
<212>  PRT
<213>  Artificial Sequence

<220>
<223>  JUP

<400>  1

Met Met Asn Leu Met Gln Pro Lys Thr Gln Gln Thr Tyr Thr Tyr Asp
1               5                   10                  15


<210>  2
<211>  21
<212>  PRT
<213>  Artificial Sequence

<220>
<223>  ADA17

<400>  2

Gly Arg Gly Ser Thr Thr Thr Asn Tyr Leu Leu Asp Arg Asp Asp Tyr
1               5                   10                  15


Arg Asn Thr Ser Asp
            20


<210>  3
<211>  22
<212>  PRT
<213>  Artificial Sequence

<220>
<223>  BAP31

<400>  3

Leu Lys Lys Gly Ala Ala Asp Gly Gly Lys Leu Asp Gly Asn Ala Lys
1               5                   10                  15


Leu Asn Arg Ser Leu Lys
            20

<210>  4
<211>  22
<212>  PRT
<213>  Artificial Sequence

<220>
<223>  TOP2A

<400>  4

Phe Pro Pro Lys Asp Asp His Thr Leu Lys Phe Leu Tyr Asp Asp Asn
1               5                   10                  15

Gln Arg Pro Tyr Pro Pro
            20

<210>  5
<211>  21
<212>  PRT
<213>  Artificial Sequence

<220>
<223>  Abl-2

<400>  5

Arg Tyr Arg Lys Pro Asp Tyr Thr Leu Asp Asp Gly His Gly Leu Leu
1               5                   10                  15

Arg Phe Lys Ser Thr
            20

<210>  6
<211>  18
<212>  PRT
<213>  Artificial Sequence

<220>
<223>  DDR1

<400>  6

Gln Arg Pro Pro Phe Ser Gln Leu His Arg Phe Leu Ala Asp Ala Leu
1               5                   10                  15

Asn Thr

<210>  7
<211>  25
<212>  PRT
<213>  Artificial Sequence

<220>
<223>  ITGB8

<400>  7

Ala Leu Asp Gln Cys Lys Thr Ser Cys Ala Leu Met Gln Gln His Tyr
1               5                   10                  15

Asp Gln Thr Ser Cys Phe Ser Ser Pro
            20                  25

<210>  8
<211>  25
<212>  PRT
<213>  Artificial Sequence

<220>
<223>  MUC-1

<400>  8

Ser Thr Ala Pro Pro Ala His Gly Val Thr Ser Ala Pro Asp Thr Arg
1               5                   10                  15

Pro Ala Pro Gly Ser Thr Ala Pro Pro
            20                  25

<210>  9
<211>  9
<212>  PRT
<213>  Artificial Sequence

<220>
<223>  gp100

<400>  9

Tyr Leu Glu Pro Gly Pro Val Thr Ala
1               5

<210>  10
<211>  21
<212>  PRT
<213>  Artificial Sequence

<220>
<223>  MUC-1

<400>  10

Met Thr Pro Gly Thr Gln Ser Pro Phe Phe Leu Leu Leu Leu Leu Thr
1               5                   10                  15

Val Leu Thr Val Val
            20

<210>  11
<211>  9

<212>  PRT
<213>  Artificial Sequence

<220>
<223>  Bcr-Abl

<400>  11

Ser Ser Lys Ala Leu Gln Arg Pro Val
1               5


<210>  12
<211>  9
<212>  PRT
<213>  Artificial Sequence

<220>
<223>  WT-1

<400>  12

Arg Met Phe Pro Asn Ala Pro Tyr Leu
1               5


<210>  13
<211>  22
<212>  PRT
<213>  Artificial Sequence

<220>
<223>  AKAP4

<400>  13

Gln Lys Gln Leu Gln Ala Val Leu Gln Trp Ile Ala Ala Ser Gln Phe
1               5                   10                  15


Asn Val Pro Met Leu Tyr
            20


<210>  14
<211>  20
<212>  PRT
<213>  Artificial Sequence

<220>
<223>  BORIS

<400>  14

Asp Glu Arg Ser Asp Glu Ile Val Leu Thr Val Ser Asn Ser Asn Val
1               5                   10                  15


Glu Glu Gln Glu
            20


<210>  15

<211>  20
<212>  PRT
<213>  Artificial Sequence

<220>
<223>  SPAG9

<400>  15

Arg Glu Glu Ala Gln Lys Met Ser Ser Leu Leu Pro Thr Met Trp Leu
1               5                   10                  15

Gly Ala Gln Asn
            20

<210>  16
<211>  20
<212>  PRT
<213>  Artificial Sequence

<220>
<223>  OY-TES-1

<400>  16

Val Glu Ser Thr Pro Met Ile Met Glu Asn Ile Gln Glu Leu Ile Arg
1               5                   10                  15

Ser Ala Gln Glu
            20

<210>  17
<211>  20
<212>  PRT
<213>  Artificial Sequence

<220>
<223>  SP17

<400>  17

Ala Tyr Phe Glu Ser Leu Leu Glu Lys Arg Glu Lys Thr Asn Phe Asp
1               5                   10                  15

Pro Ala Glu Trp
            20

<210>  18
<211>  20
<212>  PRT
<213>  Artificial Sequence

<220>
<223>  WT1

<400>  18

Ala Ser Ser Gly Gln Ala Arg Met Phe Pro Asn Ala Pro Tyr Leu Pro
1               5                   10                  15

Ser Cys Leu Glu
            20

<210>  19
<211>  20
<212>  PRT
<213>  Artificial Sequence

<220>
<223>  HIWI

<400>  19

Ile Ala Gly Phe Val Ala Ser Ile Asn Glu Gly Met Thr Arg Trp Phe
1               5                   10                  15

Ser Arg Cys Ile
            20

<210>  20
<211>  20
<212>  PRT
<213>  Artificial Sequence

<220>
<223>  PRAME

<400>  20

Arg His Ser Gln Thr Leu Lys Ala Met Val Gln Ala Trp Pro Phe Thr
1               5                   10                  15

Cys Leu Pro Leu
            20

<210>  21
<211>  20
<212>  PRT
<213>  Artificial Sequence

<220>
<223>  MAGE-A10

<400>  21

Asp Pro Thr Gly His Ser Phe Val Leu Val Thr Ser Leu Gly Leu Thr
1               5                   10                  15

Tyr Asp Gly Met
            20

<210>  22

<211>  20
<212>  PRT
<213>  Artificial Sequence

<220>
<223>  MAGE-A4

<400>  22

Lys Val Leu Glu His Val Val Arg Val Asn Ala Arg Val Arg Ile Ala
1               5                   10                  15

Tyr Pro Ser Leu
            20

<210>  23
<211>  20
<212>  PRT
<213>  Artificial Sequence

<220>
<223>  MAGE-A9

<400>  23

Gln Glu Ala Leu Lys Leu Lys Val Ala Glu Leu Val His Phe Leu Leu
1               5                   10                  15

His Lys Tyr Arg
            20

<210>  24
<211>  20
<212>  PRT
<213>  Artificial Sequence

<220>
<223>  DPPA2

<400>  24

Lys Gly Trp Val Arg Leu Gln Phe His Ala Gly Gln Ala Trp Val Pro
1               5                   10                  15

Thr Thr His Arg
            20

<210>  25
<211>  20
<212>  PRT
<213>  Artificial Sequence

<220>
<223>  FSIP1

<400>  25

Ile Ser Asp Thr Lys Asp Tyr Phe Met Ser Lys Thr Leu Gly Ile Gly
1               5                   10                  15

Arg Leu Lys Arg
            20

<210>  26
<211>  20
<212>  PRT
<213>  Artificial Sequence

<220>
<223>  SPAG9

<400>  26

Arg Asp Ala Val Lys Phe Phe Val Ala Val Pro Gly Gln Val Ile Ser
1               5                   10                  15

Pro Gln Ser Ser
            20

<210>  27
<211>  20
<212>  PRT
<213>  Artificial Sequence

<220>
<223>  AKAP4 (CT99)

<400>  27

Ser Gln Lys Met Asp Met Ser Asn Ile Val Leu Met Leu Ile Gln Lys
1               5                   10                  15

Leu Leu Asn Glu
            20

<210>  28
<211>  20
<212>  PRT
<213>  Artificial Sequence

<220>
<223>  BORIS

<400>  28

Ser Ala Val Phe His Glu Arg Tyr Ala Leu Ile Gln His Gln Lys Thr
1               5                   10                  15

His Lys Asn Glu
            20

<210>  29

<211>  20
<212>  PRT
<213>  Artificial Sequence

<220>
<223>  MAGE-A11

<400>  29

Glu Val Asp Pro Thr Ser His Ser Tyr Val Leu Val Thr Ser Leu Asn
1               5                   10                  15

Leu Ser Tyr Asp
            20

<210>  30
<211>  20
<212>  PRT
<213>  Artificial Sequence

<220>
<223>  NY-SAR-35

<400>  30

Ser Ala Leu Glu Ala Leu Leu Asn Phe Phe Phe Pro Thr Thr Cys Asn
1               5                   10                  15

Leu Arg Glu Asn
            20

<210>  31
<211>  20
<212>  PRT
<213>  Artificial Sequence

<220>
<223>  HOM-TES-85

<400>  31

Met Ala Ser Phe Arg Lys Leu Thr Leu Ser Glu Lys Val Pro Pro Asn
1               5                   10                  15

His Pro Ser Arg
            20

<210>  32
<211>  20
<212>  PRT
<213>  Artificial Sequence

<220>
<223>  NY-BR-1

<400>  32

Lys Arg Ala Ser Gln Tyr Ser Gly Gln Leu Lys Val Leu Ile Ala Glu
1               5                   10                  15

Asn Thr Met Leu
            20

<210>  33
<211>  20
<212>  PRT
<213>  Artificial Sequence

<220>
<223>  MAGE-A9

<400>  33

Val Asp Pro Ala Gln Leu Glu Phe Met Phe Gln Glu Ala Leu Lys Leu
1               5                   10                  15

Lys Val Ala Glu
            20

<210>  34
<211>  20
<212>  PRT
<213>  Artificial Sequence

<220>
<223>  SCP-1

<400>  34

Glu Tyr Glu Arg Glu Glu Thr Arg Gln Val Tyr Met Asp Leu Asn Asn
1               5                   10                  15

Asn Ile Glu Lys
            20

<210>  35
<211>  20
<212>  PRT
<213>  Artificial Sequence

<220>
<223>  MAGE-A1

<400>  35

Pro Glu Ile Phe Gly Lys Ala Ser Glu Ser Leu Gln Leu Val Phe Gly
1               5                   10                  15

Ile Asp Val Lys
            20

<210>  36

<211>  20
<212>  PRT
<213>  Artificial Sequence

<220>
<223>  MAGE-A10

<400>  36

Asn Tyr Glu Asp His Phe Pro Leu Leu Phe Ser Glu Ala Ser Glu Cys
1               5                   10                  15

Met Leu Leu Val
            20